# EUROPÄISCHE PATENTSCHRIFT

(11) **EP 0 915 868 B1**
(45) Veröffentlichungstag und Bekanntmachung des Hinweises auf die Patenterteilung: **08.12.2004**
(21) Anmeldenummer: 97930522.4
(22) Anmeldetag: 11.07.1997
(51) Int. Cl.: C07D 333/38, A01N 37/22, C07D 231/14, C07D 213/82, C07C 233/75, C07D 277/56, C07D 327/06

(54) **CARBANILIDE ALS SCHÄDLINGSBEKÄMPFUNGSMITTEL**
CARBANILIDES USED AS PESTICIDES
CARBANILIDES S'UTILISANT COMME AGENTS DE LUTTE CONTRE LES PARASITES

(30) Priorität: 24.07.1996 DE 19629828
(43) Veröffentlichungstag der Anmeldung: 19.05.1999
(60) Teilanmeldung: 04009928.5
(73) Patentinhaber: Bayer CropScience AG, 40789 Monheim (DE)
(72) Erfinder: ELBE, Hans-Ludwig, D-42329 Wuppertal (DE); KRÜGER, Bernd-Wieland, D-51467 Bergisch Gladbach (DE); MARKERT, Robert, D-51065 Köln (DE); TIEMANN, Ralf, D-51375 Leverkusen (DE); KUHNT, Dietmar, D-41399 Burscheid (DE); DUTZMANN, Stefan, D-40764 Langenfeld (DE); STENZEL, Klaus, D-40595 Düsseldorf (DE); ERDELEN, Christoph, D-42799 Leichlingen (DE); KUGLER, Martin, D-42799 Leichlingen (DE); BUSCHHAUS, Hans-Ulrich, D-47800 Krefeld (DE)
(86) Internationale Anmeldenummer: PCT/EP1997/003694
(87) Internationale Veröffentlichungsnummer: WO 1998/003500

(56) Entgegenhaltungen:
- EP-A- 0 545 099
- EP-A- 0 589 301
- WO-A-93/11117
- WO-A-95/25723
- DE-A- 1 913 178
- US-A- 3 657 449
- CHEMICAL ABSTRACTS, vol. 106, no. 25, 22.Juni 1987 Columbus, Ohio, US; abstract no. 209380, CARMELLINO, M. L. ET AL: "Activity of some 2-iodobenzanilide derivatives on plant-pathogenic fungi" XP002044966 & FARMACO, ED. SCI. (1987), 42(3), 231-5 CODEN: FRPSAX;ISSN: 0430-0920, 1987,
- WHITE, G. A. ET AL: "Thiophene carboxamide fungicides: structure-activity relationships with the succinate dehydrogenase complex from wild-type and carboxin-resistant mutant strains of Aspergillus nidulans" PESTIC. BIOCHEM. PHYSIOL. (1986), 25(2), 188-204 CODEN: PCBPBS;ISSN: 0048-3575, 1986, XP002045315
- CHEMICAL ABSTRACTS, vol. 115, no. 19, 11.November 1991 Columbus, Ohio, US; abstract no. 207662, MEGURO, KANJI ET AL: "Preparation of 2-amino-5-methylbenzophenones" XP002044968 & JP 03 130 252 A (TAKEDA CHEMICAL INDUSTRIES, LTD., JAPAN)
- FUERSTNER, ALOIS ET AL: "Palladium-catalyzed arylation of polar organometallics mediated by 9-methoxy-9-borabicyclo[3.3.1]nonane: Suzuki reactions of extended scope" TETRAHEDRON (1995), 51(41), 11165-76 CODEN: TETRAB;ISSN: 0040-4020, 1995, XP002044964
- FUERSTNER, ALOIS ET AL: ""Site Selective" Formation of Low-Valent Titanium Reagents: An "Instant" Procedure for the Reductive Coupling of Oxo Amides to Indoles" J. ORG. CHEM. (1994), 59(18), 5215-29 CODEN: JOCEAH;ISSN: 0022-3263, 1994, XP002044965

## Beschreibung

Die vorliegende Erfindung betrifft neue Carbanilide, mehrere Verfahren zu deren Herstellung und deren Verwendung zur Bekämpfung von pflanzlichen und tierischen Schädlingen.

Es ist bereits bekannt, daß zahlreiche Carboxamide fungizide Eigenschaften besitzen (vgl. WO 93-11 117, EP-A 0 545 099, EP-A 0 589 301 und EP-A 0 589 313). So sind beispielsweise Benzanilide aus Farmaco, Ed. Sci. **1987,** *42,* 231-235, Tetrahedron **1995,** *41*. 11165-11176, J. Org. Chem. **1994**, *59,* 5215-5229, DE-A 19 13 178 und JP-A 3-130252,Pyrazolylcarboxanilide aus WO 93/1117 und EP-A 0 589 301. 2-Thienylcarboxanilide aus Pestic. Biochem. Physiol. **1986,** *25,* 188-204, Nicotinsäureanilide aus WO 95/25723 und EP-A 0 545 099, Oxathiincarboxamide aus US 3,657,449, sowie Furylcarboxanilide aus Trends QSAR Mol. Modell. 92, Prox. Eur. Symp. Struct.-Act. Relat.: QSAR Mol. Modell., 9^{th} Meeting, 1993, 577-579 bekannt. Die Wirksamkeit dieser Stoffe ist gut, läßt aber in manchen Fällen zu wünschen übrig.

Es wurden nun neue Carbanilide der Formel 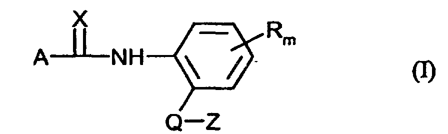 in welcher
- R: für Halogen, Nitro, Cyano, Alkyl mit 1 bis 8 Kohlenstoffatomen, Halogenalkyl mit 1 bis 6 Kohlenstoffatomen und 1 bis 5 Halogenatomen, Alkoxy mit 1 bis 8 Kohlenstoffatomen, Halogenalkoxy mit 1 bis 6 Kohlenstoffatomen und 1 bis 5 Halogenatomen, Alkylthio mit 1 bis 8 Kohlenstoffatomen, Halogenalkylthio mit 1 bis 6 Kohlenstoffatomen und 1 bis 5 Halogenatomen, Alkenyloxy mit 2 bis 8 Kohlenstoffatomen, Alkinyloxy mit 2 bis 8 Kohlenstoffatomen, Cycloalkyl mit 3 bis 8 Kohlenstoffatomen, Carbalkoxy mit 1 bis 8 Kohlenstoffatomen im Alkoxyteil oder Alkoximinoalkyl mit 1 bis 6 Kohlenstoffatomen im Alkoxyteil und 1 bis 6 Kohlenstoffatomen im Alkylteil steht,
- m: für die Zahlen 0, 1, 2, 3 oder 4 steht,
- A: für einen Rest der Formel 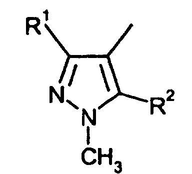 steht, worin
R¹ für Halogen, Alkyl mit 1 bis 4 Kohlenstoffatomen oder Halogenalkyl mit 1 bis 4 Kohlenstoffatomen und 1 bis 5 Halogenatomen steht und
R² für Wasserstoff Halogen, Cyano oder Alkyl mit 1 bis 4 Kohlenstoffatomen steht, oder
oder
- A: für einen Rest der Formel 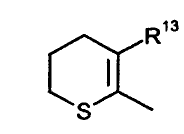 steht, worin
R¹³ für Alkyl mit 1 bis 4 Kohlenstoffatomen oder für Halogenalkyl mit 1 bis 4 Kohlenstoffatomen und 1 bis 5 Halogenatomen steht,
oder
- A: für einen Rest der Formel 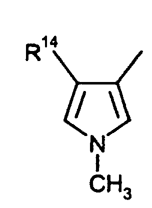 steht, worin
R¹⁴ für Halogen, Cyano, Alkyl mit 1 bis 4 Kohlenstoffatomen oder Halogenalkyl mit 1 bis 4 Kohlenstoffatomen und 1 bis 5 Halogenatomen steht,
oder
- A: für einen Rest der Formel 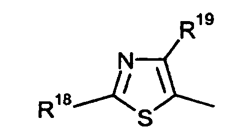 steht, worin
R¹⁸ für Wasserstoff, Halogen, Amino, Cyano oder Alkyl mit 1 bis 4 Kohlenstoffatomen steht und
R¹⁹ für Halogen, Alkyl mit 1 bis 4 Kohlenstoffatomen oder Halogenalkyl mit 1 bis 4 Kohlenstoffatomen und 1 bis 5 Halogenatomen steht,
oder
- A: für einen Rest der Formel 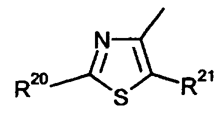 steht, worin
R²⁰ für Wasserstoff, Halogen, Amino, Cyano oder Alkyl mit 1 bis 4 Kohlenstoffatomen steht und
R²¹ für Halogen, Alkyl mit 1 bis 4 Kohlenstoffatomen oder Halogenalkyl mit 1 bis 4 Kohlenstoffatomen und 1 bis 5 Halogenatomen steht,
oder
- A: für einen Rest der Formel 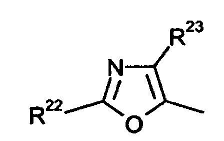 steht, worin
R²² für Wasserstoff oder Alkyl mit 1 bis 4 Kohlenstoffatomen steht und
R²³ für Halogen oder Alkyl mit 1 bis 4 Kohlenstoffatomen steht,
oder
- A: für einen Rest der Formel 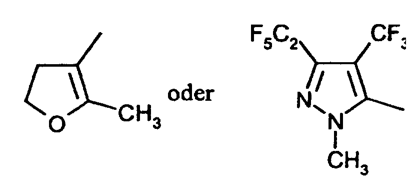 steht,
- Q: für Alkylen mit 1 bis 4 Kohlenstoffatomen, Alkenylen mit 2 bis 4 Kohlenstoffatomen, Alkinylen mit 2 bis 4 Kohlenstoffatomen oder eine Gruppe der Formel 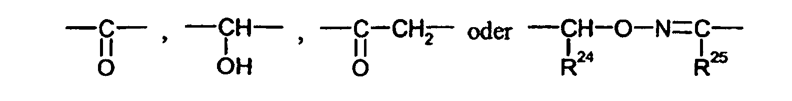 steht, worin
R²⁴ und R²⁵ unabhängig voneinander für Wasserstoff, Alkyl mit 1 bis 4 Kohlenstoffatomen, Cycloalkyl mit 3 bis 6 Kohlenstoffatomen, Alkenyl mit 2 bis 4 Kohlenstoffatomen oder Alkinyl mit 2 bis 4 Kohlenstoffatomen stehen, oder
- Q: für eine Gruppe der Formel 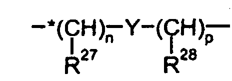 steht, worin
R²⁷ und R²⁸ unabhängig voneinander für Wasserstoff oder Alkyl mit 1 bis 4 Kohlenstoffatomen stehen,
Y für ein Sauerstoffatom oder für S(O)ᵣ steht, wobei
r für die Zahlen 0, 1 oder 2 steht, und
n und p unabhängig voneinander für die Zahlen 0, 1 oder 2 stehen, wobei der durch (*) gekennzeichnete Molekülteil jeweils mit dem Phenylrest des Anilinteiles verbunden ist,
- X: für Sauerstoff oder Schwefel steht und
- Z: für gegebenenfalls substituiertes Phenyl, gegebenenfalls substituiertes Naphthyl, gegebenenfalls substituiertes Anthracenyl oder für gegebenenfalls substituiertes Hetaryl steht,
gefunden.

Weiterhin wurde gefunden, daß man Carbanilide der Formel (I) erhält, wenn man
a) Säurehalogenide der Formel 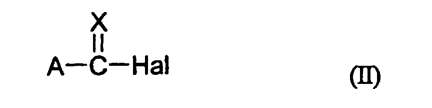 in welcher
   A und X die oben angegebenen Bedeutungen haben und
   Hal für Halogen steht,
   mit Anilin-Derivaten der Formel 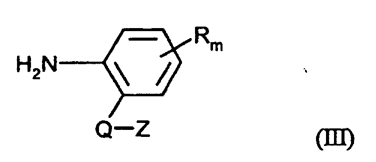 in welcher
   Q, R, Z und m die oben angegebenen Bedeutungen haben,
   gegebenenfalls in Gegenwart eines Säurebindemittels und gegebenenfalls in Gegenwart eines Verdünnungsmittels umsetzt,
   oder
b) Carbanilid-Derivate der Formel 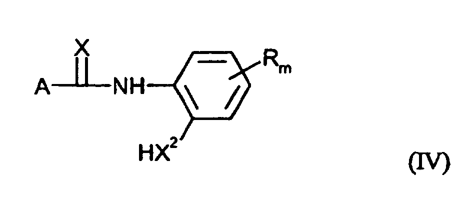 in welcher
   A, R, X und m die oben angegebenen Bedeutungen haben und
   - X²: für Sauerstoff oder Schwefel steht,
   mit Verbindungen der Formel 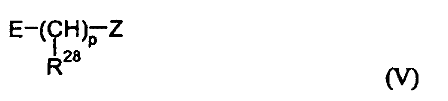 in welcher
   R²⁸, Z und p die oben angegebenen Bedeutungen haben und
   - E: für eine Austrittsgruppe steht,
   gegebenenfalls in Gegenwart eines Säurebindemittels und gegebenenfalls in Gegenwart eines Verdünnungsmittels umsetzt, oder
c) Carbanilid-Derivate der Formel 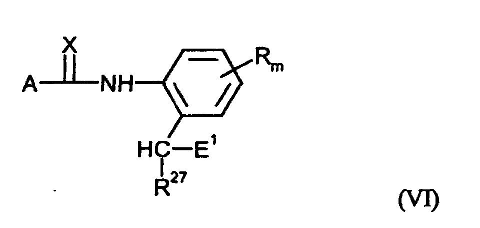 in welcher
   A, R, R²⁷, X und m die oben angegebenen Bedeutungen haben und
   - E¹: für eine Austrittsgruppe steht,
   mit Verbindungen der Formel 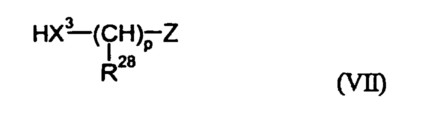 in welcher
   - R²⁸, Z und p: die oben angegebenen Bedeutungen haben und
   - X³: für Sauerstoff oder Schwefel steht,
   gegebenenfalls in Gegenwart eines Säurebindemittels und gegebenenfalls in Gegenwart eines Verdünnungsmittels umsetzt, oder
d) Carbanilid-Derivate der Formel 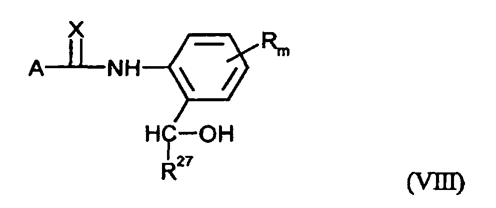 in welcher
   A, R, R²⁷, X und m die oben angegebenen Bedeutungen haben,
   mit Verbindungen der Formel 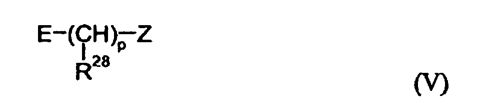 in welcher
   R²⁸, Z und p die oben angegebenen Bedeutungen haben und
   - E: für eine Austrittsgruppe steht,
   gegebenenfalls in Gegenwart eines Säurebindemittels und gegebenenfalls in Gegenwart eines Verdünnungsmittels umsetzt.

Schließlich wurde gefunden, daß die neuen Carbanilide der Formel (I) sehr gut als Schädlingsbekämpfungsmittel verwendbar sind. Sie besitzen mikrobizide Eigenschaften und können zur Bekämpfung unerwünschter Mikroorganismen sowohl im Pflanzenschutz als auch im Materialschutz eingesetzt werden. Außerdem eignen sie sich zur Bekämpfung von tierischen Schädlingen.

Überraschenderweise zeigen die erfindungsgemäßen Carbanilide der Formel (I) eine wesentlich bessere fungizide Wirksamkeit als die konstitutionell ähnlichsten, vorbekannten Carboxamide gleicher Wirkungsrichtung.

Die erfindungsgemäßen Carbanilide sind durch die Formel (I) allgemein definiert.
- R: steht vorzugsweise für Fluor, Chlor, Brom, Nitro, Cyano, Alkyl, mit 1 bis 6 Kohlenstoffatomen, Halogenalkyl mit 1 oder 2 Kohlenstoffatomen und 1 bis 5 Fluor-, Chlor- und/oder Bromatomen, Alkoxy mit 1 bis 6 Kohlenstoffatomen, Halogenalkoxy mit 1 oder 2 Kohlenstoffatomen und 1 bis 5 Fluor-, Chlor- und/oder Bromatomen, Alkylthio mit 1 bis 6 Kohlenstoffatomen, Halogenalkylthio mit 1 oder 2 Kohlenstoffatomen und 1 bis 5 Fluor-, Chlor- und/oder Bromatomen, Alkenyloxy mit 2 bis 6 Kohlenstoffatomen, Alkinyloxy mit 2 bis 6 Kohlenstoffatomen, Cycloalkyl mit 3 bis 7 Kohlenstoffatomen, Carbalkoxy mit 1 bis 4 Kohlenstoffatomen im Alkoxyteil oder für Alkoximinoalkyl mit 1 bis 4 Kohlenstoffatomen im Alkoxyteil und 1 bis 4 Kohlenstoffatomen im Alkylteil.
- m: steht vorzugsweise für die Zahlen 0, 1, 2 oder 3, wobei R für gleiche oder verschiedene Reste steht, wenn m für 2 oder 3 steht.
- A: steht vorzugsweise für einen Rest der Formel 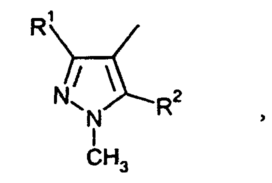 worin
R¹ für Fluor, Chlor, Brom, Iod, Methyl, Ethyl, Isopropyl oder Halogenalkyl mit 1 oder 2 Kohlenstoffatomen und 1 bis 5 Fluor-, Chlor- und/oder Bromatomen steht und
R² für Wasserstoff, Fluor, Chlor, Brom, Iod, Methyl oder Ethyl steht.
- A: steht außerdem vorzugsweise für einen Rest der Formel 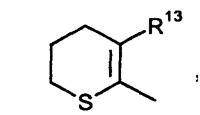 worin
R¹³ für Methyl, Ethyl oder Halogenalkyl, mit 1 oder 2 Kohlenstoffatomen und 1 bis 5 Fluor-, Chlor- und/oder Bromatomen steht.
- A: steht außerdem vorzugsweise für einen Rest der Formel 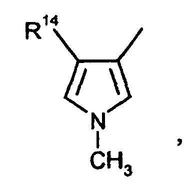 worin
R¹⁴ für Fluor, Chlor, Brom, Cyano, Methyl, Ethyl oder Halogenalkyl mit 1 oder 2 Kohlenstoffatomen und 1 bis 5 Fluor-, Chlor- und/oder Bromatomen steht.
- A: steht außerdem vorzugsweise für einen Rest der Formel 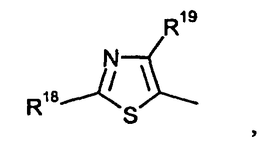 worin
R¹⁸ für Wasserstoff, Fluor, Chlor, Brom, Amino, Cyano, Methyl oder Ethyl steht und
R¹⁹ für Fluor, Chlor, Brom, Methyl, Ethyl oder Halogenalkyl mit 1 oder 2 Kohlenstoffatomen und 1 bis 5 Fluor-, Chlor- und/oder Bromatomen steht.
- A: steht außerdem vorzugsweise für einen Rest der Formel 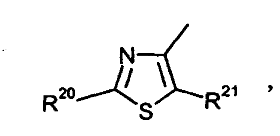 worin
R²⁰ für Wasserstoff, Fluor, Chlor, Brom, Amino, Cyano, Methyl oder Ethyl steht und
R²¹ für Fluor, Chlor, Brom, Methyl, Ethyl oder Halogenalkyl mit 1 oder 2 Kohlenstoffatomen und 1 bis 5 Fluor-, Chlor- und/oder Bromatomen steht.
- A: steht außerdem vorzugsweise für einen Rest der Formel 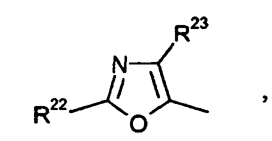 worin
R²² für Wasserstoff Methyl oder Ethyl steht und
R²³ für Fluor, Chlor, Brom, Methyl oder Ethyl steht.
- A: steht außerdem vorzugsweise für einen Rest der Formel 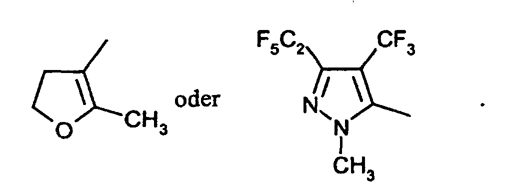
- Q: steht vorzugsweise für Alkylen mit 1 bis 3 Kohlenstoffatomen, Alkenylen mit 2 oder 3 Kohlenstoffatomen, Alkinylen mit 2 oder 3 Kohlenstoffatomen oder für eine Gruppe der Formel 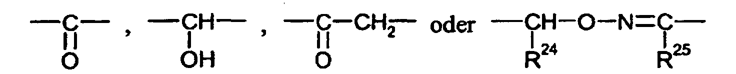 , worin
R²⁴ und R²⁵ unabhängig voneinander für Wasserstoff, Methyl, Ethyl, Cyclopropyl, Cyclopentyl, Cyclohexyl, Alkenyl mit 2 oder 3 Kohlenstoffatomen oder Alkinyl mit 2 oder 3 Kohlenstoffatomen stehen.
- Q: steht außerdem vorzugsweise für eine Gruppe der Formel 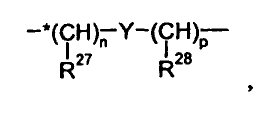 worin
R²⁷ und R²⁸ unabhängig voneinander für Wasserstoff, Methyl oder Ethyl stehen,
Y für ein Sauerstoffatom oder für S(O)ᵣ steht, wobei
r für die Zahlen 0, 1 oder 2 steht, und
n und p unabhängig voneinander für die Zahlen 0, 1 oder 2 stehen, wobei der durch (*) gekennzeichnete Molekülteil jeweils mit dem Phenylrest des Anilinteiles verbunden ist.
- X: steht auch vorzugsweise für Sauerstoff oder Schwefel.
- Z: steht vorzugsweise für Phenyl, Naphthyl oder Anthracenyl, wobei jeder dieser Reste einfach bis dreifach, gleichartig oder verschieden substituiert sein kann durch Halogen, Alkyl mit 1 bis 4 Kohlenstoffatomen, Alkylthio mit 1 bis 4 Kohlenstoffatomen, Halogenalkyl mit 1 bis 5 Fluor-, Chlor- und/oder Bromatomen, Halogenalkoxy mit 1 oder 2 Kohlenstoffatomen und 1 bis 5 Fluor-, Chlor- und/oder Bromatomen, Phenyl und/oder Phenoxy.
- Z: steht außerdem vorzugsweise für Hetaryl mit 5 oder 6 Ringgliedern und 1 bis 3 Heteroatomen, wie Sauerstoff, Schwefel und/oder Stickstoff, wobei jeder der Heterocyclen einfach oder zweifach, gleichartig oder verschieden substituiert sein kann durch Halogen, Cyano, Nitro, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 Fluor-, Chlor- und/oder Bromatomen, Halogenalkoxy mit 1 oder 2 Kohlenstoffatomen und 1 bis 5 Fluor-, Chlor- und/oder Bromatomen, Phenyl und/oder Phenoxy.
- R: steht besonders bevorzugt für Fluor, Chlor, Brom, Nitro, Cyano, Methyl, Ethyl, n-Propyl, Isopropyl, n-Butyl, sek.-Butyl, i-Butyl, tert.-Butyl, Trichlormethyl, Trifluormethyl, Difluormethyl, Difluorchlormethyl, Methoxy, Ethoxy, Difluormethoxy, Trifluormethoxy, Difluorchlormethylthio, Allyloxy, Propargyloxy, Cyclopropyl, Cyclopentyl, Cyclohexyl, Cycloheptyl, Methoxycarbonyl, Ethoxycarbonyl, Methoximinomethyl, Ethoximinomethyl, Methoximinoethyl oder Ethoximinoethyl.
- m: steht besonders bevorzugt für die Zahlen 0, 1, 2 oder 3, wobei R für gleiche oder verschiedene Reste steht, wenn m für 2 oder 3 steht.
- A: steht besonders bevorzugt für einen Rest der Formel 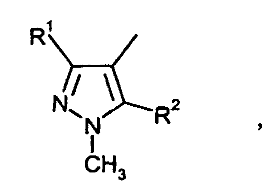 worin
R¹ für Fluor, Chlor, Brom, Iod, Methyl, Ethyl, Isopropyl, Difluormethyl, Trifluormethyl, Difluorchlormethyl oder Trichlormethyl steht und
R² für Wasserstoff, Fluor, Chlor, Brom, Iod, Methyl oder Ethyl steht.
- A: steht außerdem besonders bevorzugt für einen Rest der Formel 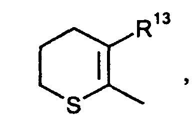 worin
R¹³ für Methyl, Ethyl, Trifluormethyl, Difluormethyl, Difluorchlormethyl oder Trichlormethyl steht.
- A: steht außerdem besonders bevorzugt für einen Rest der Formel 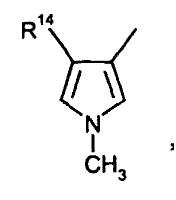 worin
R¹⁴ für Fluor, Chlor, Brom, Cyano, Methyl, Ethyl, Trifluormethyl, Difluormethyl, Difluorchlormethyl oder Trichlormethyl steht.
- A: steht außerdem besonders bevorzugt für einen Rest der Formel 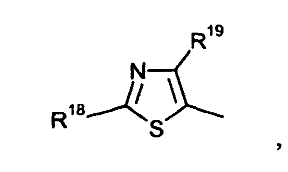 worin
R¹⁸ für Wasserstoff, Fluor, Chlor, Brom, Amino, Cyano, Methyl oder Ethyl steht und
R¹⁹ für Fluor, Chlor, Brom, Methyl, Ethyl, Trifluormethyl, Difluormethyl, Difluorchlormethyl oder Trichlormethyl steht.
- A: steht außerdem besonders bevorzugt für einen Rest der Formel 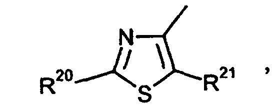 worin
R²⁰ für Wasserstoff, Fluor, Chlor, Brom, Amino, Cyano, Methyl oder Ethyl steht und
R²¹ für Fluor, Chlor, Brom, Methyl, Ethyl, Trifluormethyl, Difluormethyl, Difluorchlormethyl oder Trichlormethyl steht.
- A: steht außerdem besonders bevorzugt für einen Rest der Formel 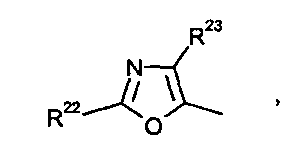 worin
R²² für Wasserstoff Methyl oder Ethyl steht und
R²³ für Fluor, Chlor, Brom, Methyl oder Ethyl steht.
- A: steht außerdem besonders bevorzugt für einen Rest der Formel 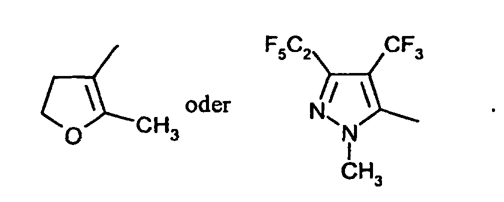
- Q: steht besonders bevorzugt für Alkylen mit 1 bis 3 Kohlenstoffatomen, Alkenylen mit 2 oder 3 Kohlenstoffatomen, Alkinylen mit 2 oder 3 Kohlenstoffatomen oder für eine Gruppe der Formel 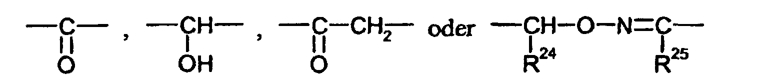 , worin
R²⁴ und R²⁵ unabhängig voneinander für Wasserstoff, Methyl, Ethyl, Cyclopropyl, Cyclopentyl, Cyclohexyl, Allyl oder Propargyl stehen.
- Q: steht außerdem besonders bevorzugt für eine Gruppe der Formel 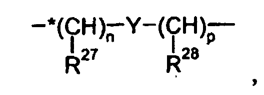 worin
R²⁷ und R²⁸ unabhängig voneinander für Wasserstoff, Methyl oder Ethyl stehen,
Y für ein Sauerstoffatom oder für S(O)ᵣ steht, wobei
r für die Zahlen 0, 1 oder 2 steht, und
n und p unabhängig voneinander für die Zahlen 0, 1 oder 2 stehen, wobei der durch (*) gekennzeichnete Molekülteil jeweils mit dem Phenylrest des Anilinteiles verbunden ist.
- X: steht auch besonders bevorzugt für Sauerstoff oder Schwefel.
- Z: steht besonders bevorzugt für Phenyl, Naphthyl oder Anthracenyl, wobei jeder dieser Reste einfach bis dreifach, gleichartig oder verschieden substituiert sein kann durch Fluor, Chlor, Brom, Methyl, Ethyl, n-Propyl, Isopropyl, n-Butyl, sek.-Butyl, i-Butyl, tert.-Butyl, Methoxy, Ethoxy, Methylthio, Ethylthio, Trifluormethyl, Difluormethyl, Difluorchlormethyl, Trichlormethyl, Trifluormethoxy, Difluormethoxy, Difluorchlormethoxy, Trichlormethoxy, Phenyl und/oder Phenoxy.
- Z: steht außerdem besonders bevorzugt für Pyrrolyl, Furyl, Pyrazolyl, Imidazolyl, Thiazolyl, Isothiazolyl, Oxazolyl, Isoxazolyl, Oxadiazolyl, Thiadiazolyl, 1,2,3-Triazinyl, 1,2,4-Triazinyl, 1,3,5-Triazinyl, Pyridyl, Pyrimidyl, Pyrazinyl oder Pyridazinyl, wobei jeder dieser Reste einfach oder zweifach, gleichartig oder verschieden substituiert sein kann durch Fluor, Chlor, Brom, Cyano, Nitro, Methyl, Ethyl, n-Propyl, Isopropyl, n-Butyl, sek.-Butyl, i-Butyl, tert.-Butyl, Methoxy, Ethoxy, Methylthio, Ethylthio, Trifluormethyl, Difluormethyl, Difluorchlormethyl, Trichlormethyl, Trifluormethoxy, Difluormethoxy, Difluorchlormethoxy, Trichlormethoxy, Phenyl und/oder Phenoxy.
- Z: steht außerdem vorzugsweise für Hetaryl mit 5 oder 6 Ringgliedern und 1 bis 3 Heteroatomen, wie Sauerstoff, Schwefel und/oder Stickstoff, wobei jeder der Heterocyclen einfach oder zweifach, gleichartig oder verschieden substituiert sein kann durch Halogen, Cyano, Nitro, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 Fluor-, Chlor- und/oder Bromatomen, Halogenalkoxy mit 1 oder 2 Kohlenstoffatomen und 1 bis 5 Fluor-, Chlor- und/oder Bromatomen, Phenyl und/oder Phenoxy.
- R: steht besonders bevorzugt für Fluor, Chlor, Brom, Nitro, Cyano, Methyl, Ethyl, n-Propyl, Isopropyl, n-Butyl, sek.-Butyl, i-Butyl, tert.-Butyl, Trichlormethyl, Trifluormethyl, Difluormethyl, Difluorchlormethyl, Methoxy, Ethoxy, Difluormethoxy, Trifluormethoxy, Difluorchlormethylthio, Allyloxy, Propargyloxy, Cyclopropyl, Cyclopentyl, Cyclohexyl, Cycloheptyl, Methoxycarbonyl, Ethoxycarbonyl, Methoximinomethyl, Ethoximinomethyl, Methoximinoethyl oder Ethoximinoethyl.
- m: steht besonders bevorzugt für die Zahlen 0, 1, 2 oder 3, wobei R für gleiche oder verschiedene Reste steht, wenn m für 2 oder 3 steht.
- A: steht besonders bevorzugt für einen Rest der Formel 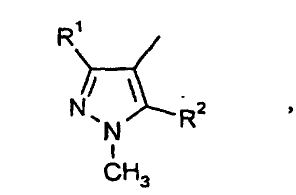 worin
R¹ für Fluor, Chlor, Brom, Iod, Methyl, Ethyl, Isopropyl, Difluormethyl, Trifluormethyl, Difluorchlormethyl oder Trichlormethyl steht und
R² für Wasserstoff, Fluor, Chlor, Brom, Iod, Methyl oder Ethyl steht.
- A: steht außerdem besonders bevorzugt für einen Rest der Formel 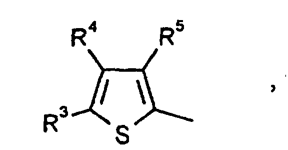 worin
R³ und R⁴ unabhängig voneinander für Wasserstoff, Fluor, Chlor, Brom, Methyl, Ethyl, Trifluormethyl, Difluormethyl, Difluorchlormethyl oder Trichlormethyl stehen und
R⁵ für Fluor, Chlor, Brom, Cyano, Methyl, Ethyl, Trifluormethoxy, Difluormethoxy, Difluorchlormethoxy oder Trichlormethoxy steht.
- A: steht außerdem besonders bevorzugt für einen Rest der Formel 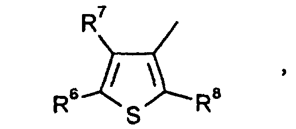 worin
R⁶ und R⁷ unabhängig voneinander für Wasserstoff, Fluor, Chlor, Brom, Methyl, Ethyl, Trifluormethyl, Difluormethyl, Difluorchlormethyl oder Trichlormethyl stehen und
R⁸ für Wasserstoff, Fluor, Chlor, Brom, Methyl oder Ethyl steht.
- A: steht außerdem besonders bevorzugt für einen Rest der Formel 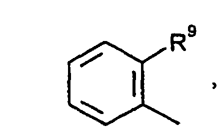 worin
R⁹ für Fluor, Chlor, Brom, Iod, Cyano, Methyl, Ethyl, n-Propyl, isopropyl, n-Butyl, i-Butyl, sek.-Butyl, tert.-Butyl, Trifluormethyl, Difluormethyl, Difluorchlormethyl, Trichlormethyl, Trifluormethoxy, Difluormethoxy, Difluorchlormethoxy, Trichlormethoxy, Trifluormethylthio, Difluormethylthio, Difluorchlormethylthio oder Trichlormethylthio steht.
- A: steht außerdem besonders bevorzugt für einen Rest der Formel 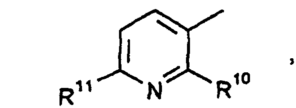 worin
R¹⁰ für Fluor, Chlor, Brom, Iod, Cyano, Methyl, Ethyl, n-Propyl, Isopropyl, n-Butyl, i-Butyl, sek.-Butyl, tert.-Butyl, Trifluormethyl, Difluormethyl, Difluorchlormethyl, Trichlormethyl, Methoxy, Ethoxy, Methylthio, Ethylthio, Trifluormethoxy, Difluormethoxy, Difluorchlormethoxy oder Trichlormethoxy steht und
R¹¹ für Wasserstoff, Fluor, Chlor, Brom, Iod, Cyano, Methyl, Ethyl, n-Propyl, Isopropyl, n-Butyl, i-Butyl, sek.-Butyl, tert.-Butyl, Trifluormethyl, Difluormethyl, Difluorchlormethyl, Trichlormethyl, Methoxy, Ethoxy, Methylthio, Ethylthio, Trifluormethoxy, Difluormethoxy, Difluorchlormethoxy oder Trichlormethoxy steht.
- A: steht außerdem besonders bevorzugt für einen Rest der Formel 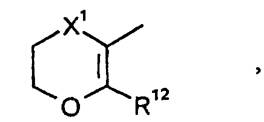 worin
R¹² für Methyl, Ethyl, Trifluormethyl, Difluormethyl, Difluorchlormethyl oder Trichlormethyl steht und
X¹ für ein Schwefelatom, für SO, SO₂ oder -CH₂- steht.
- A: steht außerdem besonders bevorzugt für einen Rest der Formel 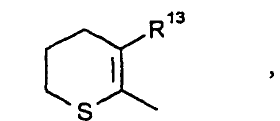 worin
R¹³ für Methyl, Ethyl, Trifluormethyl, Difluormethyl, Difluorchlormethyl oder Trichlormethyl steht.
- A: steht außerdem besonders bevorzugt für einen Rest der Formel 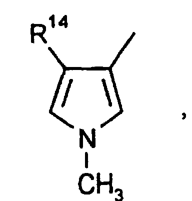 worin
R¹⁴ für Fluor, Chlor, Brom, Cyano, Methyl, Ethyl, Trifluormethyl, Difluormethyl, Difluorchlormethyl oder Trichlormethyl steht.
- A: steht außerdem besonders bevorzugt für einen Rest der Formel 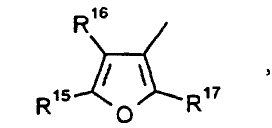 worin
R¹⁵ und R¹⁶ unabhängig voneinander für Wasserstoff, Fluor, Chlor, Brom, Methyl, Ethyl, Trifluormethyl, Difluormethyl, Difluorchlormethyl oder Trichlormethyl stehen und
R¹⁷ für Wasserstoff, Fluor, Chlor, Brom, Methyl oder Ethyl steht.
- A: steht außerdem besonders bevorzugt für einen Rest der Formel 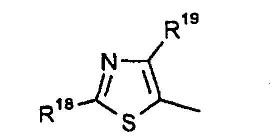 worin
R¹⁸ für Wasserstoff, Fluor, Chlor, Brom, Amino, Cyano, Methyl oder Ethyl steht und
R¹⁹ für Fluor, Chlor, Brom, Methyl, Ethyl, Trifluormethyl, Difluormethyl, Difluorchlormethyl oder Trichlormethyl steht.
- A: steht außerdem besonders bevorzugt für einen Rest der Formel 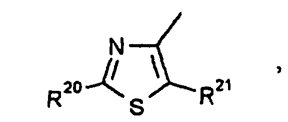 worin
R²⁰ für Wasserstoff, Fluor, Chlor, Brom, Amino, Cyano, Methyl oder Ethyl steht und
R²¹ für Fluor, Chlor, Brom, Methyl, Ethyl, Trifluormethyl, Difluormethyl, Difluorchlormethyl oder Trichlormethyl steht.
- A: steht außerdem besonders bevorzugt für einen Rest der Formel 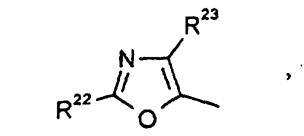 worin
R²² für Wasserstoff, Methyl oder Ethyl steht und
R²³ für Fluor, Chlor, Brom, Methyl oder Ethyl steht.
- A: steht außerdem besonders bevorzugt für einen Rest der Formel 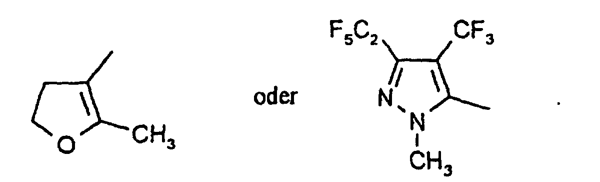
- Q: steht besonders bevorzugt für Alkylen mit 1 bis 3 Kohlenstoffatomen, Alkenylen mit 2 oder 3 Kohlenstoffatomen, Alkinylen mit 2 oder 3 Kohlenstoffatomen oder für eine Gruppe der Formel 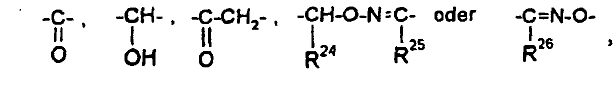 worin
R²⁴, R²⁵ und R²⁶ unabhängig voneinander für Wasserstoff, Methyl, Ethyl, Cyclopropyl, Cyclopentyl, Cyclohexyl, Allyl oder Propargyl stehen.
- Q: steht außerdem besonders bevorzugt für eine Gruppe der Formel 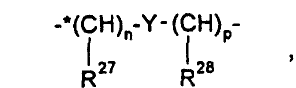 worin
R²⁷ und R²⁸ unabhängig voneinander für Wasserstoff, Methyl oder Ethyl stehen,
Y für ein Sauerstoffatom oder für S(O)ᵣ steht, wobei
r für die Zahlen 0, 1 oder 2 steht, und
n und p unabhängig voneinander für die Zahlen 0, 1 oder 2 stehen, wobei der durch (*) gekennzeichnete Molekülteil jeweils mit dem Phenylrest des Anilinteiles verbunden ist.
- X: steht auch besonders bevorzugt für Sauerstoff oder Schwefel.
- Z: steht besonders bevorzugt für Phenyl, Naphthyl oder Anthracenyl, wobei jeder dieser Reste einfach bis dreifach, gleichartig oder verschieden substituiert sein kann durch Fluor, Chlor, Brom, Methyl, Ethyl, n-Propyl, Isopropyl, n-Butyl, sek.-Butyl, i-Butyl, tert.-Butyl, Methoxy, Ethoxy, Methylthio, Ethylthio, Trifluormethyl, Difluormethyl, Difluorchlormethyl, Trichlormethyl, Trifluormethoxy, Difluormethoxy, Difluorchlormethoxy, Trichlormethoxy, Phenyl und/oder Phenoxy.
- Z: steht außerdem besonders bevorzugt für Pyrrolyl, Furyl, Pyrazolyl, Imidazolyl, Thiazolyl, Isothiazolyl, Oxazolyl, Isoxazolyl, Oxadiazolyl, Thiadiazolyl, 1,2,3-Triazinyl, 1,2,4-Triazinyl, 1,3,5-Triazinyl, Pyridyl, Pyrimidyl, Pyrazinyl oder Pyridazinyl, wobei jeder dieser Reste einfach oder zweifach, gleichartig oder verschieden substituiert sein kann durch Fluor, Chlor, Brom, Cyano, Nitro, Methyl, Ethyl, n-Propyl, Isopropyl, n-Butyl, sek.-Butyl, i-Butyl, tert.-Butyl, Methoxy, Ethoxy, Methylthio, Ethylthio, Trifluormethyl, Difluormethyl, Difluorchlormethyl, Trichlormethyl, Trifluormethoxy, Difluormethoxy, Difluorchlormethoxy, Trichlormethoxy, Phenyl und/oder Phenoxy.

Verwendet man 3-Difluormethoxy-thiophen-2-carbonsäurechlorid und 2-(2-Phenylethen-1-yl)-anilin als Ausgangsstoffe, so kann der Verlauf des erfindungsgemäßen Verfahrens (a) durch das folgende Formelschema veranschaulicht werden.

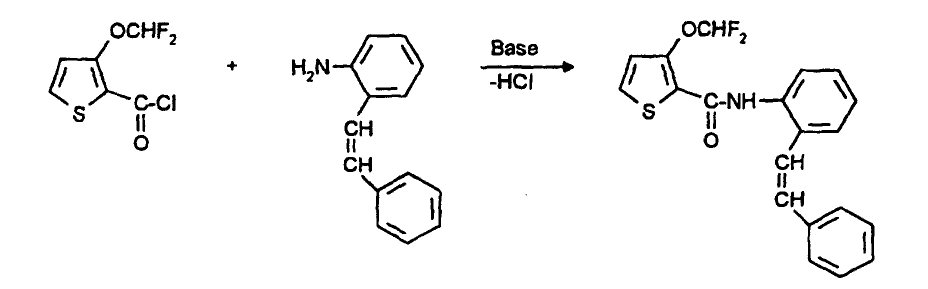

Verwendet man 1-Methyl-3-trifluormethyl-pyrazol-4-carbonsäure-(2-hydroxy)-anilid und 2,4-Dimethyl-benzylchlorid als Ausgangsstoffe, so kann der Verlauf des erfindungsgemäßen Verfahrens (b) durch das folgende Formelschema veranschaulicht werden.

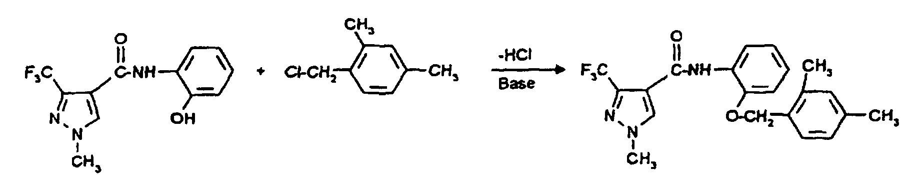

Verwendet man 1-Methyl-3-trifluormethyl-pyrazol-4-carbonsäure-(2-brommethyl)-anilid und 4-Chlor-thiophenol als Ausgangsstoffe, so kann der Verlauf des erfindungsgemäßen Verfahrens (c) durch das folgende Formelschema veranschaulicht werden.

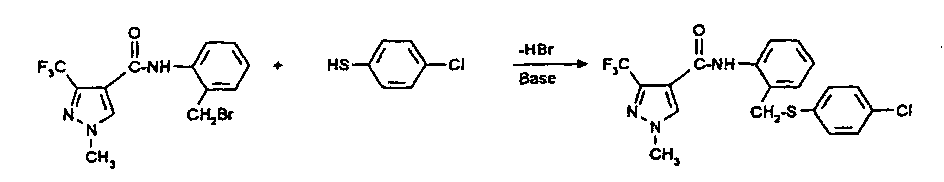

Verwendet man 1-Methyl-3-trifluormethyl-pyrazol-4-carbonsäure-(2-hydroxymethyl)-anilid und 2,4-Dimethylbenzylchlorid als Ausgangsstoffe, so kann der Verlauf des erfindungsgemäßen Verfahrens (d) durch das folgende Formelschema veranschaulicht werden.

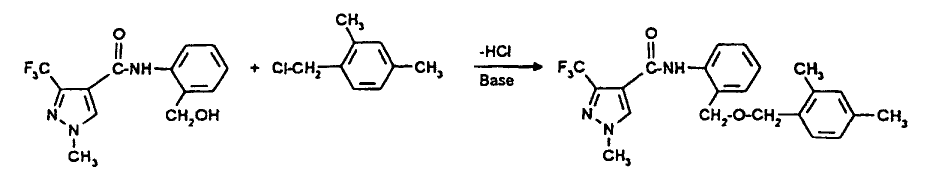

Die bei der Durchführung des erfindungsgemäßen Verfahrens (a) als Ausgangsstoffe benötigten Säurehalogenide sind durch die Formel (II) allgemein definiert. In dieser Formel haben A und X vorzugsweise diejenigen Bedeutungen, die bereits im Zusammenhang mit der Beschreibung der erfindungsgemäßen Stoffe der Formel (I) für diese Reste als bevorzugt genannt wurden. Hal steht vorzugsweise für Fluor, Chlor oder Brom.

Die Säurehalogenide der Formel (II) sind bekannt oder lassen sich nach bekannten Methoden herstellen (vgl. WO 93-11 117, EP-A 0 545 099, EP-A 0 589 301 und EP-A 0 589 313).

Die bei der Durchführung des erfindungsgemäßen Verfahrens (a) als Reaktionskomponenten benötigten Anilin-Derivate sind durch die Formel (III) allgemein definiert. In dieser Formel haben Q, R, Z und m vorzugsweise diejenigen Bedeutungen, die bereits im Zusammenhang mit der Beschreibung der erfindungsgemäßen Stoffe der Formel (I) vorzugsweise für diese Reste bzw. diesen Index genannt wurden.

Die Anilin-Derivate der Formel (III) sind bekannt oder lassen sich nach bekannten Methoden herstellen (vgl. WO 93-11 117, EP-A 0 545 099, EP-A 0 589 301, EP-A 0 371 950 und EP-A 0 292 990).

Als Säurebindemittel kommen bei der Durchführung des erfindungsgemäßen Verfahrens (a) alle für derartige Reaktionen üblichen anorganischen und organischen Basen in Betracht. Vorzugsweise verwendbar sind Erdalkali- oder Alkalimetallhydroxide, wie Natriumhydroxid, Calciumhydroxid, Kaliumhydroxid, oder auch Ammoniumhydroxid, Alkalimetallcarbonate, wie Natriumcarbonat, Kaliumcarbonat, Kaliumhydrogencarbonat, Natriumhydrogencarbonat, Alkali- oder Erdalkalimetallacetate wie Natriumacetat, Kaliumacetat, Calciumacetat, sowie tertiäre Amine, wie Trimethylamin, Triethylamin, Tributylamin, N,N-Dimethylanilin, Pyridin, N-Methylpiperidin, N,N-Dimethylaminopyridin, Diazabicyclooctan (DABCO), Diazabicyclononen (DBN) oder Diazabicycloundecen (DBU). Es ist jedoch auch möglich, ohne zusätzliches Säurebindemittel zu arbeiten, oder die Aminkomponente in einem Überschuß einzusetzen, so daß sie gleichzeitig als Säurebindemittel fungiert.

Als Verdünnungsmittel kommen bei der Durchführung des erfindungsgemäßen Verfahrens (a) alle üblichen inerten, organischen Solventien in Frage. Vorzugsweise verwendbar sind gegebenenfalls halogenierte aliphatische, alicyclische oder aromatische Kohlenwasserstoffe, wie Petrolether, Hexan, Heptan, Cyclohexan, Methylcyclohexan, Benzol, Toluol, Xylol oder Decalin; Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methylt-amylether, Dioxan, Tetrahydrofuran, 1,2-Dimethoxyethan, 1,2-Diethoxyethan oder Anisol; Nitrile, wie Acetoninil, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester; Sulfoxide, wie Dimethylsulfoxid oder Sulfone, wie Sulfolan.

Die Reaktionstemperaturen können bei der Durchführung des erfindungsgemäßen Verfahrens (a) in einem größeren Bereich variiert werden. Im allgemeinen arbeitet man bei Temperaturen zwischen 0°C und 120°C, vorzugsweise zwischen 10°C und 100°C.

Sowohl bei der Durchführung des erfindungsgemäßen Verfahrens (a) als auch der erfindungsgemäßen Verfahren (b) bis (d) arbeitet man im allgemeinen unter Atmosphärendruck. Es ist aber auch möglich, jeweils unter erhöhtem oder vermindertem Druck zu arbeiten.

Bei der Durchführung des erfindungsgemäßen Verfahrens (a) setzt man auf 1 Mol an Säurehalogenid der Formel (II) im allgemeinen 1 Mol oder auch einen Überschuß an Anilin-Derivat der Formel (III) sowie 1 bis 3 Mol an Säurebindemittel ein. Es ist jedoch auch möglich, die Reaktionskomponenten in anderen Verhältnissen einzusetzen. Die Aufarbeitung erfolgt nach üblichen Methoden. Im allgemeinen verfährt man in der Weise, daß man das Reaktionsgemisch mit Wasser versetzt, die organische Phase abtrennt und nach dem Trocknen unter vermindertem Druck einengt. Der verbleibende Rückstand kann gegebenenfalls nach üblichen Methoden, wie Chromatographie oder Umkristallisation, von eventuell noch vorhandenen Verunreinigungen befreit werden.

Die bei der Durchführung des erfindungsgemäßen Verfahrens (b) als Ausgangsstoffe benötigten Carbanilid-Derivate sind durch die Formel (IV) allgemein definiert. In dieser Formel haben A, R, X und m vorzugsweise diejenigen Bedeutungen, die bereits im Zusammenhang mit der Beschreibung der erfindungsgemäßen Stoffe der Formel (I) vorzugsweise für diese Reste bzw. diesen Index genannt wurden. X² steht auch vorzugsweise für Sauerstoff oder Schwefel.

Die Carbanilid-Derivate der Formel (IV) sind bekannt oder lassen sich nach bekannten Methoden herstellen. So erhält man Verbindungen der Formel (IV), wenn man Säurehalogenide der Formel 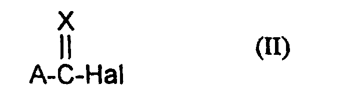 in welcher
A, X und Hal die oben angegebenen Bedeutungen haben,
mit Amino-phenolen bzw. Aminothiophenolen der Formel 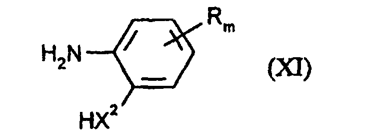 in welcher
R, X² und m die oben angegebenen Bedeutungen haben,
gegebenenfalls in Gegenwart eines Säurebindemittels, wie z.B. Kaliumcarbonat oder eines tertiären Amins, sowie gegebenenfalls in Gegenwart eines inerten organischen Verdünnungsmittels, wie z.B. Toluol, bei Temperaturen zwischen 20°C und 150°C umsetzt.

Die bei der Durchführung des obigen Verfahrens als Reaktionskomponenten benötigten Amino-phenole bzw. Aminothiophenole der Formel (XI) sind bekannt oder lassen sich nach bekannten Methoden herstellen.

Die bei der Durchführung des erfindungsgemäßen Verfahrens (b) als Reaktionskomponenten benötigten Verbindungen sind durch die Formel (V) allgemein definiert. In dieser Formel haben R²⁸, Z und p vorzugsweise diejenigen Bedeutungen, die bereits im Zusammenhang mit der Beschreibung der erfindungsgemäßen Stoffe der Formel (I) vorzugsweise für diese Reste bzw. diesen Index genannt wurden. E steht vorzugsweise für Chlor, Brom, Iod, Methylsulfonyloxy, Tolylsulfonyloxy oder einen Rest der Formel R²⁹-O-SO₂-O- oder R²⁹-O-CO-O-, worin R²⁹ für Alkyl mit 1 bis 4 Kohlenstoffatomen, vorzugsweise für Methyl oder Ethyl steht.

Die Verbindungen der Formel (V) sind ebenfalls bekannt oder lassen sich nach bekannten Methoden herstellen.

Als Säurebindemittel kommen bei der Durchführung des erfindungsgemäßen Verfahrens (b) alle für derartige Umsetzungen üblichen anorganischen und organischen Basen in Frage. Vorzugsweise verwendbar sind Erdalkalimetall- oder Alkalimetallhydride, -hydroxide, -amide, -alkoholate, -acetate, -carbonate oder - hydrogencarbonate, wie beispielsweise Natriumhydrid, Natriumamid, Natriummethylat, Natrium-ethylat, Kalium-tert.-butylat, Natriumhydroxid, Kaliumhydroxid, Natriumacetat, Kaliumacetat, Calciumacetat, Natriumcarbonat, Kaliumcarbonat, Kaliumhydrogencarbonat oder Natriumhydrogencarbonat, ferner AmmoniumVerbindungen, wie Ammoniumhydroxid, Ammoniumacetat oder Ammoniumcarbonat, und außerdem tertiäre Amine, wie Trimethylamin, Triethylamin, Tributylamin, N,N-Dimethylanilin, N,N-Dimethylbenzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Als Verdünnungsmittel kommen bei der Durchführung des erfindungsgemäßen Verfahrens (b) alle üblichen inerten organischen Solventien in Betracht. Vorzugsweise verwendbar sind alle diejenigen Verdünnungsmittel, die bereits im Zusammenhang mit der Beschreibung des erfindungsgemäßen Verfahrens (a) vorzugsweise genannt wurden.

Die Reaktionstemperaturen können auch bei der Durchführung des erfindungsgemäßen Verfahrens (b) innerhalb eines größeren Bereiches variiert werden. Im allgemeinen arbeitet man bei Temperaturen zwischen -20°C und +150°C, vorzugsweise zwischen -10°C und +120°C.

Bei der Durchführung des erfindungsgemäßen Verfahrens (b) setzt man auf 1 Mol an Carbanilid-Derivat der Formel (IV) im allgemeinen 1 bis 2 Mol an einer Verbindung der Formel (V) sowie gegebenenfalls eine äquivalente Menge oder auch einen Überschuß an Säurebindemittel ein. Die Aufarbeitung erfolgt nach üblichen Methoden. Im allgemeinen verfährt man in der Weise, daß man das Reaktionsgemisch mit Wasser versetzt, die organische Phase abtrennt und nach dem, Trocknen unter vermindertem Druck einengt. Verwendet man bei der Durchführung der Umsetzung ein mit Wasser mischbares Solvens, so fällt das gewünschte Produkt beim Verdünnen des Reaktionsgemisches mit Wasser im allgemeinen als Feststoff an. In diesem Fall erfolgt die Isolierung in der Regel durch einfaches Absaugen. Das jeweils erhaltene Produkt kann gegebenenfalls nach üblichen Methoden, wie Chromatographie oder Umkristallisation, von eventuell noch vorhandenen Verunreinigungen befreit werden.

Die bei der Durchführung des erfindungsgemäßen Verfahrens (c) als Ausgangsstoffe benötigten Carbanilid-Derivate sind durch die Vormel (VI) allgemein definiert. In dieser Formel haben A, R, R²⁷, X und m vorzugsweise diejenigen Bedeutungen, die bereits im Zusammenhang mit der Beschreibung der erfindungsgemäßen Stoffe der Formel (I) vorzugsweise für diese Reste bzw. diesen Index genannt wurden. E¹ steht vorzugsweise für Chlor, Brom, Iod, Methylsulfonyloxy, Tolylsulfonyloxy oder einen Rest der Formel R²⁹-O-SO₂-O- oder R²⁹-O-CO-O- worin R²⁹ für Alkyl mit 1 bis 4 Kohlenstoffatomen, vorzugsweise für Methyl oder Ethyl steht.

Die Carbanilid-Derivate der Formel (VI) sind bekannt oder lassen sich nach bekannten Methoden herstellen.

Die bei der Durchführung des erfindungsgemäßen Verfahrens (c) als Reaktionskomponenten benötigten Verbindungen sind durch die Formel (VII) allgemein definiert. In dieser Formel haben R²⁸, Z und p vorzugsweise diejenigen Bedeutungen, die bereits im Zusammenhang mit der Beschreibung der erfindungsgemäßen Stoffe der Formel (I) vorzugsweise für diese Reste bzw. diesen Index genannt wurden. X³ steht auch vorzugsweise für Sauerstoff oder Schwefel.

Die Verbindungen der Formel (VIII) sind ebenfalls bekannt oder lassen sich nach bekannten Methoden herstellen.

Als Säurebindemittel kommen bei der Durchführung des erfindungsgemäßen Verfahrens (c) alle für derartige Umsetzungen üblichen anorganischen und organischen Basen in Frage. Vorzugsweise verwendbar sind alle diejenigen Säureakzeptoren, die bereits im Zusammenhang mit der Beschreibung des erfindungsgemäßen Verfahrens (b) vorzugsweise genannt wurden.

Als Verdünnungsmittel kommen bei der Durchführung des erfindungsgemäßen Verfahrens (c) Wasser sowie alle üblichen inerten, organischen Solventien in Betracht. Vorzugsweise verwendbar sind aliphatische, alicyclische oder aromatische, gegebenenfalls halogenierte Kohlenwasserstoffe, wie Benzin, Benzol, Toluol, Xylol, Chlorbenzol, Dichlorbenzol, Petrolether, Hexan, Cyclohexan, Dichlormethan, Chloroform, Tetrachlormethan, Ether, wie Diethylether, Diisopropylether, Dioxan, Tetrahydrofuran oder Ethylenglykol-dimethyl- oder -diethylether; Ketone, wie Aceton, Butanon oder Methyl-isobutyl-keton; Nitrile, wie Acetonitril, Propionitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester, Sulfoxide, wie Dimethylsulfoxid, Alkohole, wie Methanol, Ethanol, n- oder i-Propynol, Ethylenglykol-monomethylether, Ethylenglykolmonoethylether, Diethylenglykolmonomethylether, Diethylenglykolmonoethylether, deren Gemische mit Wasser oder reines Wasser.

Die Reaktionstemperaturen können auch bei der Durchführung des erfindungsgemäßen Verfahrens (c) innerhalb eines größeren Bereiches variiert werden. Im allgemeinen arbeitet man bei Temperaturen zwischen -20°C und +150°C, vorzugsweise zwischen -10°C und +120°C.

Bei der Durchführung des erfindungsgemäßen Verfahrens (c) setzt man auf 1 Mol an Carbanilid-Derivat der Formel (VI) im allgemeinen 1 bis 2 Mol an einer Verbindung der Formel (VII) sowie gegebenenfalls eine äquivalente Menge oder auch einen Überschuß an Säurebindemittel ein. Die Aufarbeitung erfolgt nach üblichen Methoden.

Die bei der Durchführung des erfindungsgemäßen Verfahrens (d) als Ausgangsstoffe benötigten Carbanilid-Derivate sind durch die Formel (VIII) allgemein definiert. In dieser Formel haben A, R, R²⁷, X und m vorzugsweise diejenigen Bedeutungen, die bereits im Zusammenhang mit der Beschreibung der erfindungsgemäßen Stoffe der Formel (I) vorzugsweise für diese Reste bzw. diesen Index genannt wurden.

Die Carbanilid-Derivate der Formel (VIII) sind bekannt oder lassen sich nach bekannten Methoden herstellen.

Als Säurebindemittel kommen bei der Durchführung des erfindungsgemäßen Verfahrens (d) alle für derartige Umsetzungen üblichen anorganischen und organischen Basen in Frage. Vorzugsweise verwendbar sind alle diejenigen Säureakzeptoren, die bereits im Zusammenhang mit der Beschreibung des erfindungsgemäßen Verfahrens (b) vorzugsweise genannt wurden.

Als Verdünnungsmittel kommen bei der Durchführung des erfindungsgemäßen Verfahrens (d) alle üblichen inerten organischen Solventien in Betracht. Vorzugsweise verwendbar sind alle diejenigen Verdünnungsmittel, die bereits im Zusammenhang mit der Beschreibung des erfindungsgemäßen Verfahrens (a) vorzugsweise genannt wurden.

Die Reaktionstemperaturen können auch bei der Durchführung des erfindungsgemäßen Verfahrens (d) innerhalb eines größeren Bereiches variiert werden. Im allgemeinen arbeitet man bei Temperaturen zwischen -20°C und +150°C, vorzugsweise zwischen -10°C und +120°C.

Bei der Durchführung des erfindungsgemäßen Verfahrens (d) setzt man auf 1 Mol an Carbanilid-Derivat der Formel (VIII) im allgemeinen 1 bis 2 Mol an einer Verbindung der Formel (V) sowie gegebenenfalls eine äquivalente Menge oder auch einen Überschuß an Säurebindemittel ein. Die Aufarbeitung erfolgt nach üblichen Methoden.

Die erfindungsgemäßen Stoffe weisen eine starke mikrobizide Wirkung auf und können zur Bekämpfung von unerwünschten Mikroorganismen, wie Fungi und Bakterien, im Pflanzenschutz und im Materialschutz eingesetzt werden.

Fungizide lassen sich Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Bakterizide lassen sich im Pflanzenschutz zur Bekämpfung von Pseudomonadaceae, Rhizobiaceae, Enterobacteriaceae, Corynebacteriaceae und Streptomycetaceae einsetzen.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Bremia-Arten, wie beispielsweise Bremia lactucae;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea (Konidienform: Drechslera, Syn: Helminthosporium);
Cochliobolus-Arten, wie beispielsweise Cochliobolus sativus (Konidienform: Drechslera, Syn: Helminthosporium);
Uromyces-Arten, wie beispielsweise Uromyces appendiculatus;
Puccinia-Arten, wie beispielsweise Puccinia recondita;
Sclerotinia-Arten, wie beispielsweise Sclerotinia sclerotiorum;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Krankheiten im Wein-, Obst- und Gemüseanbau, wie beispielsweise gegen Venturia-, Podosphaera-, Phytophtora- und Plasmopara-Arten, einsetzen. Mit gutem Erfolg werden auch Reiskrankheiten, wie beispielsweise Pyricularia-Arten, bekämpft.

Im Materialschutz lassen sich die erfindungsgemäßen Stoffe zum Schutz von technischen Materialien gegen Befall und Zerstörung durch unerwünschte Mikroorganismen einsetzen.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten) sowie gegen Schleimorganismen und Algen.

Es seien beispielsweise Mikroorganismen der folgenden Gattungen genannt:
Alternaria, wie Alternaria tenuis,
Aspergillus, wie Aspergillus niger,
Chaetomium, wie Chaetomium globosum,
Coniophora, wie Coniophora puetana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Die erfindungsgemäßen Wirkstoffe eignen sich bei guter Pflanzenverträglichkeit und günstiger Warmblütertoxizität auch zur Bekämpfung von tierischen Schädlingen, insbesondere von Insekten, Spinnentieren und Nematoden, die in der Landwirtschaft, in Forsten, im Gartenbau, im Vorrats- und Materialschutz sowie auf dem Hygienesektor bzw. im veterinärmedizinischen Bereich vorkommen. Die Stoffe sind gegen normal sensible und resistente Arten sowie gegen Schädlinge in allen oder einzelnen Entwicklungsstadien wirksam. Zu den oben erwähnten tierischen Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.
Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp..

Aus der Ordnung der Anoplura z.B. Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Aphis pomi, Eriosoma lanigerum, Hyalopterus arundinis, Phylloxera vastatrix, Pemphigus spp., Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Spodoptera litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp.

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp.

Zu den pflanzenparasitären Nematoden gehören z.B. Pratylenchus spp., Radopholus similis, Ditylenchus dipsaci, Tylenchulus semipenetrans, Heterodera spp., Globodera spp., Meloidogyne spp., Aphelenchoides spp., Longidorus spp., Xiphinema spp., Trichodorus spp.

Die Wirkstoffe können in Abhängigkeit von ihren jeweiligen physikalischen und/oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen zum Beispiel folgende Verbindungen in Frage:

### Fungizide:

Aldimorph, Ampropylfos, Ampropylfos-Kalium, Andoprim, Anilazin, Azaconazol, Azoxystrobin,
Benalaxyl, Benodanil, Benomyl, Benzamacril, Benzamacryl-isobutyl, Bialaphos, Binapacryl, Biphenyl, Bitertanol, Blasticdin-S, Bromuconazol, Bupirimat, Buthiobat,
Calciumpolysulfid, Capsimycin, Captafol, Captan, Carbendazim, Carboxin, Carvon, Chinomethionat(Quinomethionat), Chlobenthiazon, Chlorfenazol, Chloroneb, Chloropicrin, Chlorothalonil, Chlozolinat, Clozylacon, Cufraneb, Cymoxanil, Cyproconazol, Cyprodinil, Cyprofuram,
Debacarb, Dichlorophen, Diclobutrazol, Diclofluanid, Diclomezin, Dicloran, Diethofencarb, Difenoconazol, Dimethirimol, Dimethomorph, Diniconazol, Diniconazol-M, Dinocap, Diphenylamin, Dipyrithione, Ditalimfos, Dithianon, Dodemorph, Dodine, Drazoxolon,
Ediphenphos, Epoxiconazol, Etaconazol, Ethirimol, Etridiazol,
Famoxadon, Fenapanil, Fenarimol, Fenbuconazol, Fenfuram, Fenitropan, Fenpiclonil, Fenpropidin, Fenpropimorph, Fentinacetat, Fentinhydroxyd, Ferbam, Ferimzon, Fluazinam, Flumetover, Fluoromid, Fluquinconazol, Flurprimidol, Flusilazol, Flusulfamid, Flutolanil, Flutriafol, Folpet, Fosetyl-Alminium, Fosetyl-Natrium, Fthalid, Fuberidazol, Furalaxyl, Furametpyr, Furcarbonil, Furconazol, Furconazol-cis, Furmecyclox,
Guazatin,
Hexachlorobenzol, Hexaconazol, Hymexazol,
Imazalil, Imibenconazol, Iminoctadin, Iminoctadinealbesilate, Iminoctadinetriacetat, Iodocarb, Ipconazol, Iprobenfos (IBP), Iprodione, Irumamycin, Isoprothiolan, Isovaledione,
Kasugamycin, Kresoxim-methyl, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
Mancopper, Mancozeb, Maneb, Meferimzone, Mepanipyrim, Mepronil, Metalaxyl, Metconazol, Methasulfocarb, Methfuroxam, Metiram, Metomeclam, Metsulfovax, Mildiomycin, Myclobutanil, Myclozolin,
Nickel-dimethyldithiocarbamat, Nitrothal-isopropyl, Nuarimol,
Ofurace, Oxadixyl, Oxamocarb, Oxolinicacid, Oxycarboxim, Oxyfenthiin,
Paclobutrazol, Pefurazoat, Penconazol, Pencycuron, Phosdiphen, Pimaricin, Piperalin, Polyoxin, Polyoxorim, Probenazol, Prochloraz, Procymidon, Propamocarb, Propanosine-Natrium, Propiconazol, Propineb, Pyrazophos, Pyrifenox, Pyrimethanil, Pyroquilon, Pyroxyfur,
Quinconazol, Quintozen (PCNB),
Schwefel und Schwefel-Zubereitungen,
Tebuconazol, Tecloftalam, Tecnazen, Tetcyclacis, Tetraconazol, Thiabendazol, Thicyofen, Thifluzamide, Thiophanate-methyl, Thiram, Tioxymid, Tolclofosmethyl, Tolylfluanid, Triadimefon, Triadimenol, Triazbutil, Triazoxid, Trichlamid, Tricyclazol, Tridemorph, Triflumizol, Triforin, Triticonazol,
Uniconazol,
Validamycin A, Vinclozolin, Viniconazol,
Zarilamid, Zineb, Ziram sowie
Dagger G,
OK-8705,
OK-8801,
2',6'-Dibrom-2-methyl-4'-trifluormethoxy-4'-trifluor-methyl-1,3-thiazol-5-carboxanilid,
2,6-Dichlor-N-(4-trifluormethylbenzyl)-benzamid,
2-Aminobutan,
2-Phenylphenol(OPP),
8-Hydroxychinolinsulfat,
cis-1-(4-Chlorphenyl)-2-(1H-1,2,4-triazol-1-yl)-cycloheptanol,
(5RS,6RS)-6-Hydroxy-2,2,7,7-tetramethyl-5-(1H-1,2,4-triazol-1-yl)-3-octanon,
α-(2,4-Dichlorphenyl)-β-methoxy-α-methyl-1H-1,2,4-triazol-1-ethanol,
α-(1,1-Dimethylethyl)-β-(2-phenoxyethyl)-1H-1,2,4-triazol-1-ethanol,
1-[1-[2-[(2,4-Dichlorphenyl)-methoxy]-phenyl]-ethenyl]-1H-imidazol,
bis-(1-Methylethyl)-3-methyl-4-[(3-methylbenzoyl)-oxy]-2,5-thiophendicarboxylat,
2,6-Dichlor-N-[[4-(trifluormethyl)-phenyl]-methyl]-benzamid,
(E)-α-(Methoxyimino)-N-methyl-2-phenoxy-phenylacetamid,
9H-Xanthen-9-carbonsäure-2-[(phenylamino)-carbonyl)-hydrazid,
O-Methyl-S-phenyl-phenylpropylphosphoramidothioate,
N-(5-Chlor-2-methylphenyl)-2-methoxy-N-(2-oxo-3-oxazolidinyl)-acetamid,
1-(2,4-Dichlorphenyl)-2-(1H-1,2,4-triazol-1-yl)-ethanon-O-(phenylmethyl)-oxim,
N-(2,6-Dimethylphenyl)-2-methoxy-N-(tetrahydro-2-oxo-3-thienyl)-acetamid,
cis-4-[3-[4-(1,1-Dimethylpropyl)-phenyl-2-methylpropyl)-2,6-dimethyl-morpholinhydrochlorid,
1-(3,5-Dichlorphenyl)-3-(2-propenyl)2,5-pyrrolidindion,
1-Methyl-5-nonyl-2-(phenylmethyl)-3-pyrrolidinol,
1-[[2-(4-Chlorphenyl)-3-phenyloxiranyl]-methyl]-1H-1,2,4-triazol,
Methantetrathiol-Natriumsalz,
2-(2,3,3-Triiod-2-propenyl)-2H-tetrazol,
N-[3-Chlor-4,5-bis(2-propinyloxy)-phenyl]-N'-methoxy-methanimidamid,
α-(5-Methyl-1,3-dioxan-5-yl)-β-[[4-(trifluormethyl)-phenyl]-methylen]-1H-1,2,4-triazol-1-ethanol,
1-(2-Methyl-1-naphthalenyl)-1H-pyrrol-2,5-dion,
N-(2,6-Dimethylphenyl)-2-methoxy-N-(tetrahydro-2-oxo-3-furanyl)-acetamid,
3,4-Dichlor-1-[4-(difluormethoxy)-phenyl]-1H-pyrrol-2,5-dion,
N-[2,2,2-Trichlor-1-[(chloracetyl)-amino]-ethyl]-benzamid, N-Formyl-N-hydroxy-DL-alanin-Natriumsalz,
N-(4-Cyclohexylphenyl)-1,4,5,6-tetrahydro-2-pyrimidinamin,
4-Methyl-tetrazolo[1,5-a]quinazolin-5(4H)-on,
2-Chlor-N-(2,6-dimethylphenyl)-N-(isothiocyanatomethyl)-acetamid,
Ethyl-[(4-chlorphenyl)-azo]-cyanoacetat,
N-(4-Hexylphenyl)-1,4,5,6-tetrahydro-2-pyrimidinamin,
N-(2-Chlor-4-nitrophenyl)-4-methyl-3-nitro-benzolsulfonamid,
Methyl-N-(chloracetyl)-N-(2,6-dimethylphenyl)-DL-alaninat,
3-[2-(4-Chlorphenyl)-5-ethoxy-3-isoxazolidinyl]-pyridin,
2-[(1-Methylethyl)sulfonyl]-5-(trichlormethyl)-1,3,4-thiadiazol,
spiro[2H]-1-Benzopyran-2,1'(3'H)-isobenzofuran]-3'-on,
Methyl-N-(2,6-dimethylphenylrN-(S-isoxazolylcarbonyl)-DL-alaninat,
Kaliumhydrogencarbonat,
1-[[2-(2,4-Dichlorphenyl)-1,3-dioxolan-2-yl]-methyl)-1H-imidazol,
1-[(Diiodmethyl)-sulfonyl]-4-methyl-benzol,
2-Brom-2-(brommethyl)-pentandinitril,
2-[[6-Deoxy-4-O-(4-O-methyl-β-D-glycopyranosyl)-α-D-glucopyranosyl]-amino]-4-methoxy-1H-pyrrolo[2,3-d]pyrimidin-5-carbonitril,
Methyl-1-(2,3-dihydro-2,2-dimethyl-1H-inden-1-yl)-1H-imidazol-5-carboxylat,
2-Chlor-N-(2,3-dihydro-1,1,3-trimethyl-1H-inden-4-yl)-3-pyridincarboxamid,
O,O-Diethyl-[2-(dipropylamino)-2-oxoethyl]-ethylphosphoramidothioat,
α-(2,4-Dichlorphenyl)-ß-fluor-ß-propyl-1H-1,2,4-triazol-1-ethanol,
3-(1,1-Dimethylpropyl-1-oxo-1H-inden-2-carbonitril,
2,6-Dichlor-5-(methylthio)-4-pyrimidinyl-thiocyanat,
S-Methyl-1,2,3-benzothiadiazol-7-carbothioat,
N-(6-Methoxy)-3-pyridinyl)-cyclopropancarboxamid,
3,5-Dichlor-N-[cyan-[(1-methyl-2-propynyl)-oxy]-methyl]-benzamid,
4-Chlor-2-cyan-N,N-dimethyl-5-(4-methylphenyl)-1H-imidazol-1-sulfonamid,
8-(1,1-Dimethylethyl)-N-ethyl-N-propyl-1,4-dioxaspiro[4.5]decan-2-methanamin,
2,2-Dichlor-N-[1-(4-chlorphenyl)-ethyl]-1-ethyl-3-methyl-cyclopropancarboxamid, N-(2,3-Dichlor-4-hydroxyphenyl)-1-methyl-cyclohexancarboxamid.

### Bakterizide:

Bronopol, Dichlorophen, Nitrapyrin, Nickel Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, AC 303 630, Acephat, Acrinathrin, Alanycarb, Aldicarb, Alphamethrin, Amitraz, Avermectin, AZ 60541, Azadirachtin, Azinphos A, Azinphos M, Azocyclotin,
Bacillus thuringiensis, Bendiocarb, Benfuracarb, Bensultap, Betacyluthrin, Bifenthrin, BPMC, Brofenprox, Bromophos A, Bufencarb, Buprofezin, Butocarboxim, Butylpyridaben,
Cadusafos, Carbaryl, Carbofuran, Carbophenothion, Carbosulfan, Cartap, CGA 157 419, CGA 184699, Chloethocarb, Chlorethoxyfos, Chlorfenvinphos, Chlorfluazuron, Chlormephos, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat,
Dimethylvinphos, Dioxathion, Disulfoton,
Edifenphos, Emamectin, Esfenvalerat, Ethiofencarb, Ethion, Ethofenprox, Ethoprophos, Etrimphos,
Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Flucycloxuron, Flucythrinat, Flufenoxuron, Flufenprox, Fluvalinate, Fonophos, Formothion, Fosthiazat, Fubfenprox, Furathiocarb,
HCH, Heptenophos, Hexaflumuron, Hexythiazox,
Imidacloprid, Iprobenfos, Isazophos, Isofenphos, Isoprocarb, Isoxathion, Ivermectin, Lambda-cyhalothrin, Lufenuron,
Malathion, Mecarbam, Mevinphos, Mesulfenphos, Metaldehyd, Methacrifos, Methamidophos, Methidathion, Methiocarb, Methomyl, Metolcarb, Milbemectin, Monocrotophos, Moxidectin,
Naled, NC 184, NI 25, Nitenpyram
Omethoat, Oxamyl, Oxydemethon M, Oxydeprofos,
Parathion A, Parathion M, Permethrin, Phenthoat, Phorat, Phosalon, Phosmet, Phosphamidon, Phoxim, Pirimicarb, Pirimiphos M, Pirimiphos A, Profenofos, Promecarb, Propaphos, Propoxur, Prothiofos, Prothoat, Pymetrozin, Pyrachlophos, Pyridaphenthion, Pyresmethrin, Pyrethrum, Pyridaben, Pyrimidifen, Pyriproxifen, Quinalphos,
RH 5992,
Salithion, Sebufos, Silafluofen, Sulfotep, Sulprofos,
Tebufenozid, Tebufenpyrad, Tebupirimiphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
Vamidothion, XMC, Xylylcarb, Zetamethrin.

Auch eine Mischung mit anderen bekannten Wirkstoffen, wie Herbiziden oder mit Düngemitteln und Wachstumsregulatoren ist möglich.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden: Sie liegen im allgemeinen zwischen 1 und 0,0001 Gew.-%, vorzugsweise zwischen 0,5 und 0,001 Gew.-%.

Bei der Saatgutbehandlung werden im allgemeinen Wirkstoffmengen von 0,001 bis 50 g je Kilogramm Saatgut, vorzugsweise 0,01 bis 10 g benötigt.

Bei der Behandlung des Bodens sind Wirkstoffkonzentrationen von 0,00001 bis 0,1 Gew.-%, vorzugsweise von 0,0001 bis 0,02 Gew.-% am Wirkungsort erforderlich.

Die zum Schutz technischer Materialien verwendeten Mittel enthalten die Wirkstoffe im allgemeinen in einer Menge von 1 bis 95%, bevorzugt von 10 bis 75 %.

Die Anwendungskonzentrationen der erfindungsgemäßen Wirkstoffe richten sich nach der Art und dem Vorkommen der zu bekämpfenden Mikroorganismen sowie nach der Zusammensetzung des zu schützenden Materials. Die optimale Einsatzmenge kann durch Testreihen ermittelt werden. Im allgemeinen liegen die Anwendungskonzentrationen im Bereich von 0,001 bis 5 Gewichts-%, vorzugsweise von 0,05 bis 1,0 Gewichts-% bezogen auf das zu schützende Material.

Die Wirksamkeit und das Wirkungsspektrum der erfindungsgemäß im Materialschutz zu verwendenden Wirkstoffe bzw. der daraus herstellbaren Mittel, Konzentrate oder ganz allgemein Formulierungen kann erhöht werden, wenn gegebenenfalls weitere antimikrobiell wirksame Verbindungen, Fungizide, Bakterizide, Herbizide, Insektizide oder andere Wirkstoffe zur Vergrößerung des Wirkungsspektrums oder Erzielung besonderer Effekte wie z.B. dem zusätzlichen Schutz vor Insekten zugesetzt werden. Diese Mischungen können ein breiteres Wirkungsspektrum besitzen als die erfindungsgemäßen Verbindungen.

Auch beim Einsatz gegen tierische Schädlinge können die erfindungsgemäßen Stoffe in handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Die Herstellung und die Verwendung der erfindungsgemäßen Stoffe geht aus den folgenden Beispielen hervor.

### Herstellungsbeispiele

### Beispiel 1 (Referenzbeispiel)

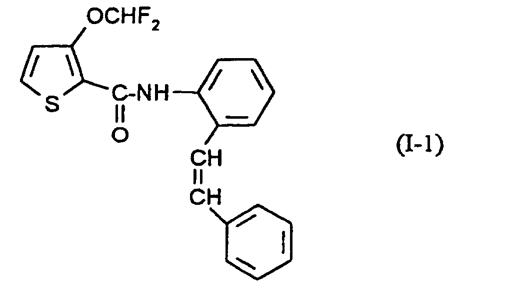

### Verfahren (a):

Eine Lösung von 0,3 g (0,0015 Mol) 2-(2-Phenyl-ethen-1-yl)-anilin in 2 ml Toluol wird bei Raumtemperatur mit einer Lösung von 0,15 g (0,0015 Mol) Triethylamin in 10 ml Toluol versetzt. In dieses Gemisch werden bei Raumtemperatur unter Rühren 0,33 g (0,0015 Mol) 3-Difluormethoxy-thiophen-2-carbonsäurechlorid gegeben. Anschließend erwärmt man auf 50°C und läßt 2 Stunden bei dieser Temperatur nachrühren. Zur Aufarbeitung wird das Reaktionsgemisch dann auf Raumtemperatur abgekühlt und mit Wasser versetzt. Die organische Phase wird abgetrennt, über Natriumsulfat getrocknet und danach bei 60°C unter vermindertem Druck eingeengt. Der verbleibende Rückstand wird mit Diethylether als Laufmittel an Kieselgel chromatographiert. Nach dem Einengen des Eluates erhält man 0,53 g (95 % der Theorie) an 3-Difluormethoxy-thiophen-2-carbonsäure-[2-(2-phenyl-ethen-1-yl)]-anilid in Form einer Festsubstanz vom Schmelzpunkt 80 bis 82°C.

### Herstellung von Ausgangssubstanzen:

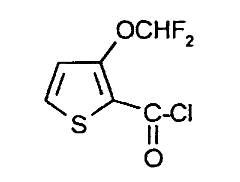

Ein Gemisch aus 4,2 g (0,022 Mol) 3-Difluormethoxy-thiophen-2-carbonsäure in 45 ml Toluol wird bei 80°C unter Rühren mit 3,1 g (0,026 Mol) Thionylchlorid versetzt. Nach beendeter Zugabe wird das Reaktionsgemisch auf 90°C erhitzt und noch 2 Stunden bei dieser Temperatur gerührt. Anschließend wird das Reaktionsgemisch unter vermindertem Druck bei 60°C eingeengt. Man erhält auf diese Weise 4,6 g (98,3 % der Theorie) an 3-Difluormethoxy-thiophen-2-carbonsäurechlorid in Form eines Öles.

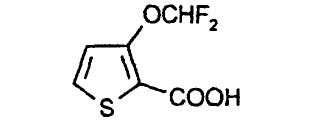

Ein Gemisch aus 2,4 g (0,012 Mol) 3-Difluormethoxy-thiophen-2-carbonsäuremethylester und 5 ml Ethanol wird bei Raumtemperatur mit einer Lösung von 2 g (0,048 Mol) Natriumhydroxid in 10 ml Wasser versetzt und dann 10 Stunden bei Raumtemperatur gerührt. Danach wird das Reaktionsgemisch mit 50 ml Wasser verdünnt und mehrfach mit Methylenchlorid extrahiert. Die wäßrige Phase wird durch Zugabe von verdünnter Salzsäure auf einen pH-Wert von 2 bis 3 gebracht. Das anfallende Festprodukt wird abgesaugt, mit Wasser gewaschen und getrocknet. Man erhält auf diese Weise 2,2 g (90,15 % der Theorie) an 3-Difluormethoxythiophen-2-carbonsäure in Form einer Festsubstanz vom Schmelzpunkt 98 bis 100°C.

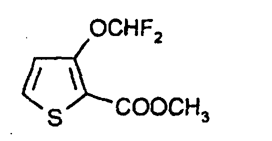

Ein Gemisch aus 10 g (0,063 Mol) 3-Hydroxy-thiophen-2-carbonsäure-methylester und 90 ml Toluol wird bei Raumtemperatur mit einer Lösung von 5,1 g (0,127 Mol) Natriumhydroxid in 8 ml Wasser versetzt. Man erwärmt das Reaktionsgemisch unter Rühren auf 90°C, gibt 1,1 g Tetrabutyl-phosphoniumbromid hinzu und leitet innerhalb von 30 Minuten 16,4 g (0,189 Mol) Chlordifluormethan ein. Danach wird eine Stunde bei 90°C nachgerührt. Anschließend wird das Reaktionsgemisch auf Raumtemperatur abgekühlt und mit Wasser versetzt. Die organische Phase wird abgetrennt, über Natriumsulfat getrocknet und dann bei 50°C unter vermindertem Druck eingeengt. Der verbleibende Rückstand wird mit Cyclohexan : Essigsäureethylester = 3:1 als Laufmittel an Kieselgel chromatographiert. Nach dem Einengen des Eluates erhält man 4,1 g (31,3 % der Theorie) an 3-Difluormethoxy-thiophen-2-carbonsäuremethylester in Form eines Öles.
¹H-NMR-Spektrum (CDCl₃/TMS): δ = 3,833 (s, 3H); 7,463/7,481 (d, 1H) ppm

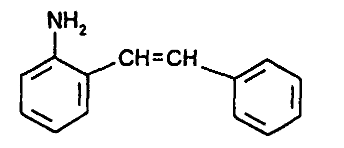

Ein Gemisch aus 12,9 g (0,057 Mol) 1-Phenyl-2-(2-nitro-phehyl)-ethen und 210 ml 17 %iger wäßriger Salzsäure wird bei Raumtemperatur mit 38,7 g Zinnpulver versetzt und langsam auf Rückflußtemperatur erhitzt. Man kocht 2 Stunden unter Rückfluß, kühlt dann auf Raumtemperatur ab und extrahiert mehrfach mit Diethylether. Die organische Phase wird unter vermindertem Druck eingeengt. Der verbleibende Rückstand wird mit Wasser versetzt, und das entstehende Gemisch wird durch Zugabe von verdünnter wäßriger Natronlauge neutral gestellt und dann mehrfach mit Methylenchlorid extrahiert. Die vereinigten organischen Phasen werden über Natriumsulfat getrocknet und anschließend unter vermindertem Druck eingeengt. Der verbleibende Rückstand wird mit Cyclohexan : Essigsäureethylester = 3:1 an Kieselgel chromatographiert. Nach dem Einengen des Eluates erhält man 8,5 g (76,4 % der Theorie) an 2-(2-Phenylethen-1-yl)-anilin in Form einer Festsubstanz vom Schmelzpunkt 80°C.

### Beispiel 2

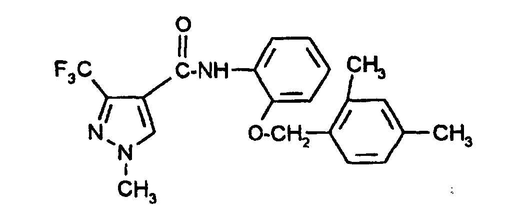

### Verfahren (b):

Ein Gemisch aus 1,0 g (0,0035 Mol) 1-Methyl-3-trifluormethyl-pyrazol-4-carbonsäure-(2-hydroxymethyl)-anilid, 0,53 g (0,0039 Mol) Kaliumcarbonat und 10 ml Acetonitril wird bei Raumtemperatur unter Rühren mit 0,57 g (0,0037 Mol) 2,4-Dimethyl-benzylchlorid versetzt. Nach der Zugabe erwärmt man auf 60°C und rührt 2 Stunden bei dieser Temperatur. Anschließend wird das Reaktionsgemisch auf Raumtemperatur abgekühlt und mit Wasser versetzt. Der anfallende Niederschlag wird abgesaugt, mit Wasser gewaschen und bei 50°C unter vermindertem Druck über Phosphorpentoxid getrocknet. Man erhält auf diese Weise 1,2 g (85 % der Theorie) an 1-Methyl-3-trifluormethyl-pyrazol-4-carbonsäure-[2-(2,4-dimethylbenzyloxy)]-anilid als Festsubstanz vom Schmelzpunkt 123 bis 125°C.
¹H-NMR-Spektrum (CDCl₃/TMS): δ = 3,945 (s, 3H); 5,082 (s, 2H) ppm

### Herstellung der Ausgangssubstanz:

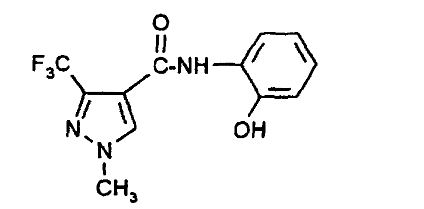

Ein Gemisch aus 2,4 g (0,022 Mol) 2-Amino-phenol und 80 ml Toluol wird bei 100°C unter Rühren mit einer Lösung von 5,0 g (0,024 Mol) 1-Methyl-3-trifluormethyl-pyrazol-4-carbonsäurechlorid in 25 ml Toluol versetzt. Das Reaktionsgemisch wird weitere 4 Stunden bei 100°C gerührt, dann auf Raumtemperatur abgekühlt und mit Wasser versetzt. Der anfallende Niederschlag wird abgesaugt und bei 50°C unter vermindertem Druck getrocknet. Man erhält auf diese Weise 5,4 g (86,1 % der Theorie) an 1-Methyl-3-trifluormethyl-pyrazol-4-carbonsäure-(2-hydroxymethyl)-anilid in Form einer Festsubstanz vom Schmelzpunkt 191°C.
¹H-NMR-Spektrum (d6-DMSO/TMS): δ = 3,972 (s, 3H)

### Beispiel 3

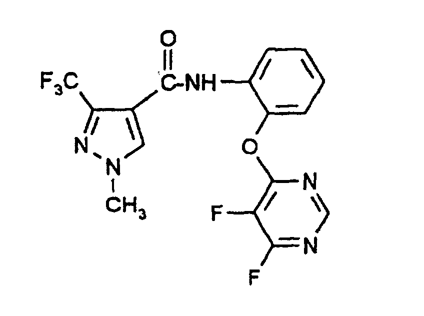

### Verfahren (b):

Eine Lösung von 3 g (10,5 mmol) 1-Methyl-3-trifluormethyl-pyrazol-4-carbonsäure-(2-hydroxymethyl)-anilid in 15 ml Dimethylformamid wird bei 15°C unter Rühren langsam mit 0,347 mg Natriumhydrid (80 %ig in Paraffin) versetzt. Anschließend wird diese Mischung bei 0°C unter Rühren in eine Lösung von 1,41 g (10,5 mmol) 4,5,6-Trifluorpyrimidin in 15 ml Dimethylformamid eingetropft. Nach beendeter Zugabe wird das Reaktionsgemisch noch 1 Stunde bei 0°C nachgerührt und dann durch Abziehen des Lösungsmittels unter vermindertem Druck eingeengt. Der verbleibende Rückstand wird mit Wasser versetzt und 2 Minuten intensiv gerührt. Das anfallende Festprodukt wird abgesaugt, getrocknet und aus Toluol umkristallisiert. Man erhält auf diese Weise 3,43 g (81 % der Theorie) an 1-Methyl-3-trifluormethyl-pyrazol-4-carbonsäure-[2-(4,5-difluorpyrimidyl-6-oxy)]-anilid in Form einer Festsubstanz vom Schmelzpunkt 197°C.

Nach den zuvor angegebenen Methoden werden auch die in der folgenden Tabelle 1 aufgeführten Carbanilide der Formel 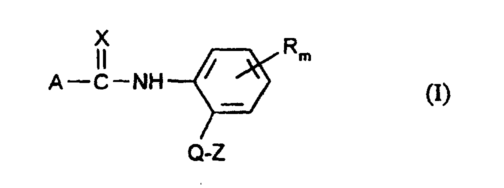 erhalten.

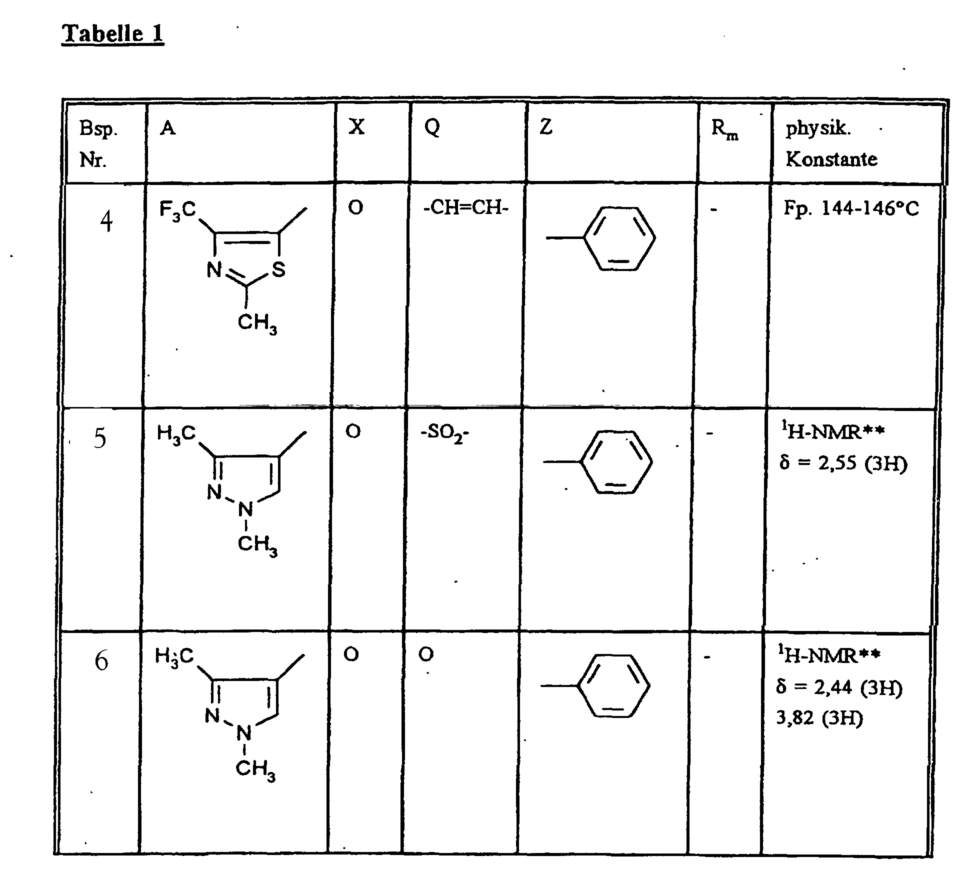

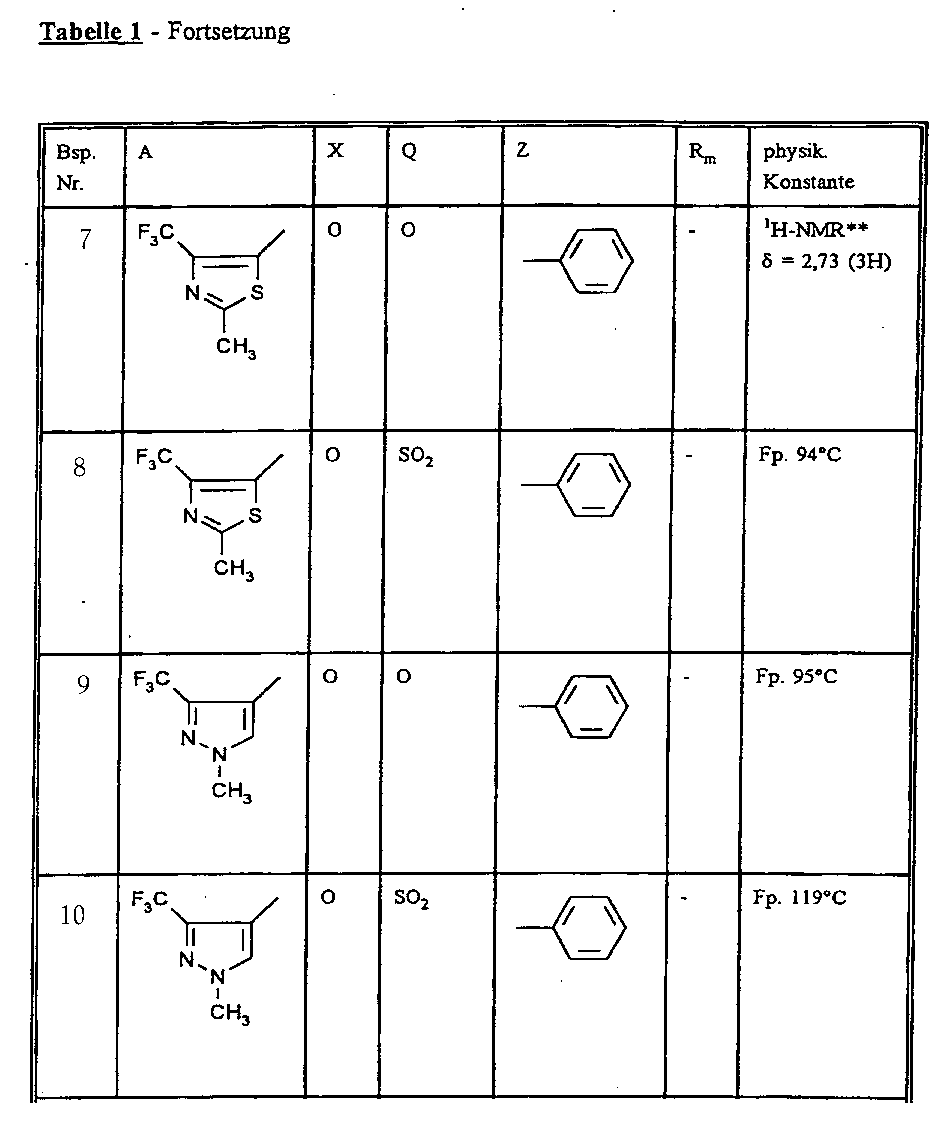

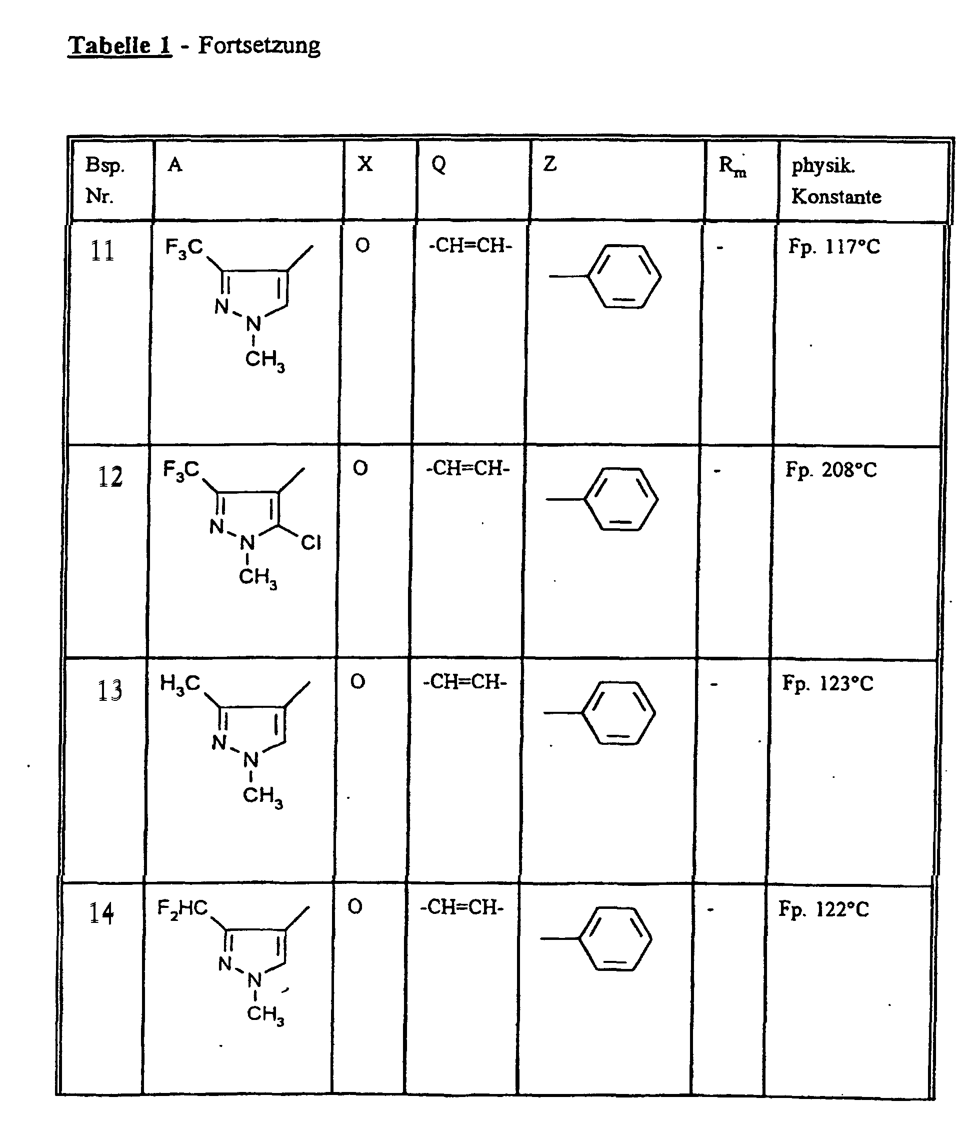

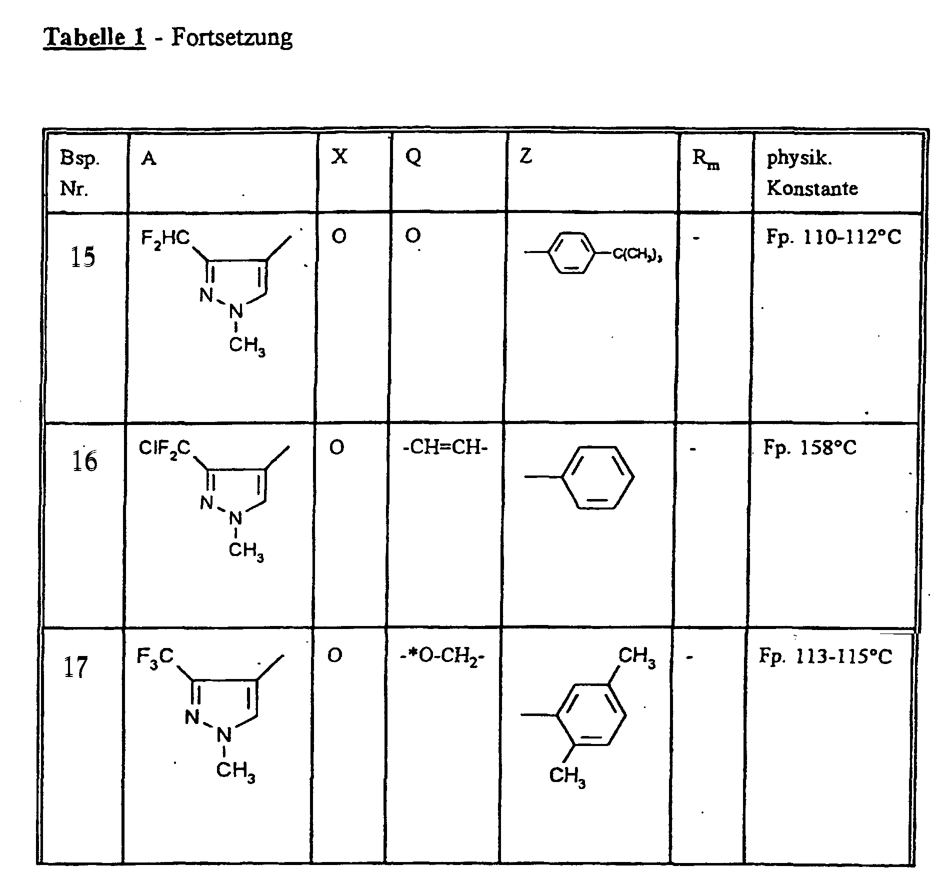

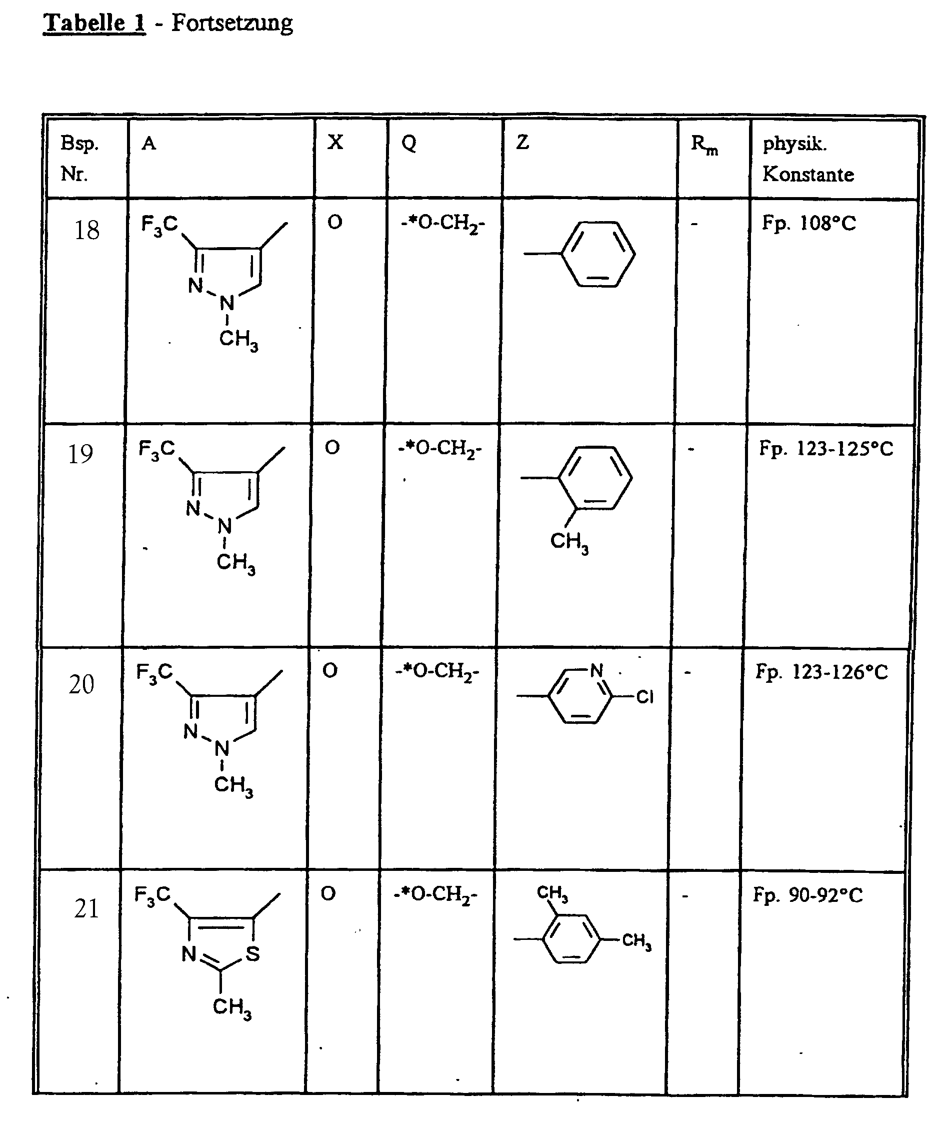

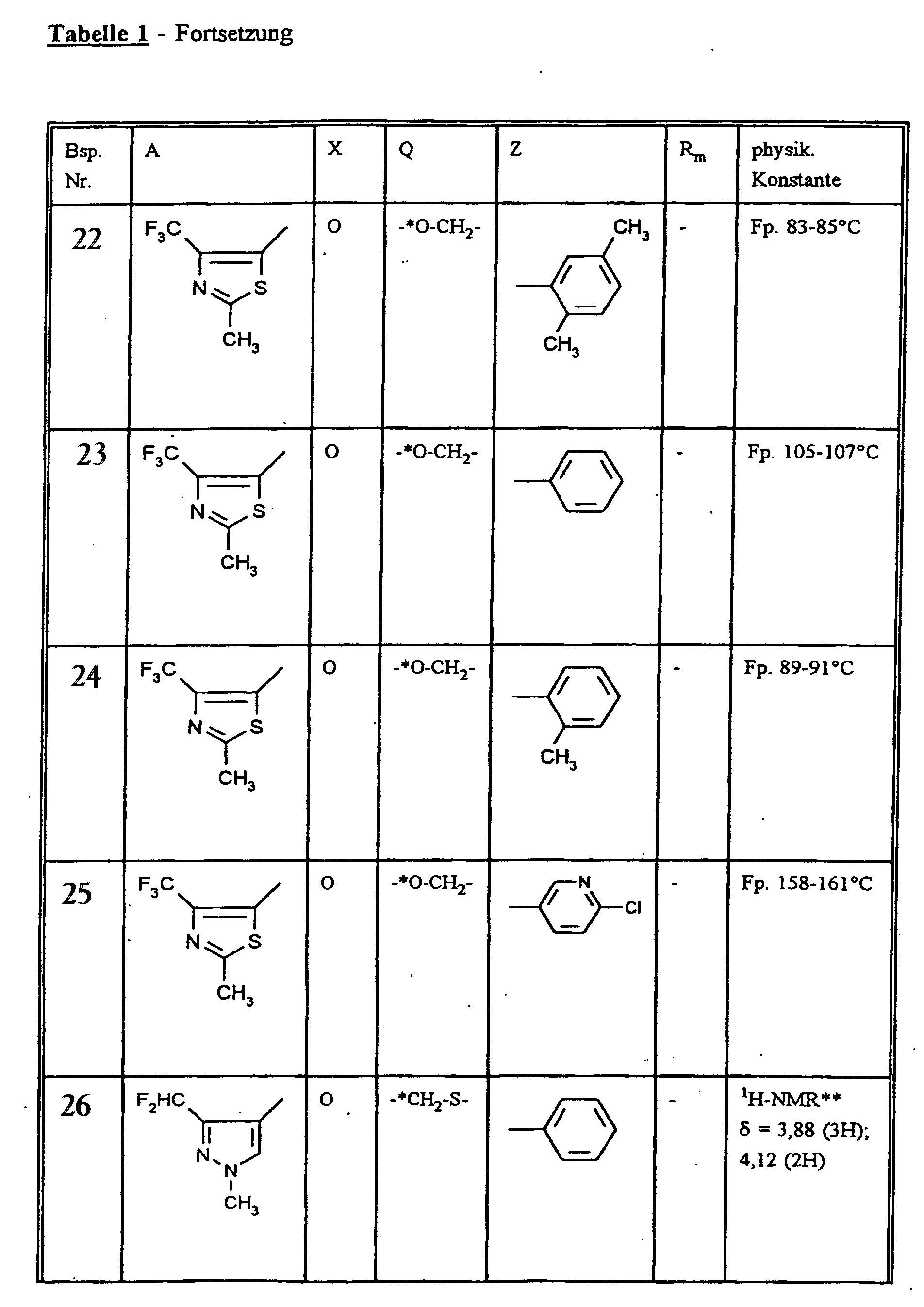

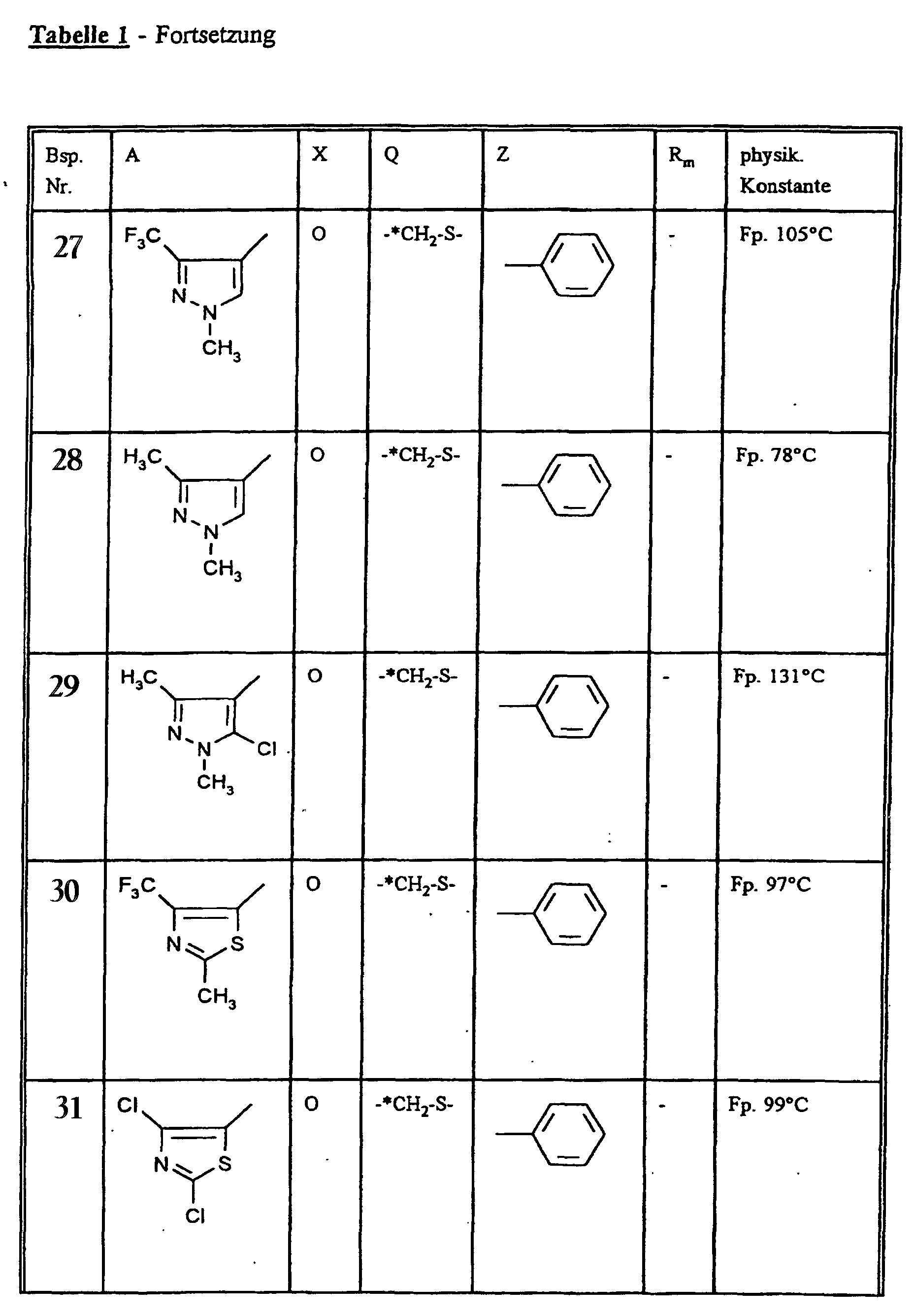

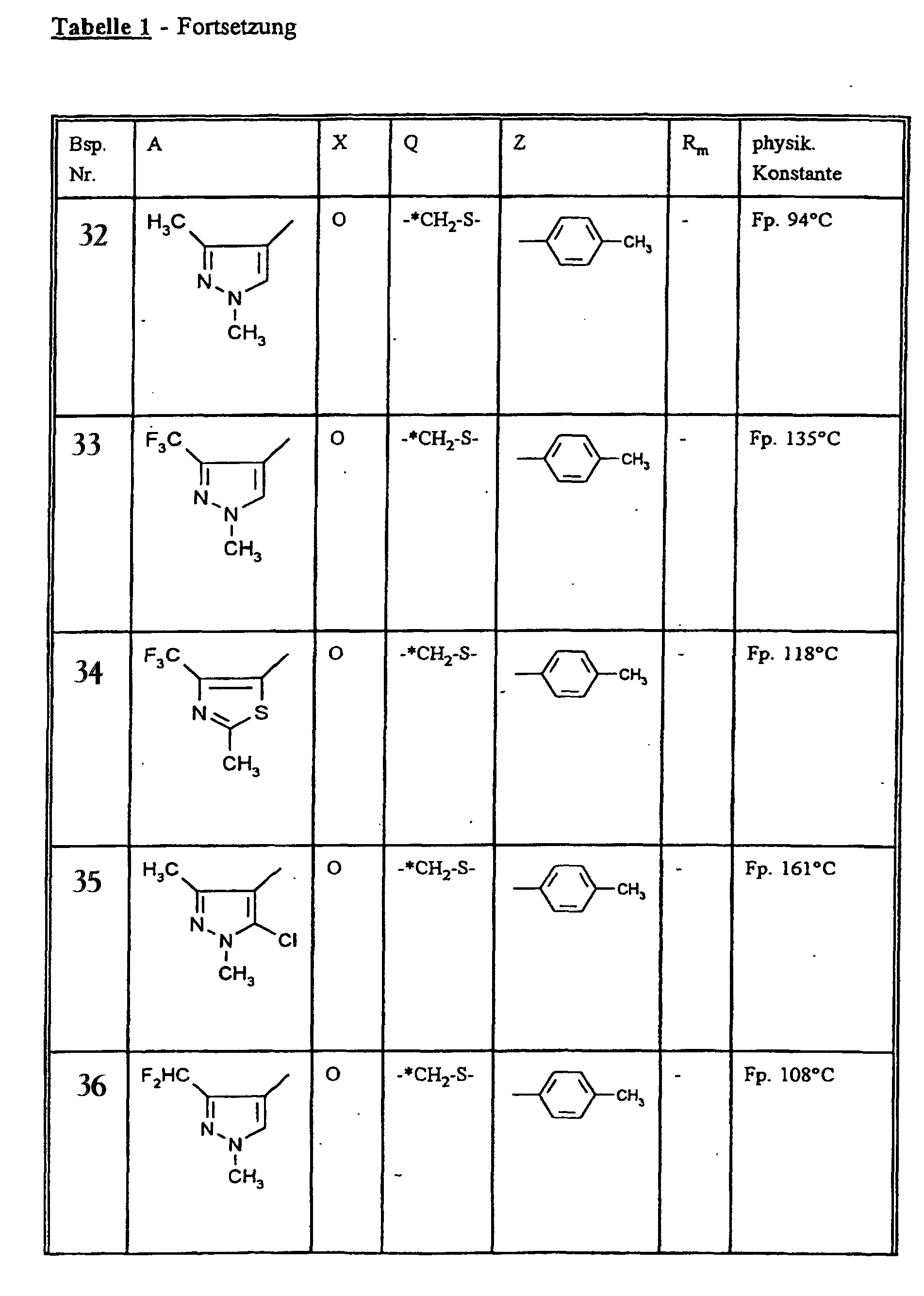

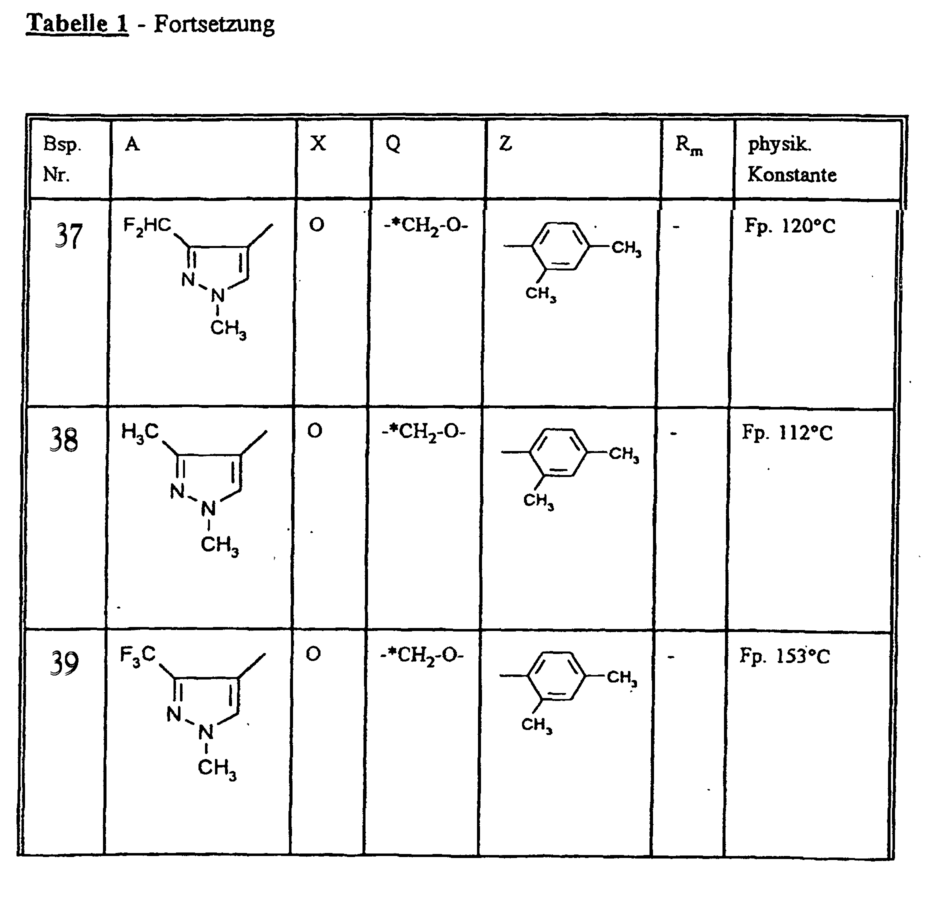

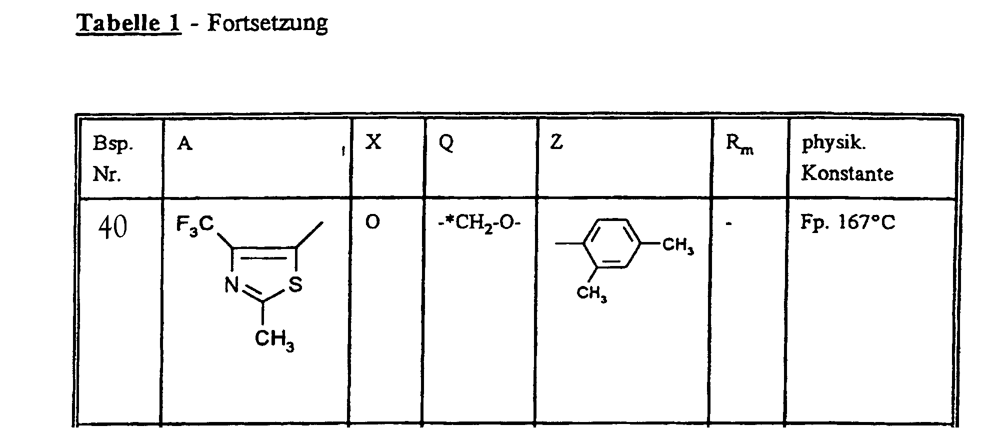

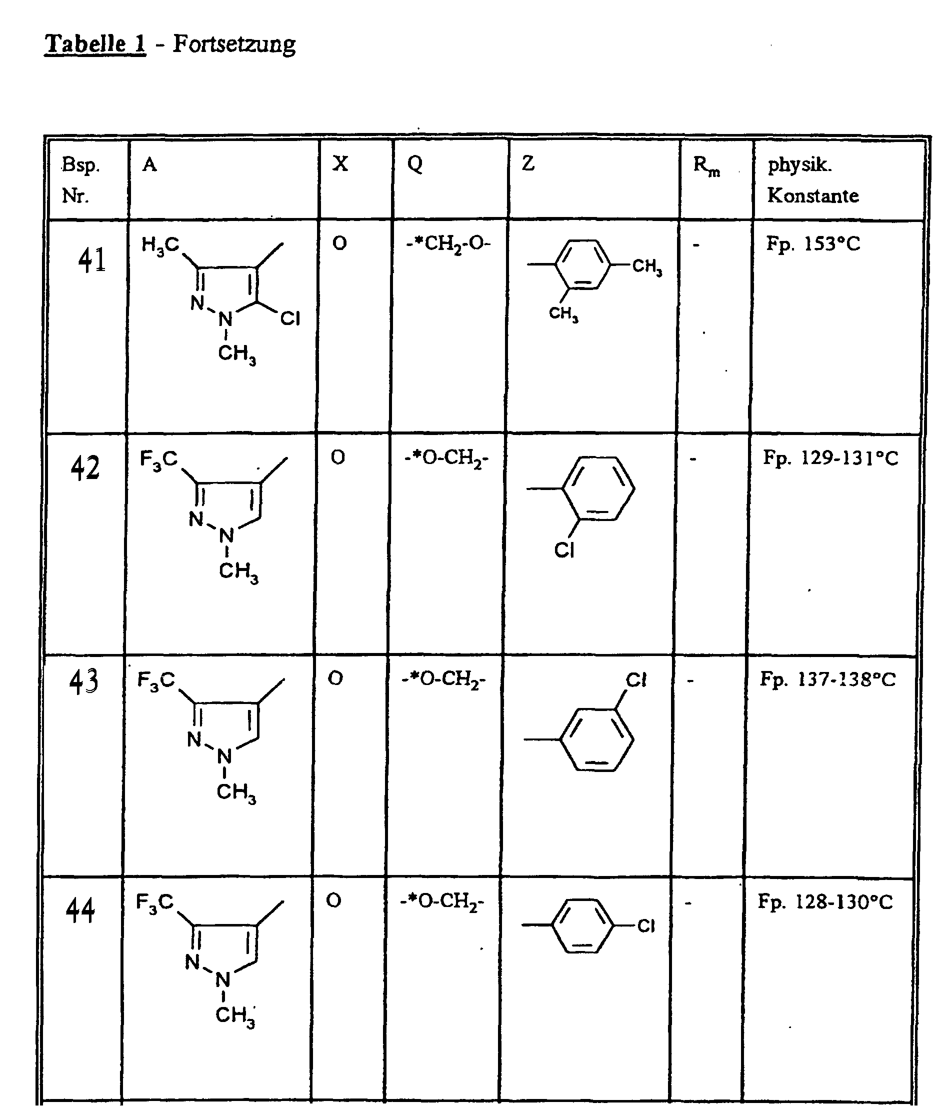

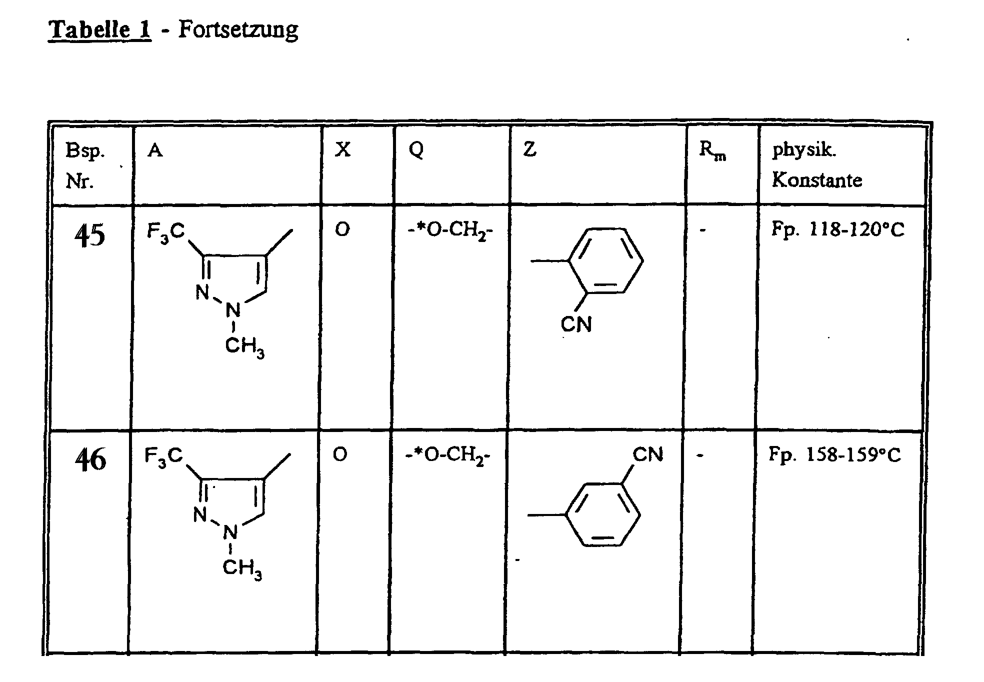

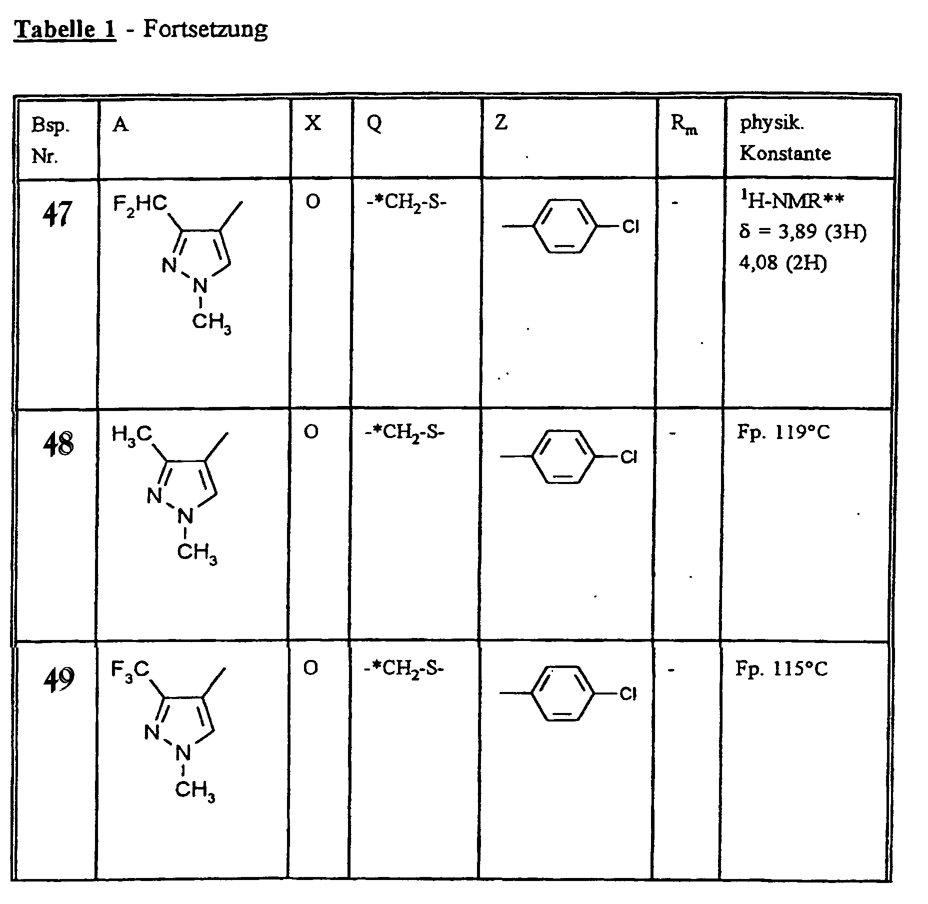

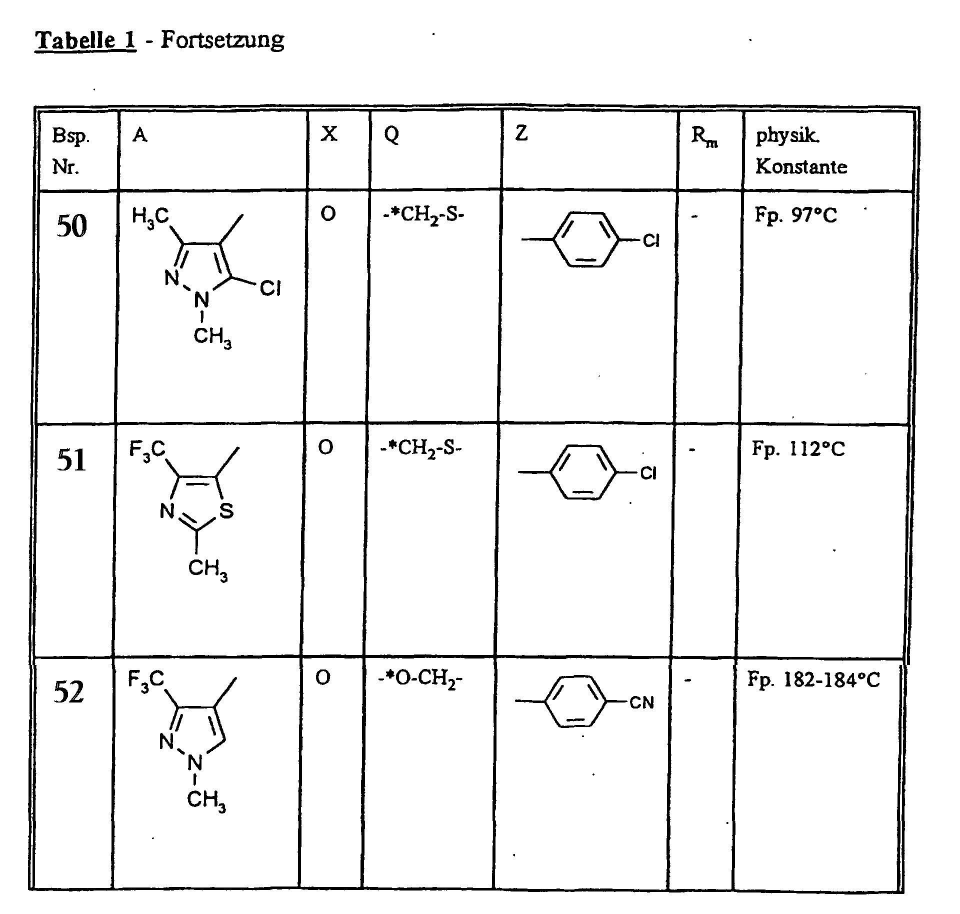

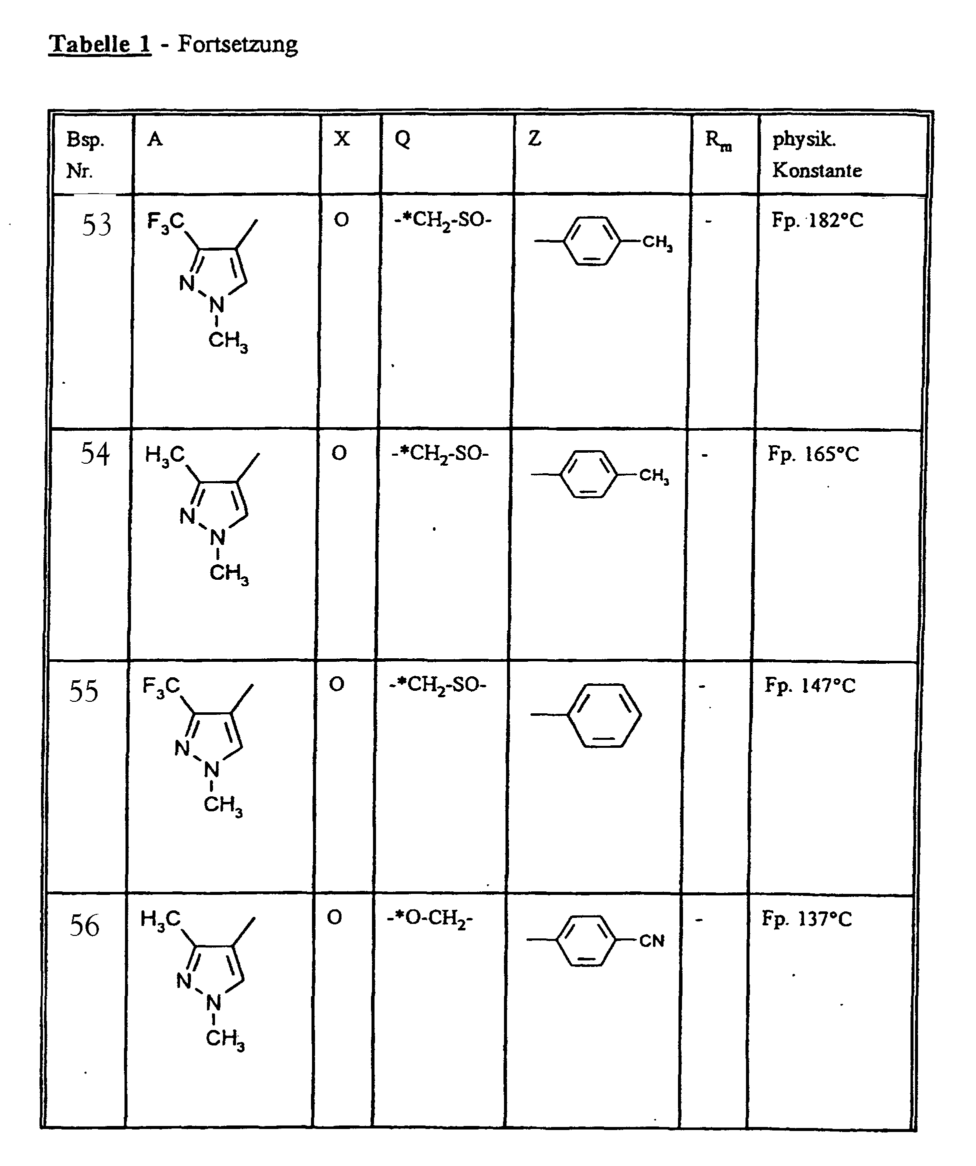

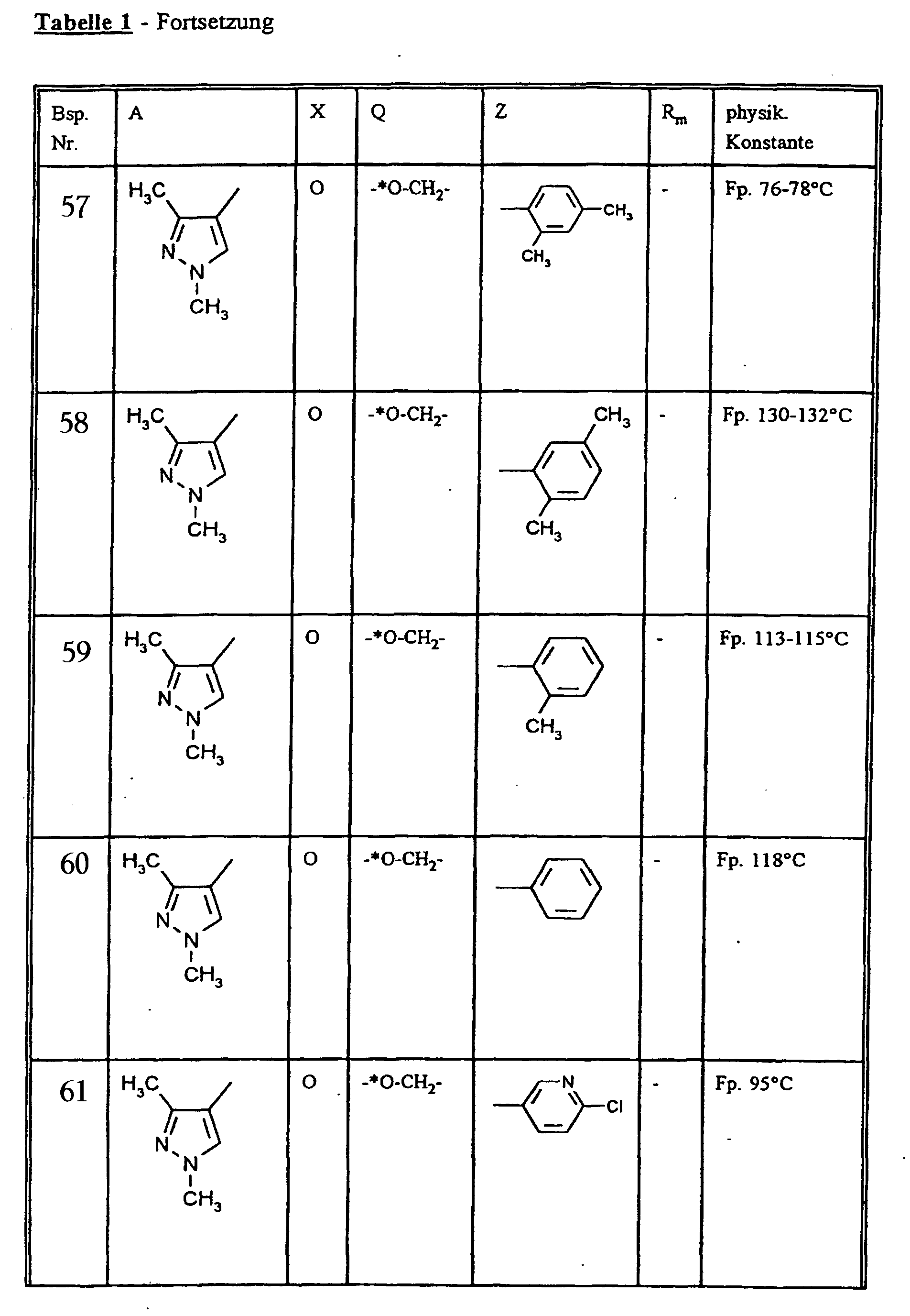

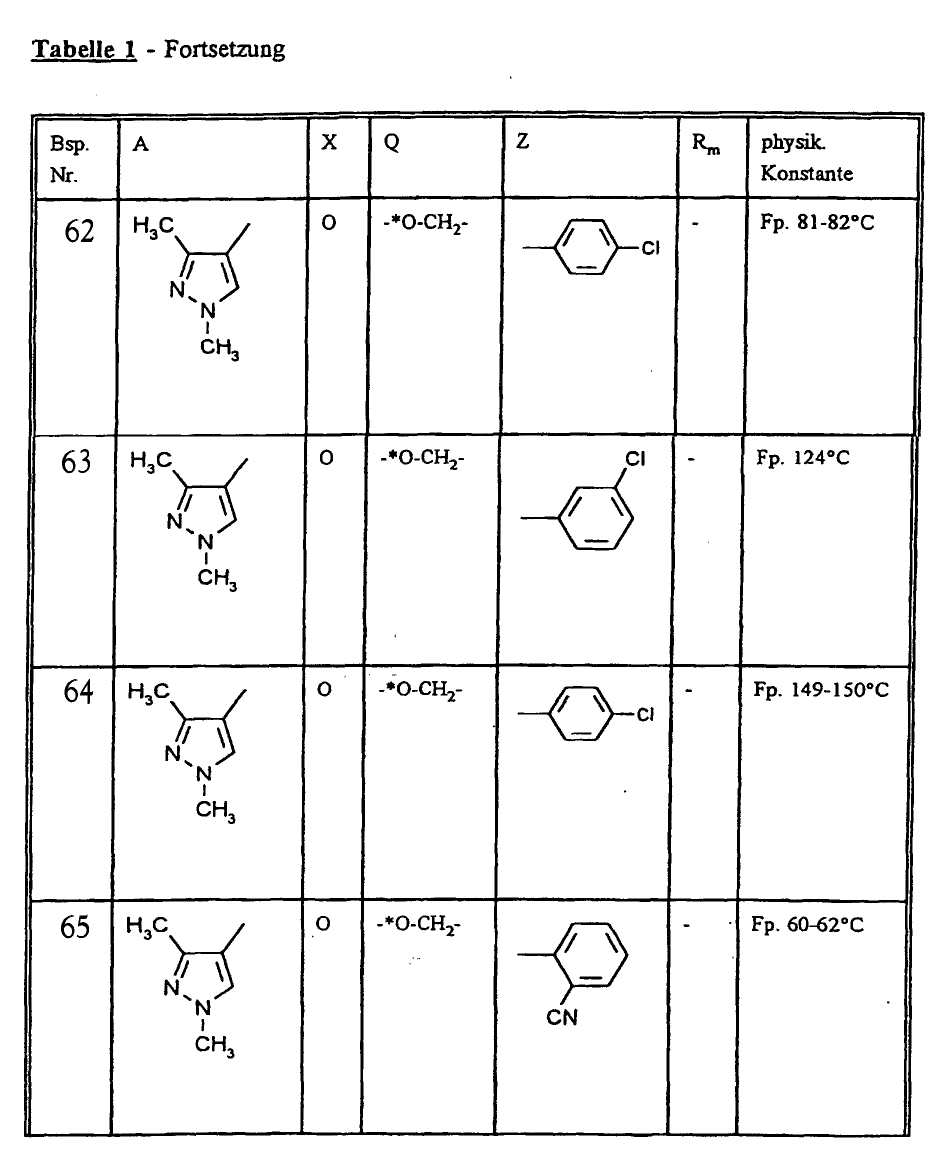

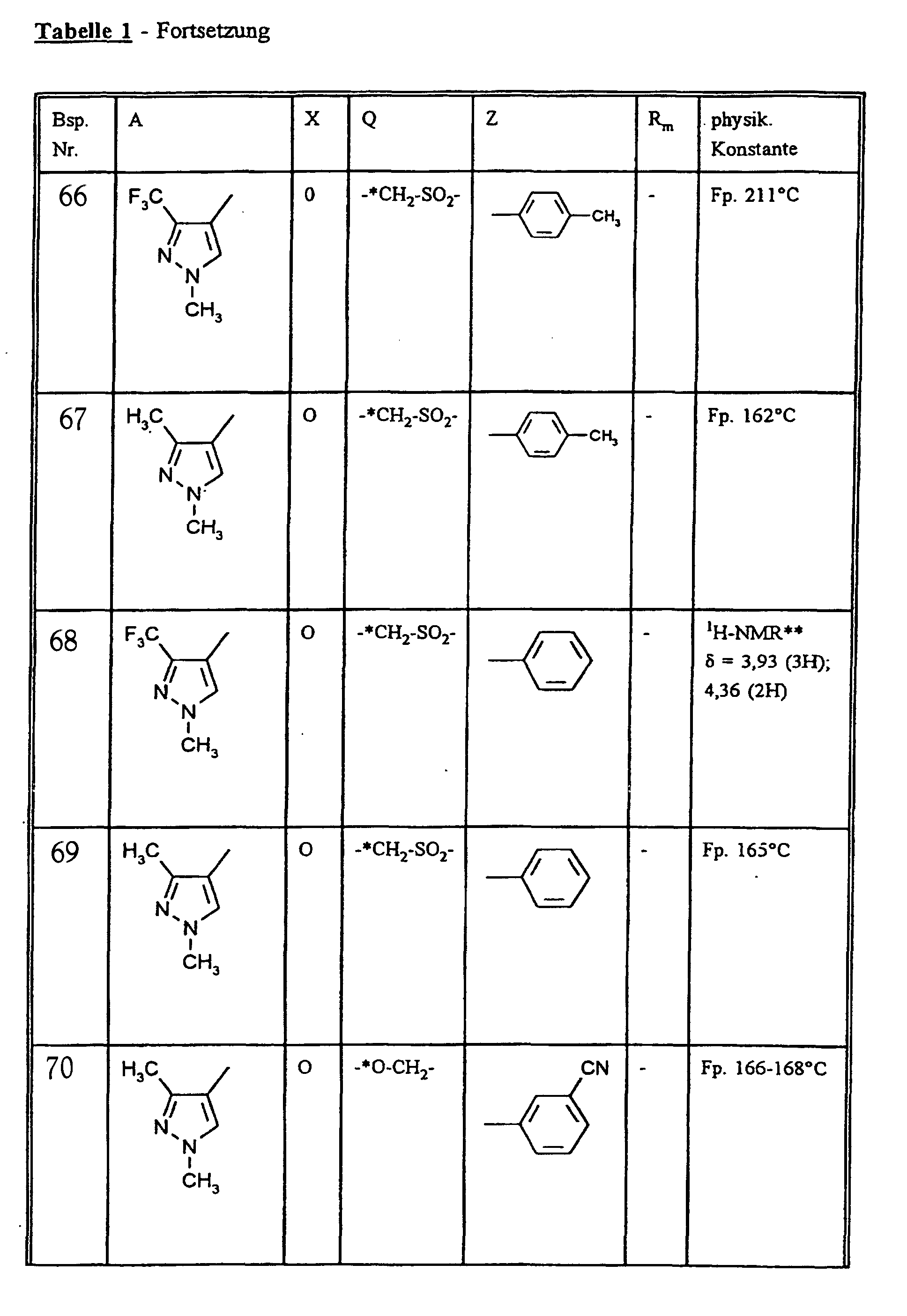

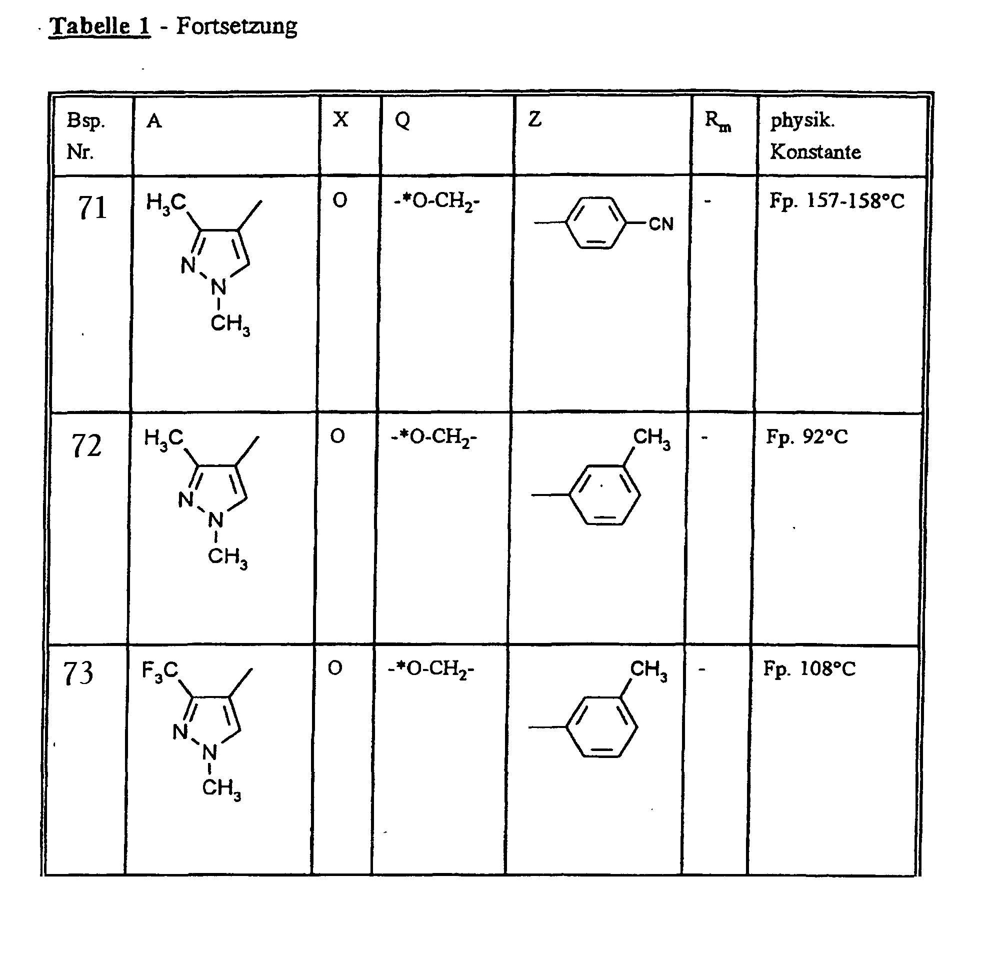

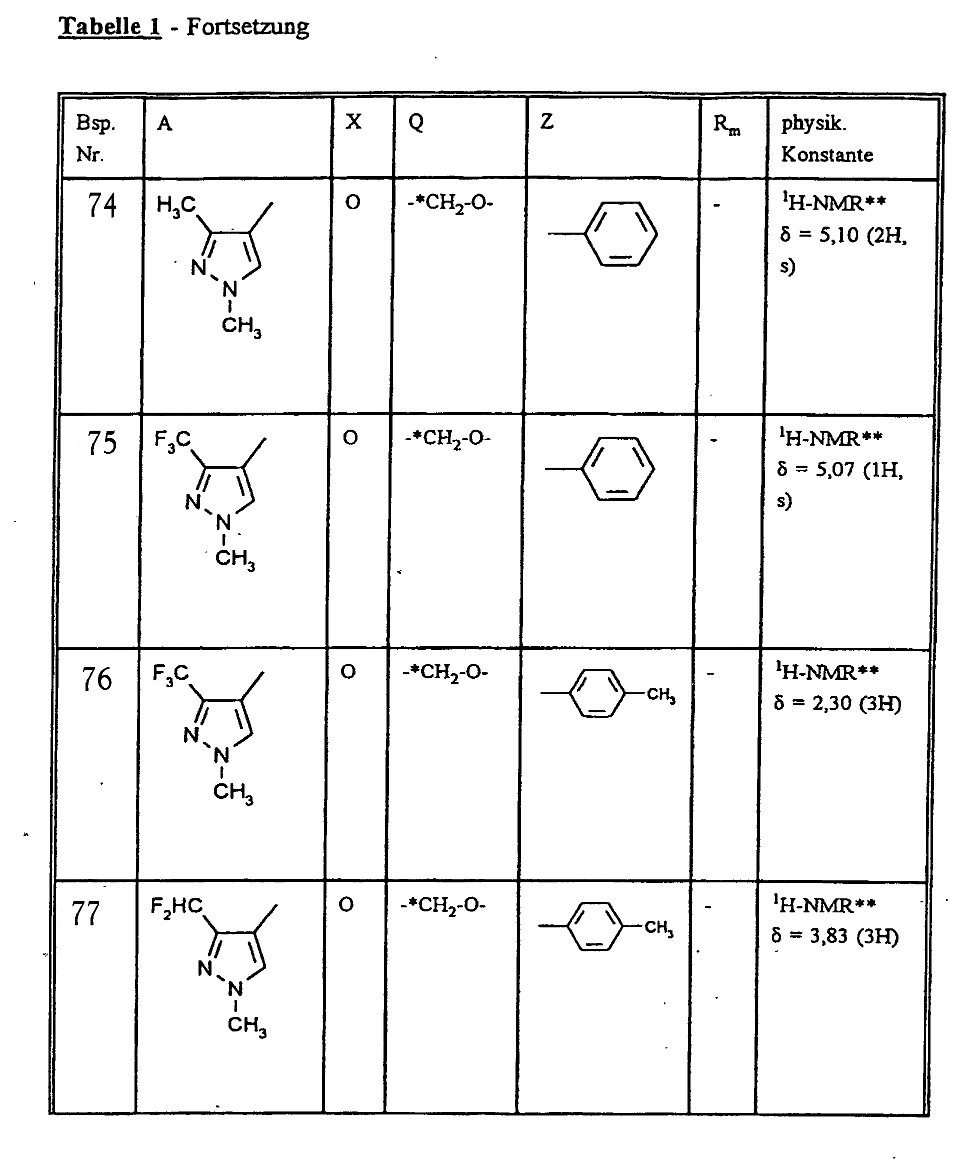

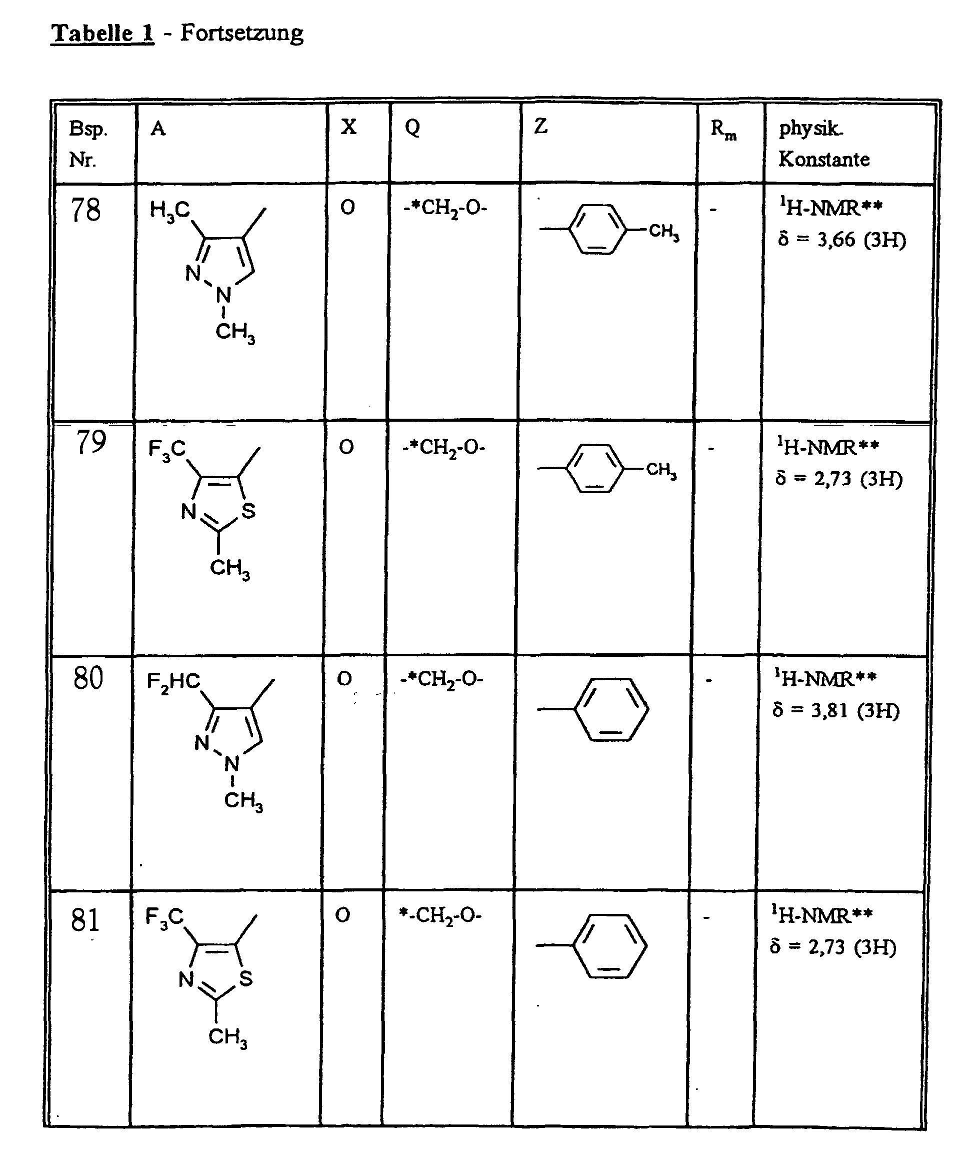

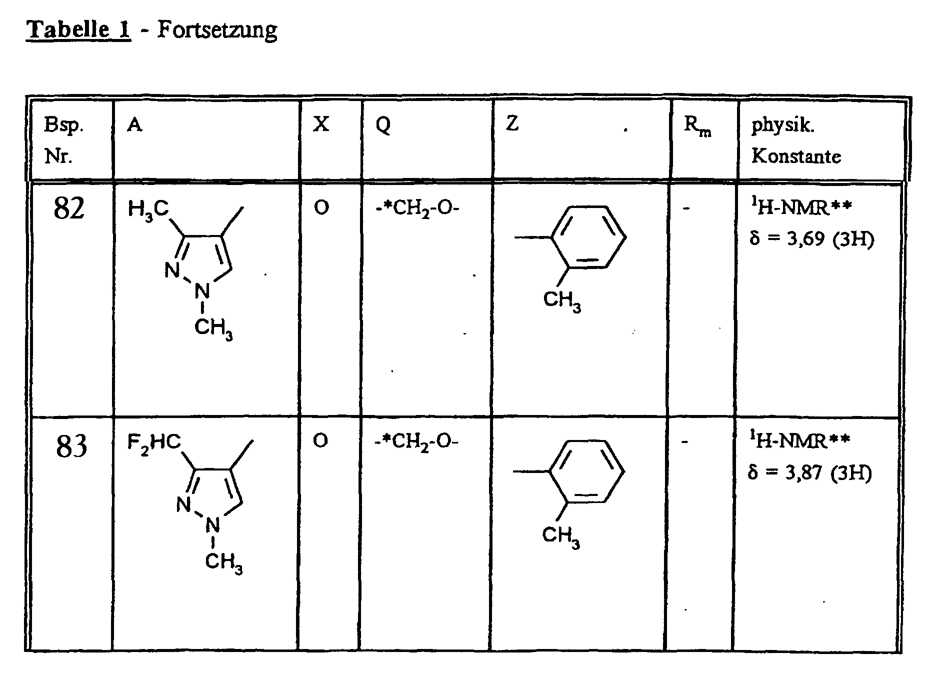

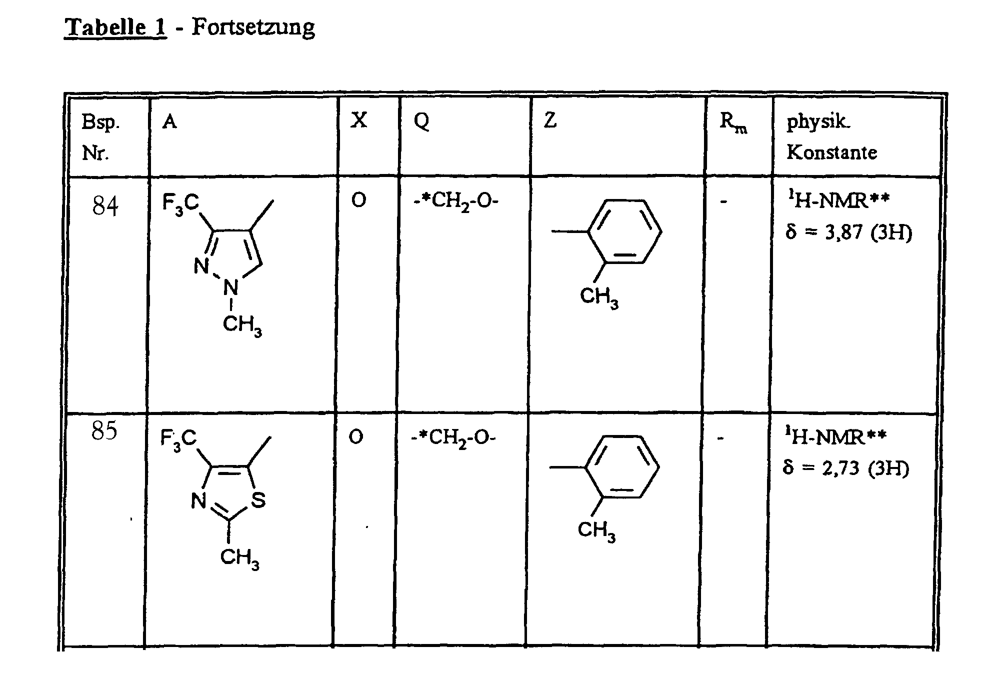

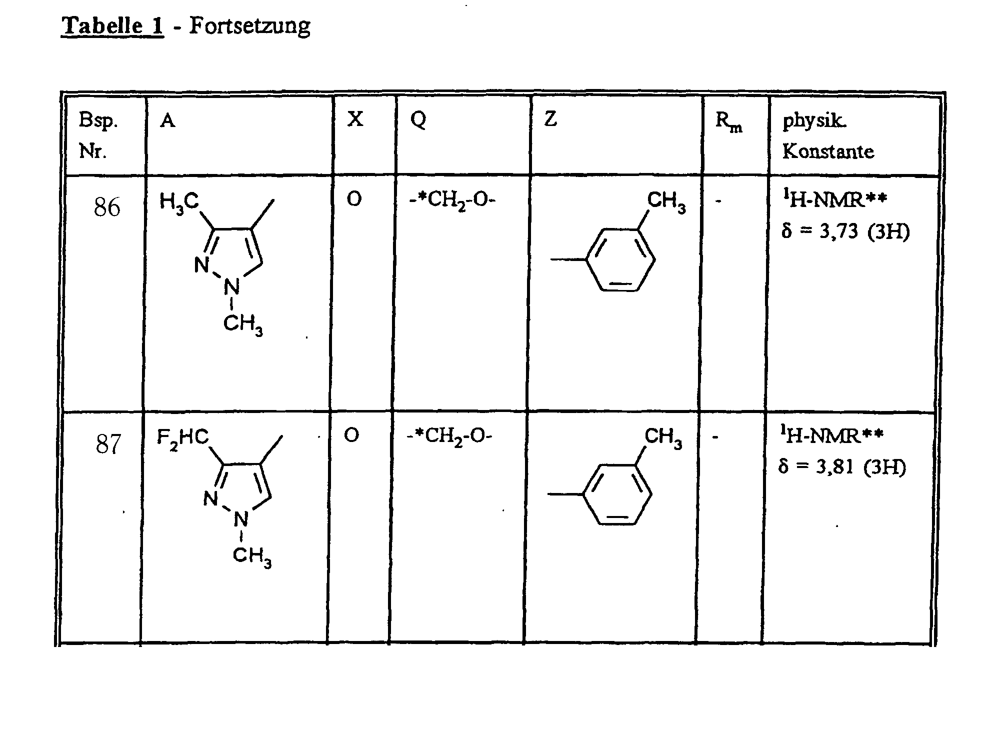

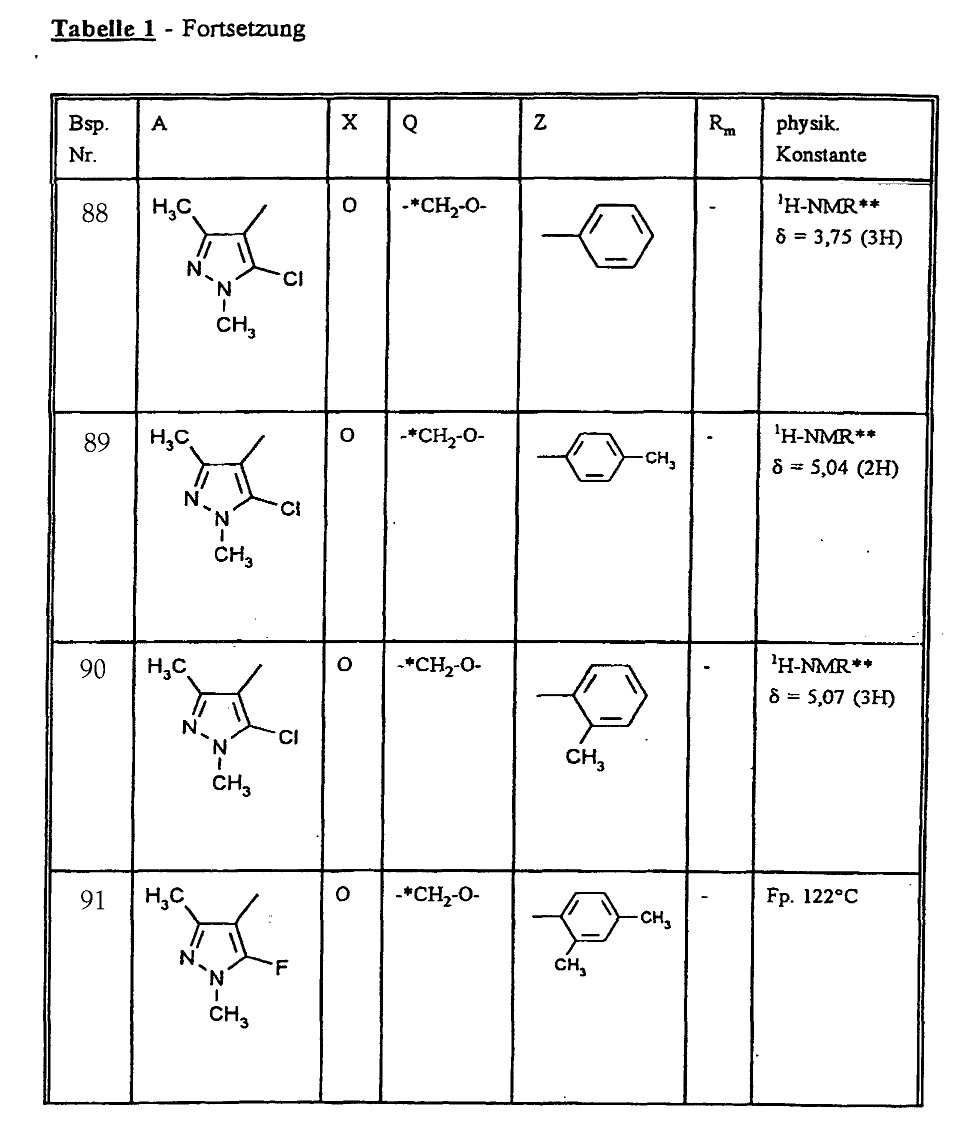

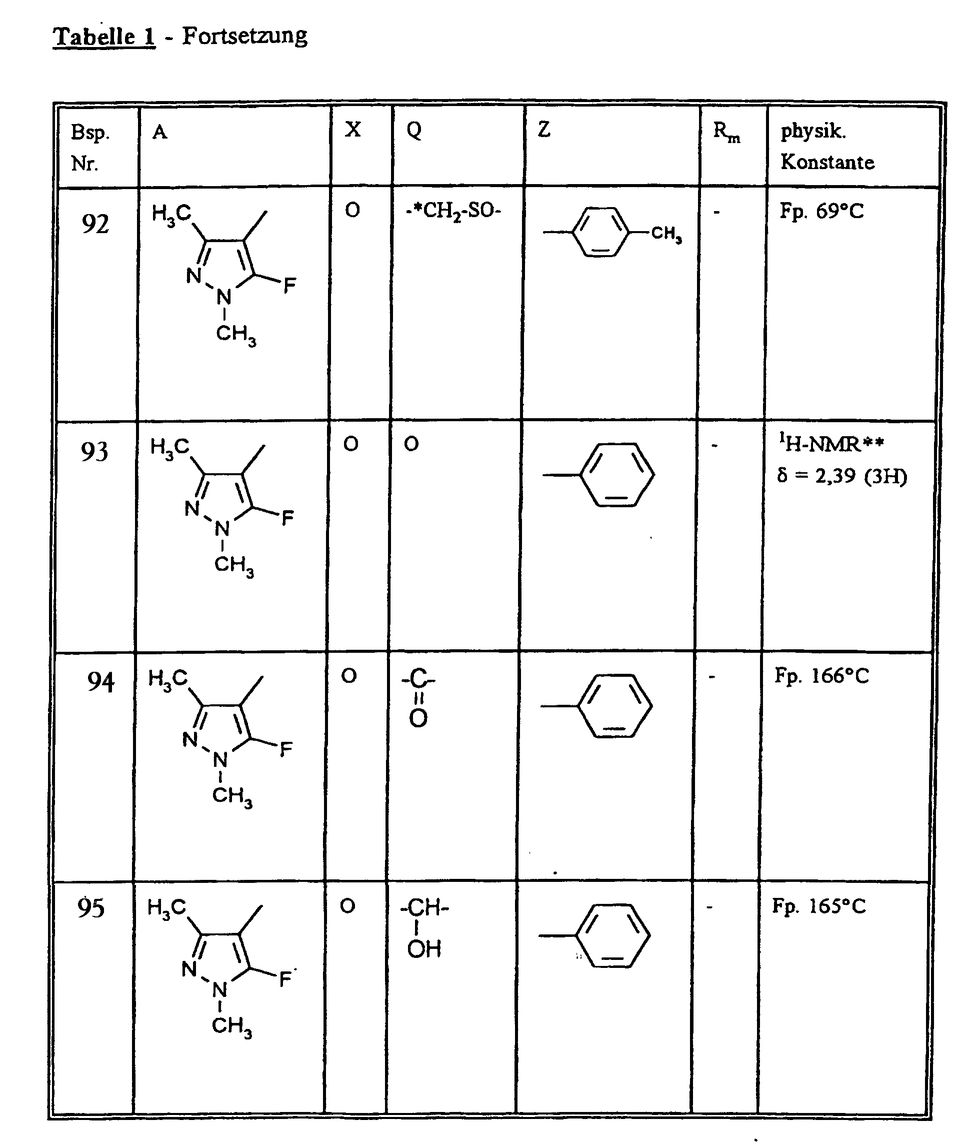

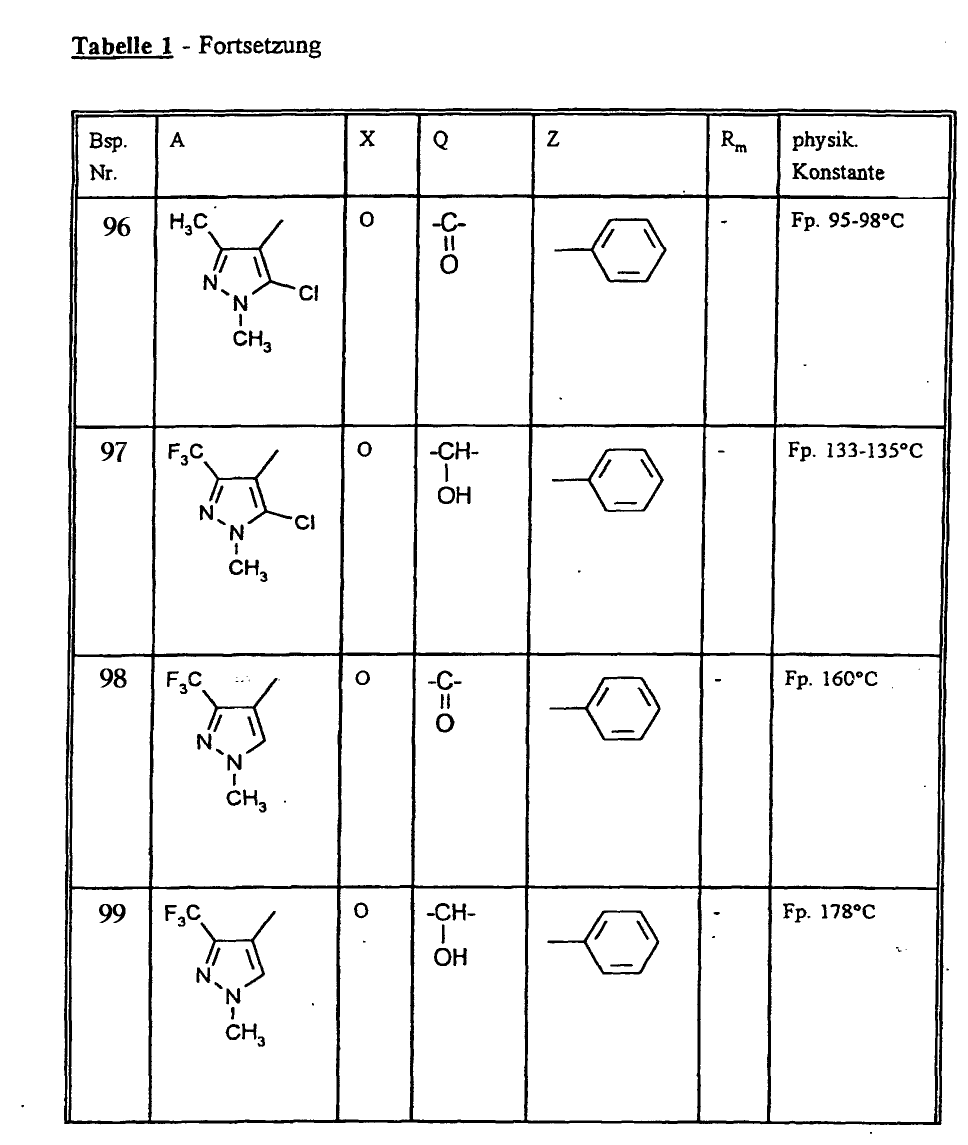

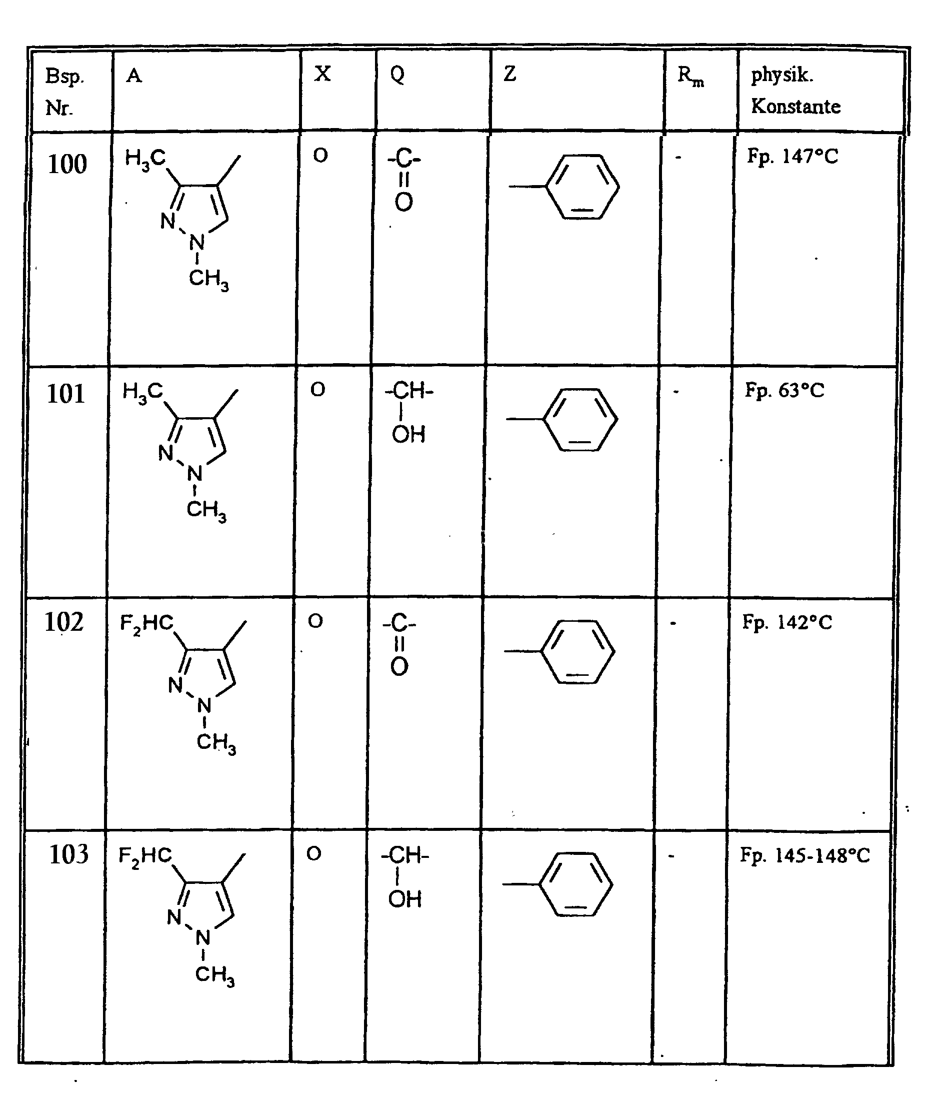

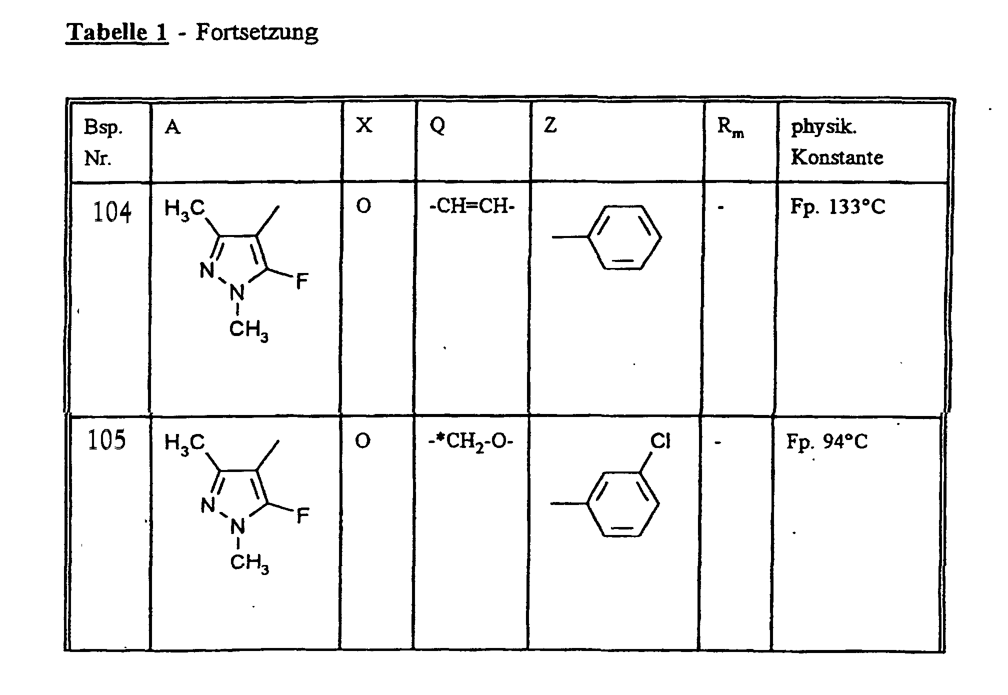

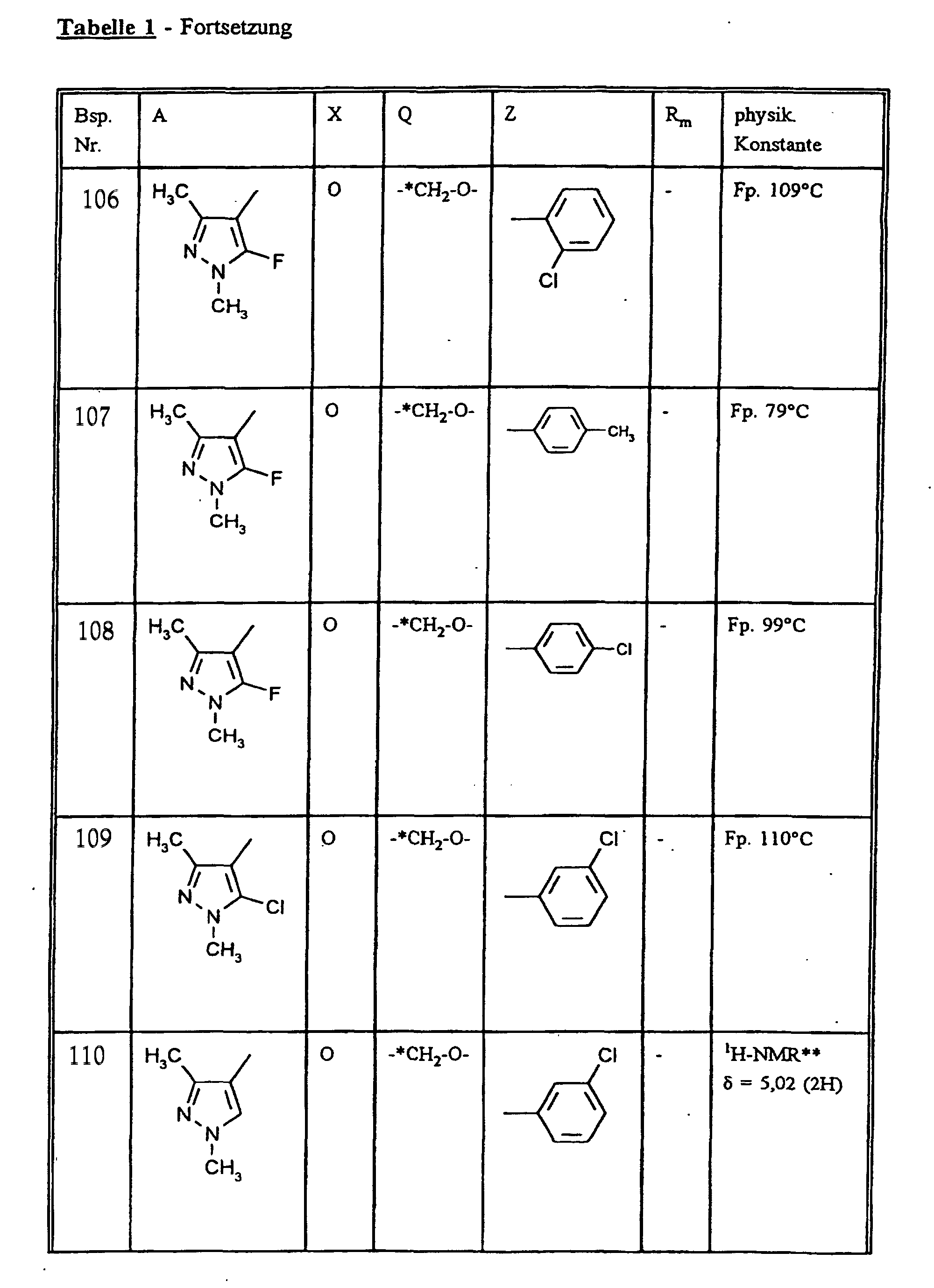

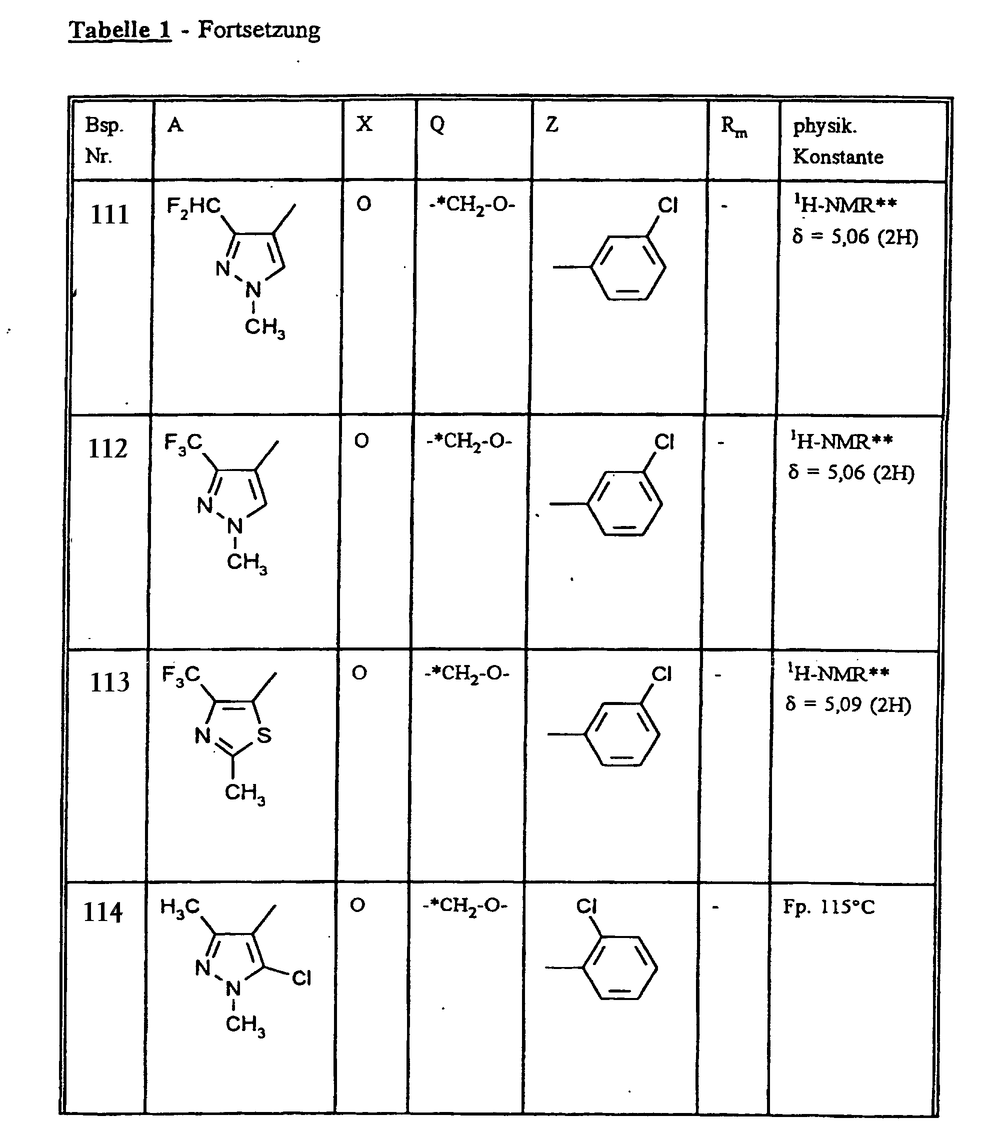

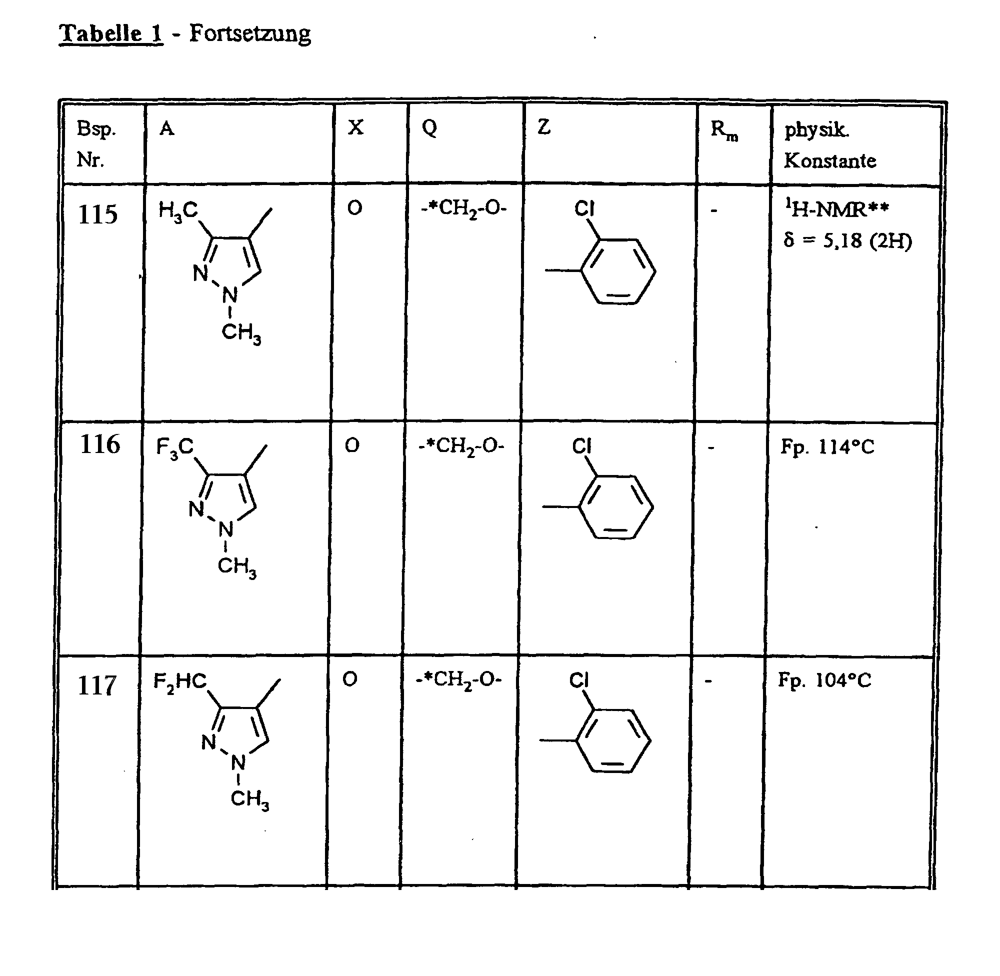

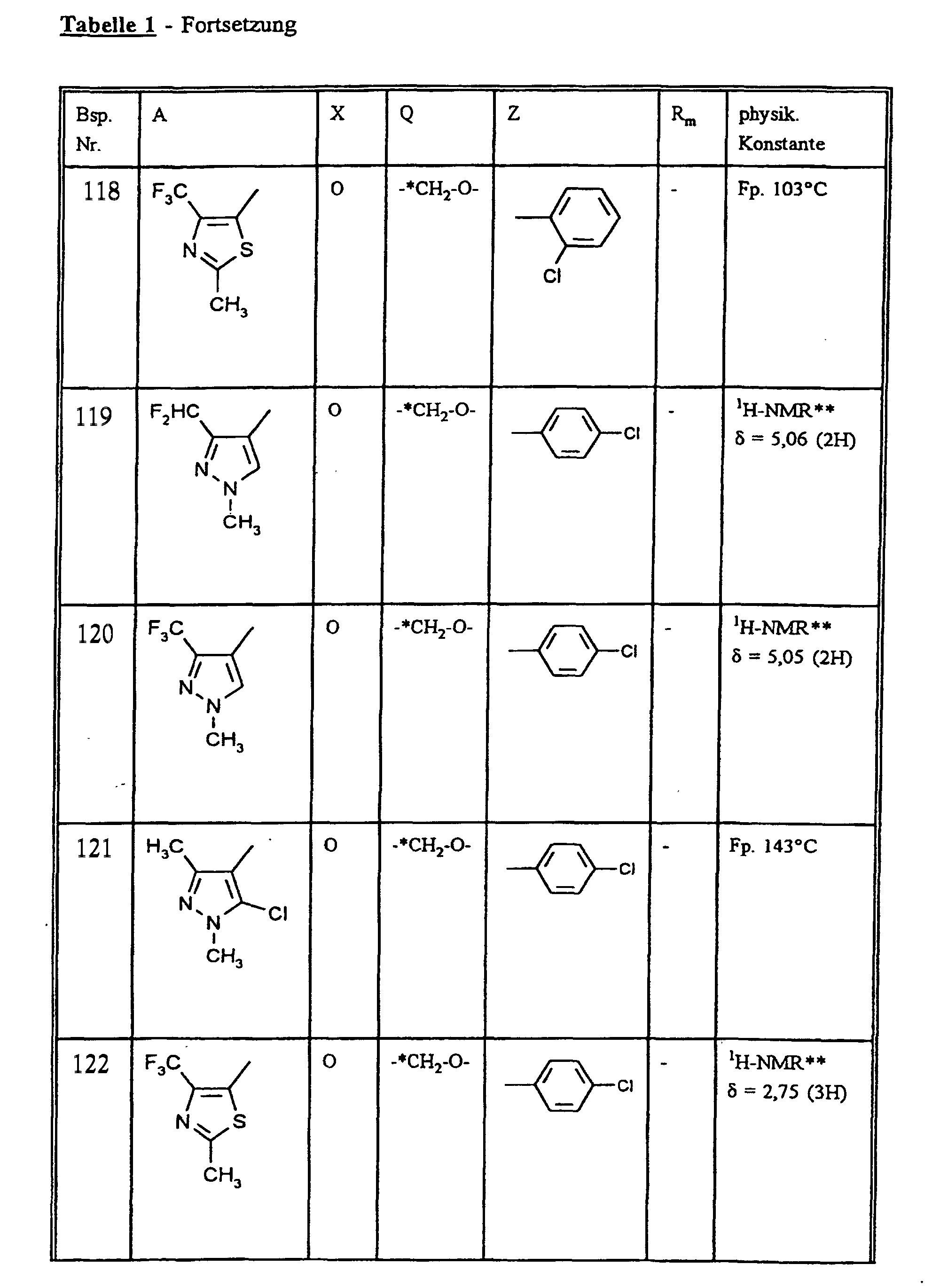

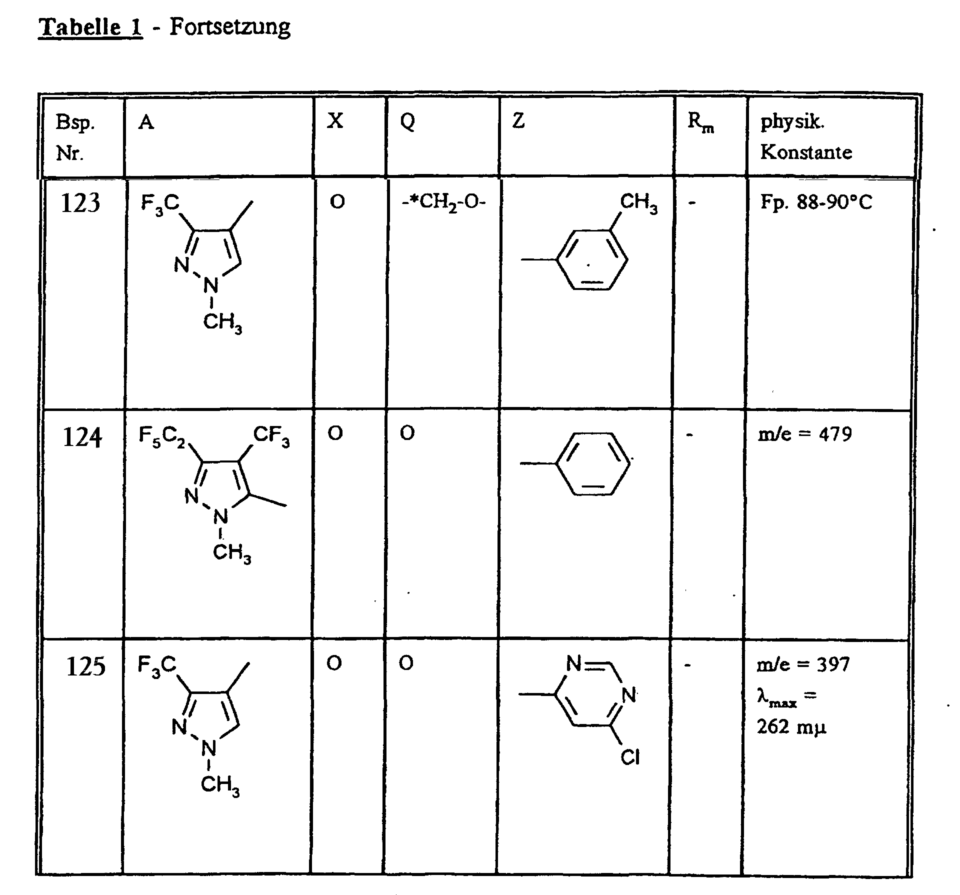

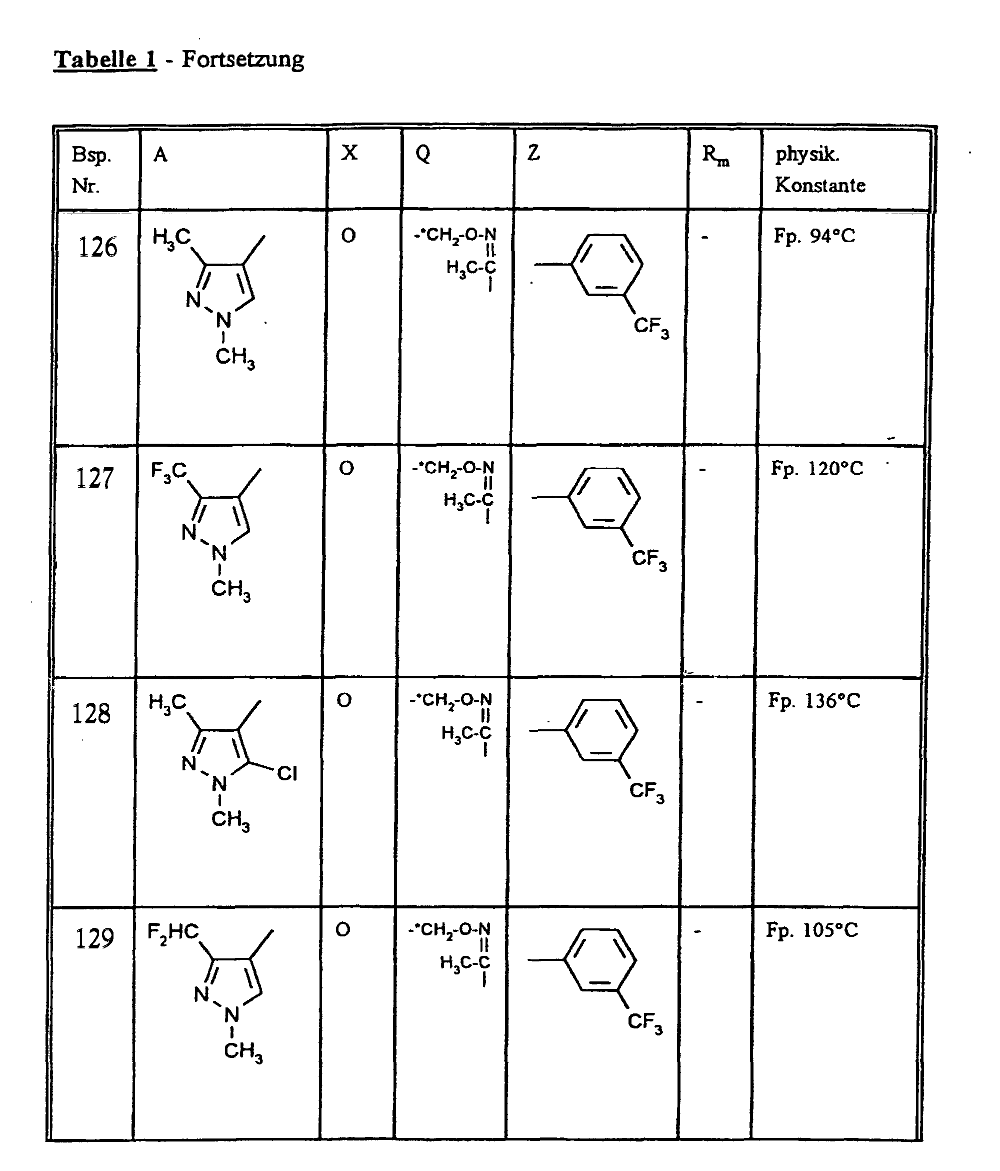

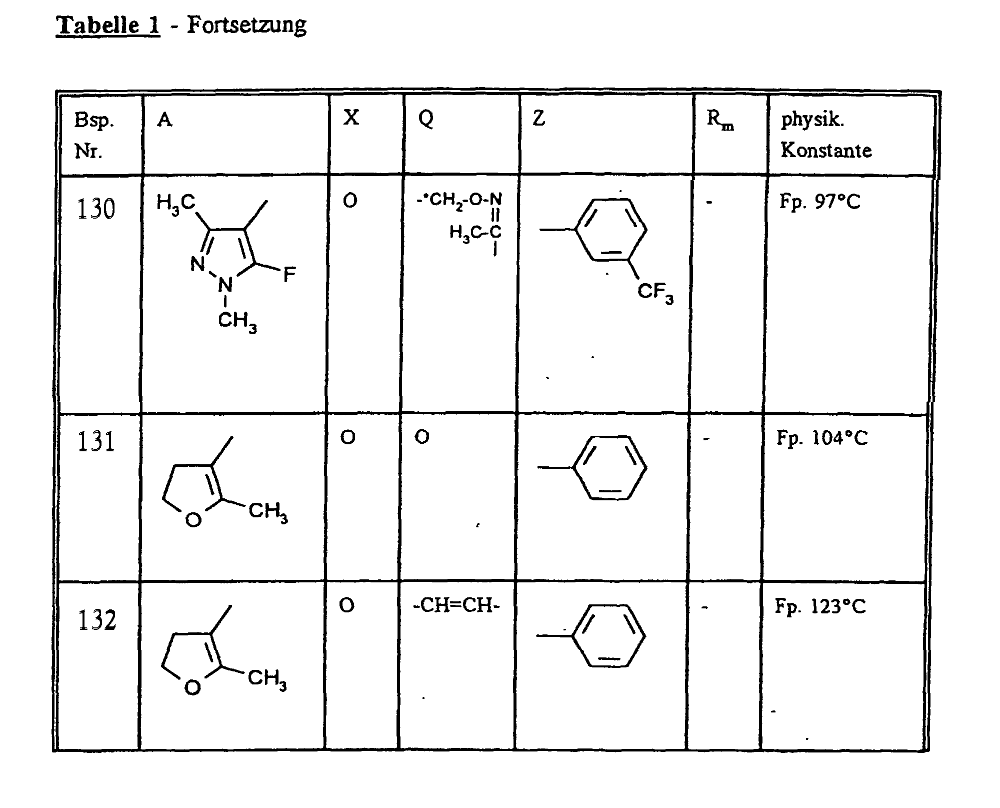

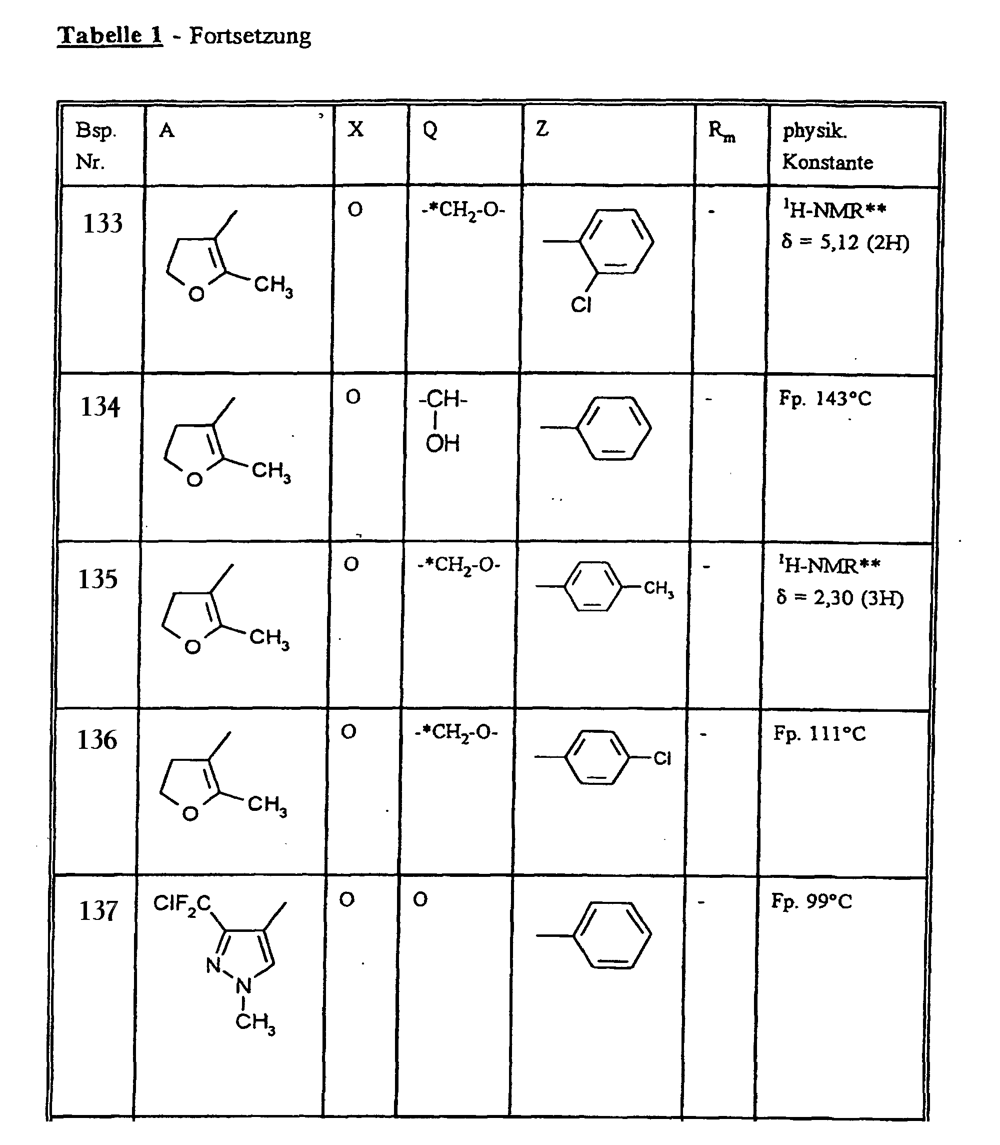

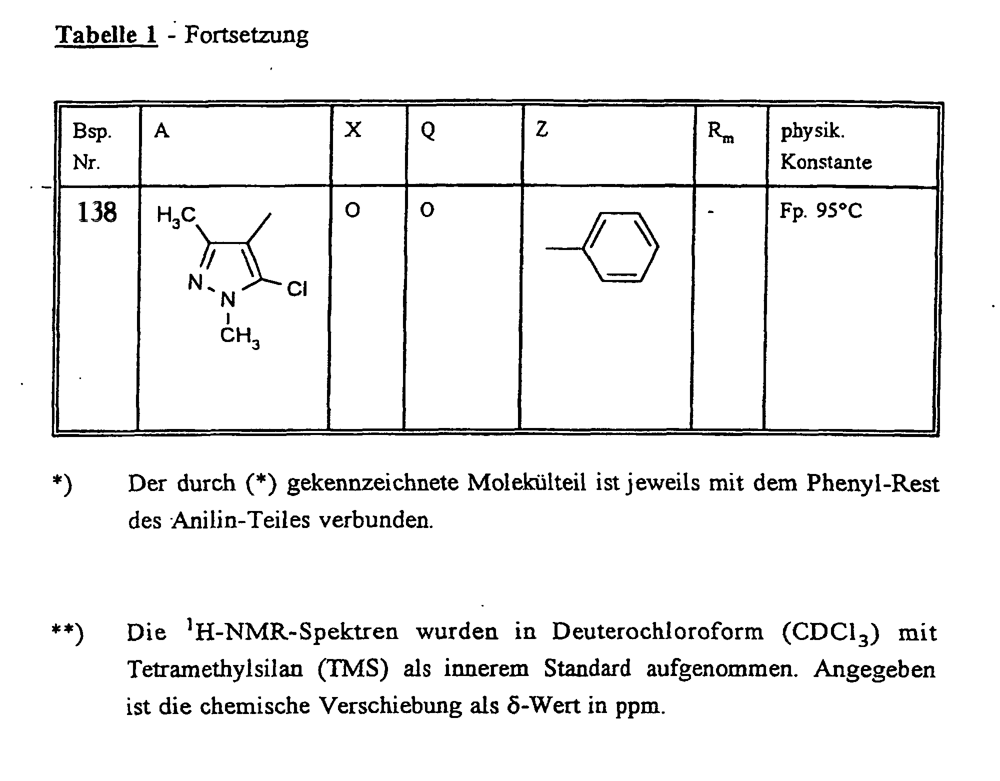

### Verwendungsbeispiele

### Beispiel A

### Podosphaera-Test (Apfel) / protektiv

- Lösungsmittel:: 47 Gewichtsteile Aceton
- Emulgator:: 3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung taufeucht besprüht. Nach Antrocknen des Spritzbelages werden die Pflanzen durch Bestäuben mit Konidien des Apfelmehltauerregers Podosphaera leucotricha inokuliert.

Die Pflanzen werden dann im Gewächshaus bei 23°C und einer relativen Luftfeuchtigkeit von ca. 70 % aufgestellt.

10 Tage nach der Inokulation erfolgt die Auswertung. Dabei bedeutet 0 % ein Wirkungsgrad, der demjenigen der unbehandelten Kontrolle entspricht, während ein Wirkungsgrad von 100 % bedeutet, daß kein Befall beobachtet wird.

Wirkstoffe, Wirkstoffkonzentrationen und Versuchsergebnisse gehen aus der folgenden Tabelle hervor.

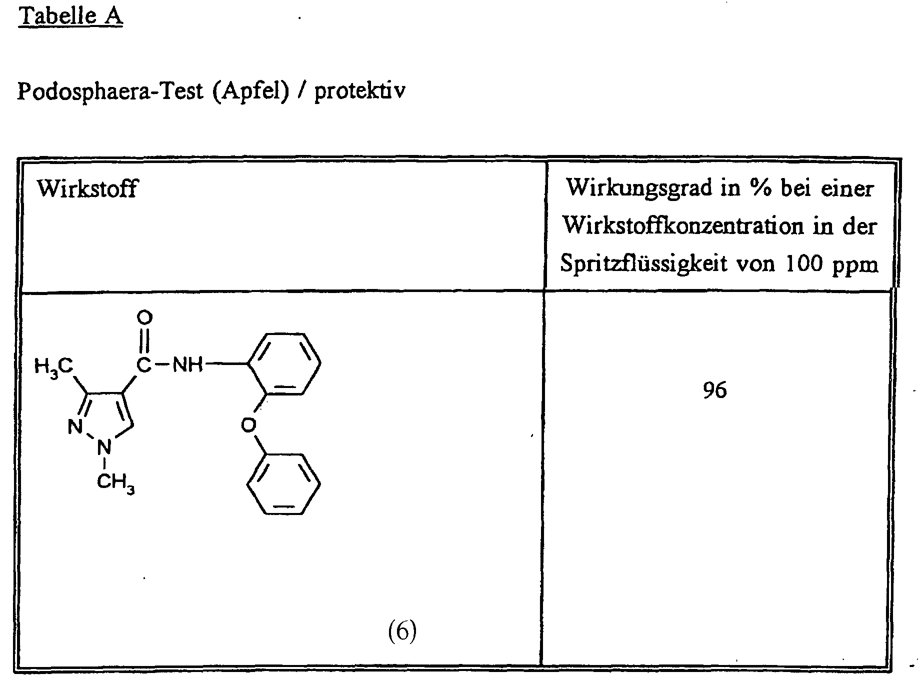

### Beispiel B

### Venturia-Test (Apfel) / protektiv

- Lösungsmittel:: 47 Gewichtsteile Aceton
- Emulgator:: 3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung taufeucht besprüht. Nach Antrocknen des Spritzbelages werden die Pflanzen mit einer wäßrigen Konidiensuspension des Apfelschorferregers Venturia inaequalis inokuliert und verbleiben dann 1 Tag bei 20°C und 100 % relativer Luftfeuchtigkeit in einer Inkubationskabine.

Die Pflanzen werden dann im Gewächshaus bei 23°C und einer relativen Luftfeuchtigkeit von ca. 70 % aufgestellt.

12 Tage nach der Inokulation erfolgt die Auswertung. Dabei bedeutet 0 % ein Wirkungsgrad, der demjenigen der unbehandelten Kontrolle entspricht, während ein Wirkungsgrad von 100 % bedeutet, daß kein Befall beobachtet wird

Wirkstoffe, Wirkstoffkonzentrationen und Versuchsergebnisse gehen aus der folgenden Tabelle hervor.

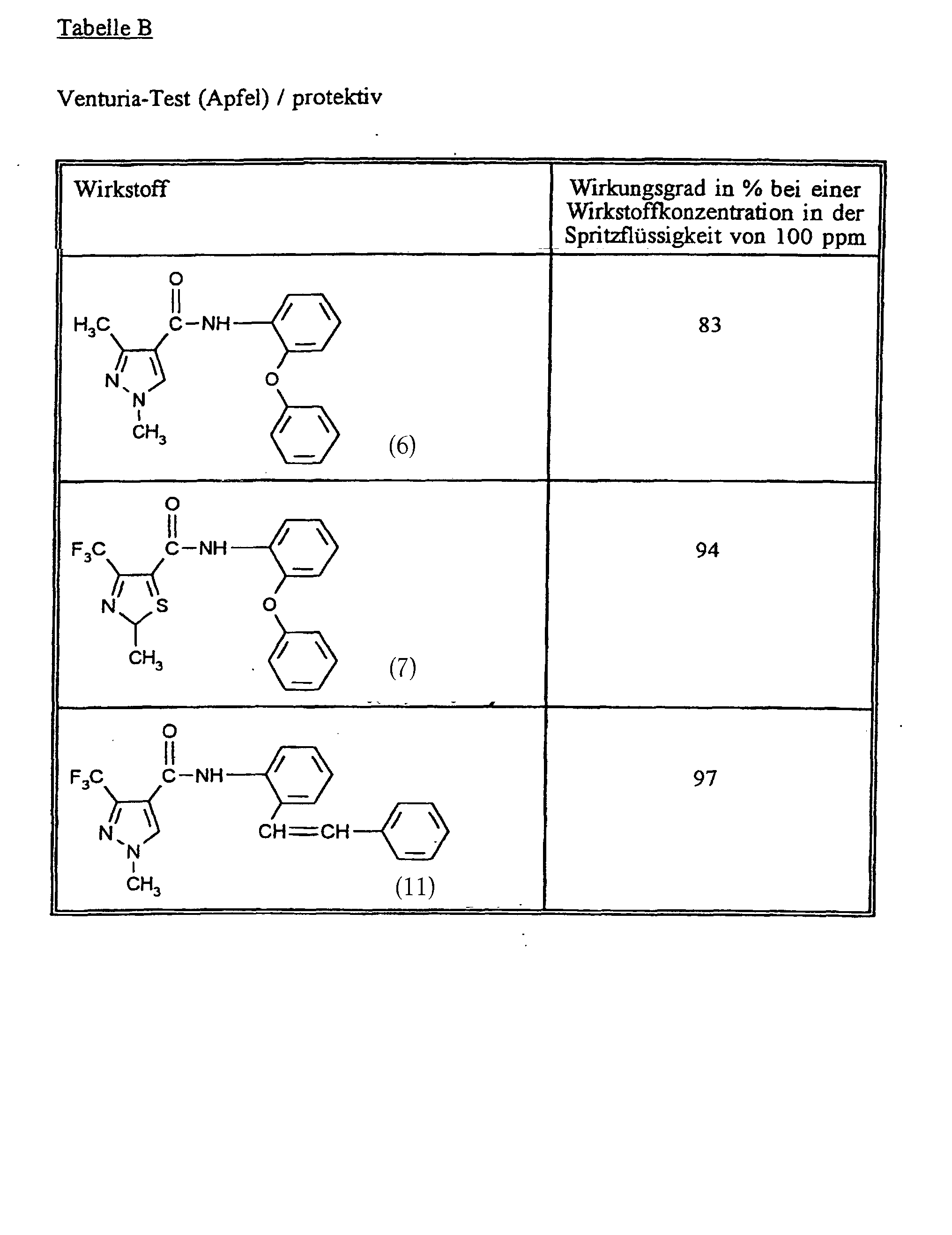

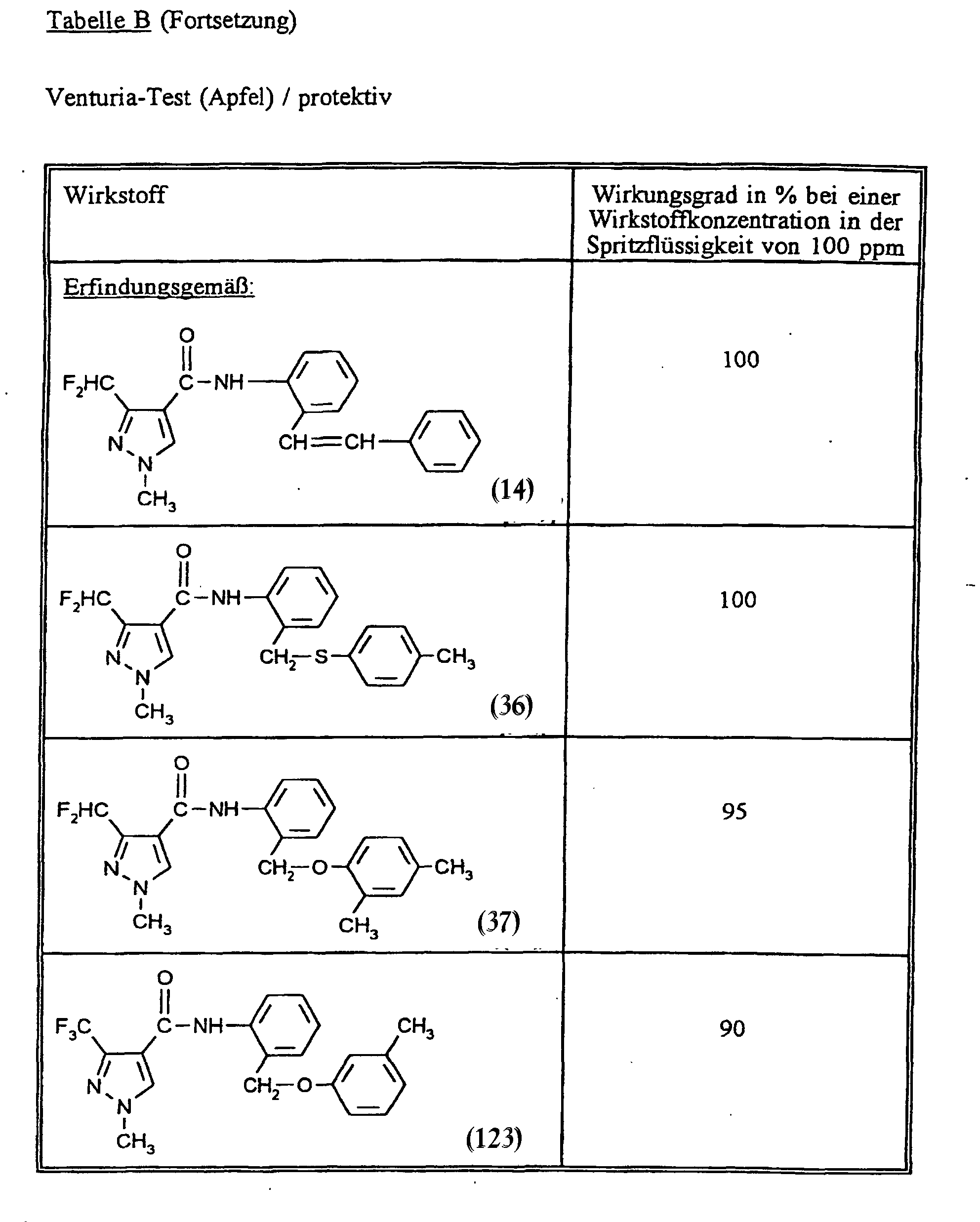

### Beispiel C

### Pyrenophora teres-Test (Gerste) / protektiv

- Lösungsmittel:: 10 Gewichtsteile N-Methyl-pyrrolidon
- Emulgator:: 0,6 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung in der angegebenen Aufwandmenge.

Nach Antrocknen des Spritzbelages werden die Pflanzen mit einer Konidiensuspension von Pyrenophora teres besprüht.

Die Pflanzen verbleiben 48 Stunden bei 20°C und 100 % relativer Luftfeuchtigkeit in einer Inkubationskabine.

Die Pflanzen werden in einem Gewächshaus bei einer Temperatur von ca. 20°C und einer relativen Luftfeuchtigkeit von ca. 80 % aufgestellt.

7 Tage nach der Inokulation erfolgt die Auswertung. Dabei bedeutet 0 % ein Wirkungsgrad, der demjenigen der unbehandelten Kontrolle entspricht, während ein Wirkungsgrad von 100 % bedeutet, daß kein Befall beobachtet wird.

Wirkstoffe, Wirkstoffkonzentrationen und Versuchsergebnisse gehen aus der folgenden Tabelle hervor.

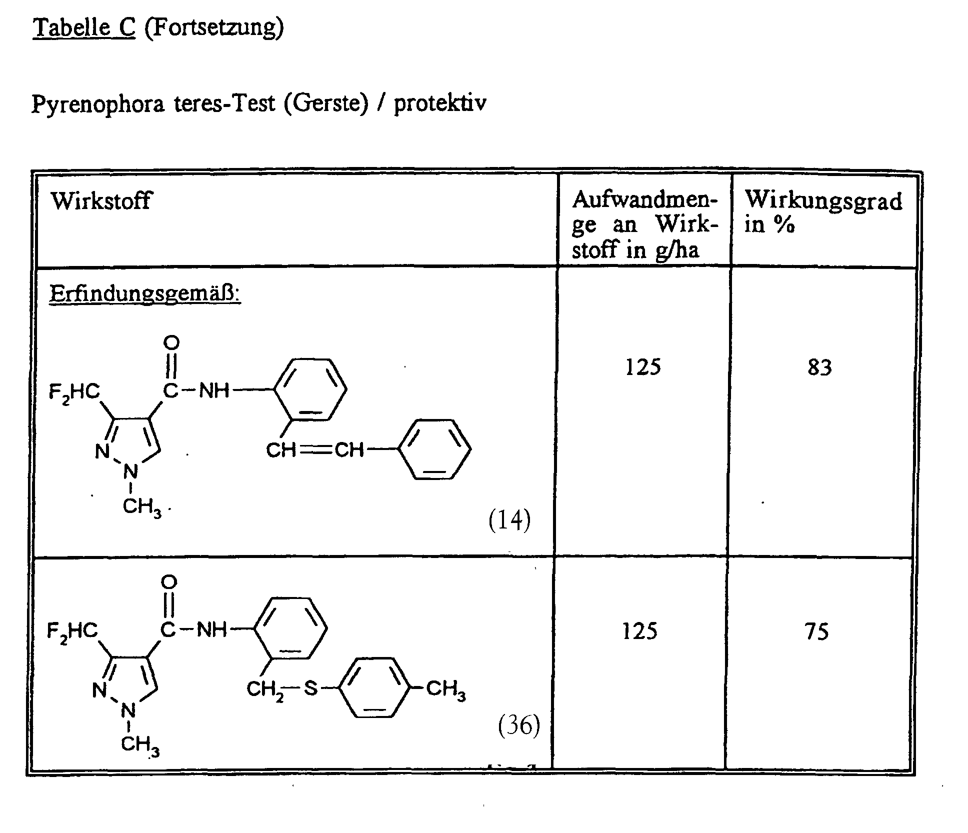

### Beispiel D

### Pseudocercosporella herpotrichoides-Test; R-Stamm

### (Weizen) / protektiv

- Lösungsmittel:: 10 Gewichtsteile N-Methyl-pyrrolidon
- Emulgator:: 0,6 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung in der angegebenen Aufwandmenge.

Nach Antrocknen des Spritzbelages werden die Pflanzen an der Halmbasis mit Sporen des R-Stammes von Pseudocercosporella herotrichoides inokuliert.

Die Pflanzen werden in einem Gewächshaus bei einer Temperatur von ca. 10°C und einer relativen Luftfeuchtigkeit von ca. 80 % aufgestellt.

21 Tage nach der Inokulation erfolgt die Auswertung. Dabei bedeutet 0 % ein Wirkungsgrad, der demjeinigen der Kontrolle entspricht, während ein Wirkungsgrad von 100 % bedeutet, daß kein Befall beobachtet wird.

Wirkstoffe, Wirkstoffkonzentrationen und Versuchsergebnisse gehen aus der folgenden Tabelle hervor.

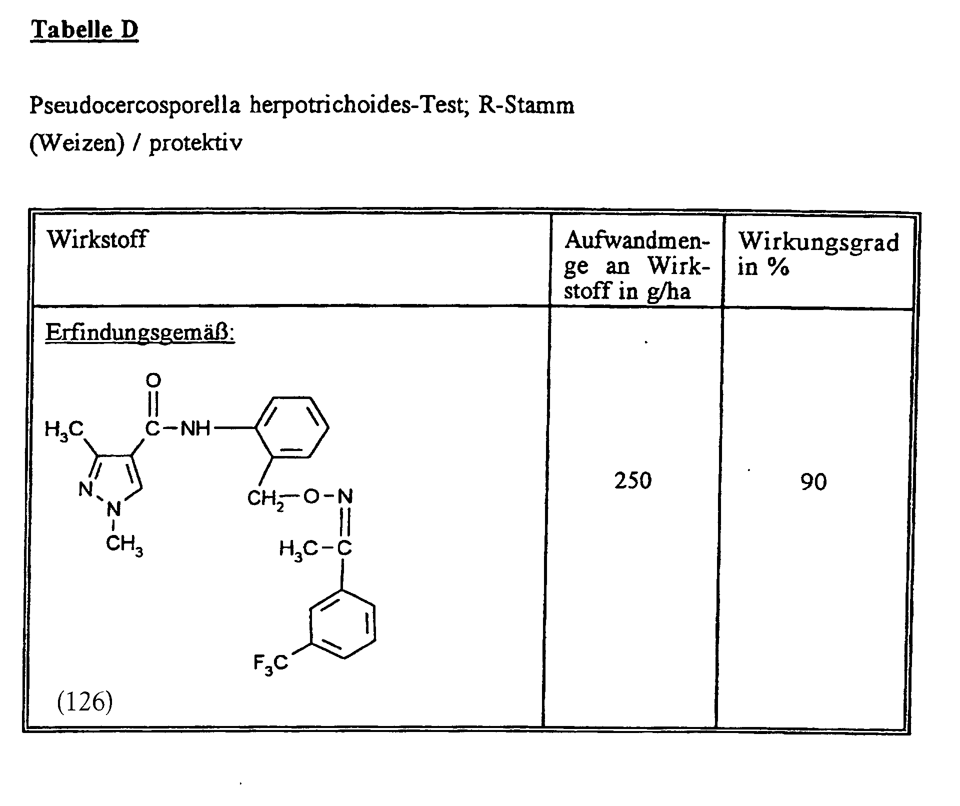

### Beispiel E

### Plutella-Test

- Lösungsmittel:: 7 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella maculipennis) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

Wirkstoffe, Wirkstoffkonzentrationen und Versuchsergebnisse gehen aus der folgenden Tabelle hervor.

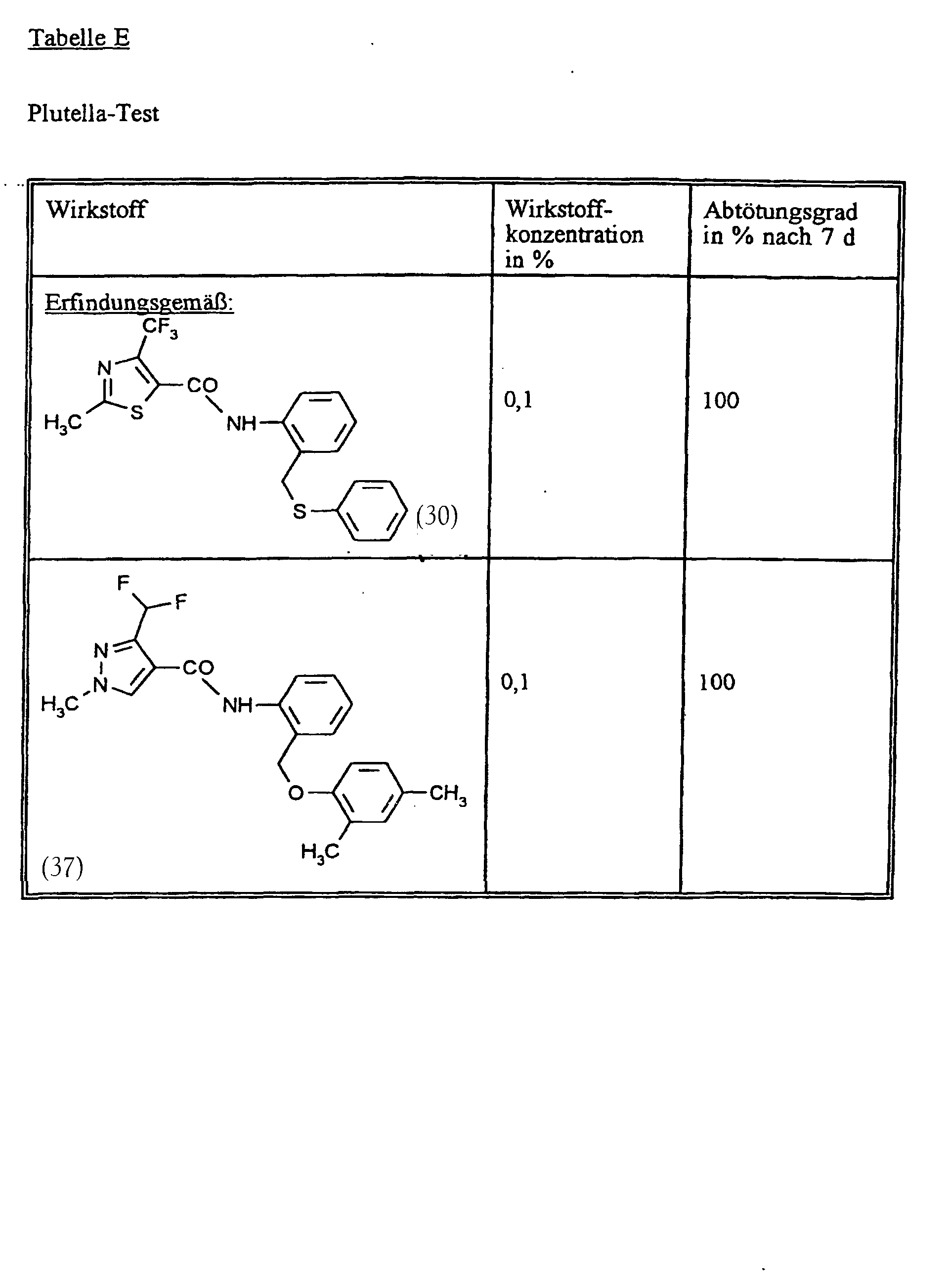

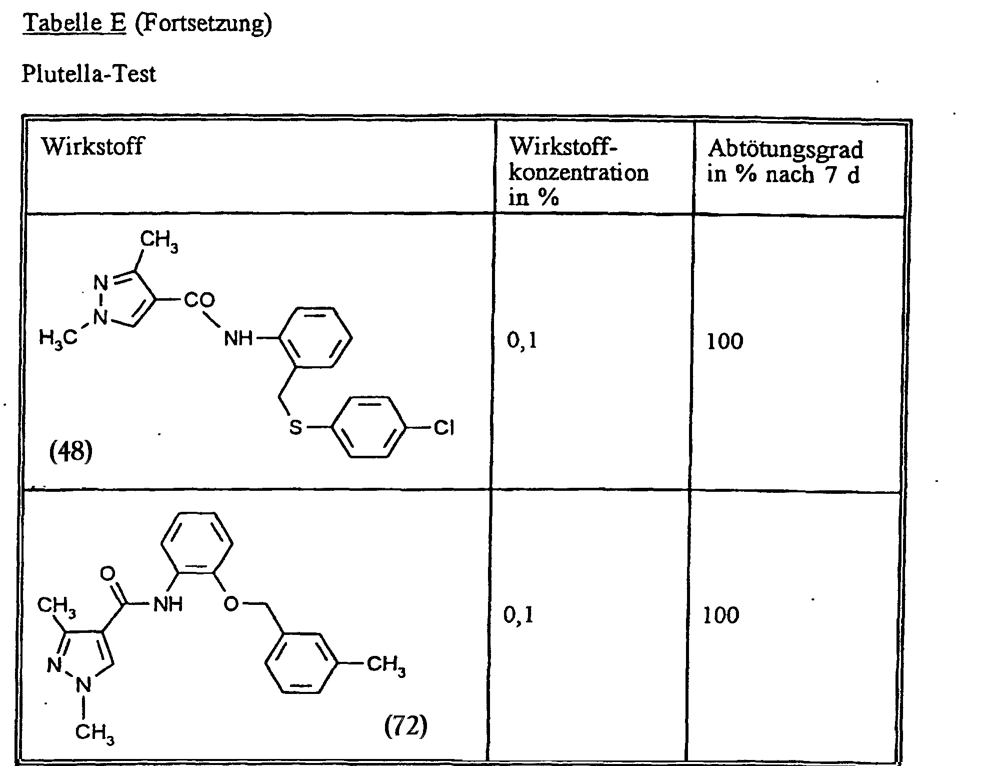

### Beispiel F

### Spodoptera-Test

- Lösungsmittel:: 7 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen des Eulenfalters (Spodoptera frugiperda) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

Wirkstoffe, Wirkstoffkonzentrationen und Versuchsergebnisse gehen aus der folgenden Tabelle hervor.

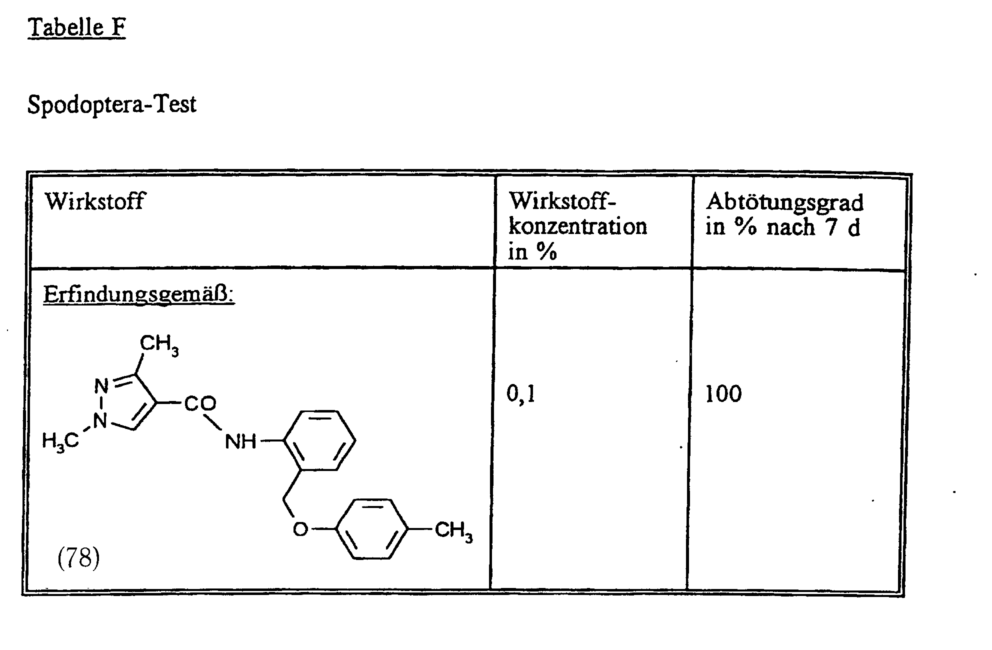

### Beispiel G

### Myzus-Test

- Lösungsmittel:: 7 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblatter (Brassica oleracea), die stark von der Pfirsichblattlaus (Myzus persicae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Blattläuse abgetötet wurden; 0 % bedeutet, daß keine Blattläuse abgetötet wurden.

Wirkstoffe, Wirkstoffkonzentrationen und Versuchsergebnisse gehen aus der folgenden Tabelle hervor.

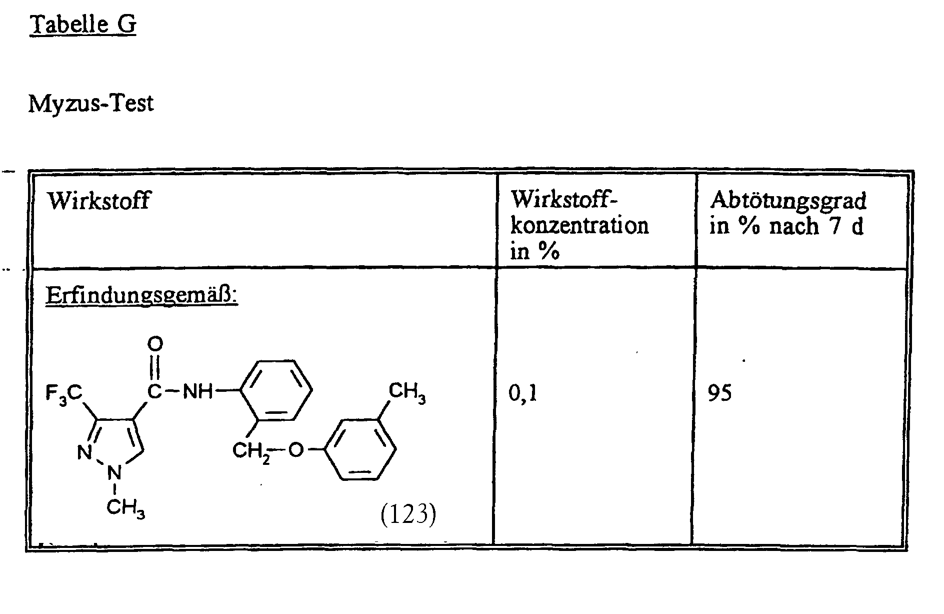

### Beispiel H

Hemmtest an Riesenkolonien von Basidiomyceten.
- Lösungsmittel:: Dimethylsulfoxid

Zur Herstellung einer zweckmäßigen Wirkstoffzubereitung werden 0,2 Gew.-Teile Wirkstoff mit 99,8 Gewichtsteilen des oben angegebenen Lösungsmittels versetzt.

Ein unter Verwendung von Malzextrakt-Pepton hergestellter Agar wird in flüssigem Zustand mit der Wirkstoffzubereitung in der jeweils gewünschten Aufwandmenge vermischt. Nach dem Aushärten wird der so hergestellte Nährstoffboden bei 26°C mit Mycelstücken inkubiert, die aus Kolonien von Coniophora puteana bzw Coriolus versicolor ausgestochen wurden.

Nach 3- bzw. 7-tägiger Lagerung bei 26°C erfolgt die Auswertung, indem das Hyphenwachstum gemessen und die im Vergleich zur unbehandelten Kontrolle aufgetretene Hemmung in Prozent bonitiert wird. Dabei bedeutet 0 % eine Wuchshemmung, die derjenigen der unbehandelten Kontrolle entspricht, während eine Wuchshemmung von 100 % bedeutet, daß kein Hyphenwachstum beobachtet wird.

Wirkstoffe, Wirkstoffkonzentrationen und Versuchsergebnisse gehen aus der folgenden Tabelle hervor.

**Tabelle H**

| Hemmtest an Riesenkolonien von Basidiomyceten | | | | | | |
|---|---|---|---|---|---|---|
| Wirkstoff gemäß Beispiel-Nr. | Hemmung des Hyphenwachstums in % bei | | | | | |
| | Coniophora puteana bei einer Wirkstoffkonzentration von | | | Coriolus versicolor bei einer Wirkstoffkonzentration von | | |
| | 1 ppm | 3 ppm | 6 ppm | 1 ppm | 3 ppm | 6 ppm |
| 4 | 70 | 80 | 100 | 70 | 90 | 100 |
| 11 | 80 | 100 | 100 | 100 | 100 | 100 |
| 13 | 70 | 100 | 100 | 70 | 100 | 100 |
| 14 | 100 | 100 | 100 | 100 | 100 | 100 |
| 29 | 70 | 80 | 90 | 90 | 100 | 100 |
| 30 | 50 | 60 | 70 | 40 | 70 | 90 |
| 35 | 70 | 80 | 90 | 90 | 100 | 100 |
| 36 | 80 | 100 | 100 | 100 | 100 | 100 |
| 37 | 100 | 100 | 100 | 100 | 100 | 100 |
| 39 | 80 | 90 | 100 | 90 | 100 | 100 |
| 48 | 70 | 90 | 100 | 90 | 90 | 100 |
| 50 | 80 | 80 | 100 | 90 | 100 | 100 |
| 51 | 70 | 80 | 80 | 70 | 70 | 90 |
| 77 | 100 | 100 | 100 | 100 | 100 | 100 |
| 80 | 100 | 100 | 100 | 100 | 100 | 100 |
| 87 | 100 | 100 | 100 | 100 | 100 | 100 |

### Beispiel J

### Plasmopara-Test (Rebe) / protektiv

- Lösungsmittel:: 47 Gewichtsteile Aceton
- Emulgator:: 3 Gewichtsteile Alkyl-Aryl-Polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Sporensuspension von Plasmopara viticola inokuliert und verbleiben dann 1 Tag in einer Inkubationskabine bei ca. 20°C und 100 % relativer Luftfeuchtigkeit. Anschließend werden die Pflanzen 5 Tage im Gewächshaus bei ca. 21°C und ca. 90 % Luftfeuchtigkeit aufgestellt. Die Pflanzen werden dann angefeuchtet und 1 Tag in eine Inkubationskabine gestellt.

6 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

### Beispiel K

### Venturia - Test (Apfel) / protektiv

- Lösungsmittel:: 47 Gewichtsteile Aceton
- Emulgator:: 3 Gewichtsteile Alkyl-Aryl-Polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Konidiensuspension des Apfelschorferregers Venturia inaequalis inokuliert und verbleiben dann 1 Tag bei ca. 20°C und 100°C relativer Luftfeuchtigkeit in einer Inkubationskabine.

Die Pflanzen werden dann im Gewächshaus bei ca. 21°C und einer relativen Luftfeuchtigkeit von ca. 90 % aufgestellt.

12 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachet wird.

## Patentansprüche

1. Carbanilide der Formel 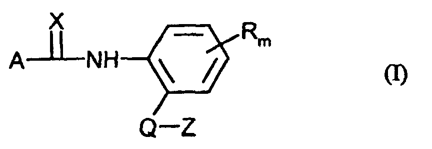 in welcher
R für Halogen, Nitro, Cyano, Alkyl mit 1 bis 8 Kohlenstoffatomen, Halogenalkyl mit 1 bis 6 Kohlenstoffatomen und 1 bis 5 Halogenatomen, Alkoxy mit 1 bis 8 Kohlenstoffatomen, Halogenalkoxy mit 1 bis 6 Kohlenstoffatomen und 1 bis 5 Halogenatomen, Alkylthio mit 1 bis 8 Kohlenstoffatomen, Halogenalkylthio mit 1 bis 6 Kohlenstoffatomen und 1 bis 5 Halogenatomen, Alkenyloxy mit 2 bis 8 Kohlenstoffatomen, Alkinyloxy mit 2 bis 8 Kohlenstoffatomen, Cycloalkyl mit 3 bis 8 Kohlenstoffatomen, Carbalkoxy mit 1 bis 8 Kohlenstoffatomen im Alkoxyteil oder Alkoximinoalkyl mit 1 bis 6 Kohlenstoffatomen im Alkoxyteil und 1 bis 6 Kohlenstoffatomen im Alkylteil steht,
m für die Zahlen 0, 1, 2, 3 oder 4 steht,
A für einen Rest der Formel 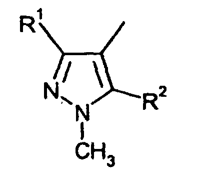 steht, worin
R¹ für Halogen, Alkyl mit 1 bis 4 Kohlenstoffatomen oder Halogenalkyl mit 1 bis 4 Kohlenstoffatomen und 1 bis 5 Halogenatomen steht und
R² für Wasserstoff, Halogen, Cyano oder Alkyl mit 1 bis 4 Kohlenstoffatomen steht, oder
oder
A für einen Rest der Formel 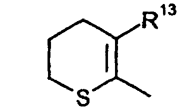 steht, worin
R¹³ für Alkyl mit 1 bis 4 Kohlenstoffatomen oder für Halogenalkyl mit 1 bis 4 Kohlenstoffatomen und 1 bis 5 Halogenatomen steht,
oder
A für einen Rest der Formel 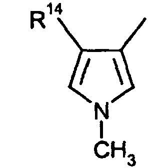 steht, worin
R¹⁴ für Halogen, Cyano, Alkyl mit 1 bis 4 Kohlenstoffatomen oder Halogenalkyl mit 1 bis 4 Kohlenstoffatomen und 1 bis 5 Halogenatomen steht,
oder
A für einen Rest der Formel 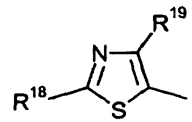 steht, worin
R¹⁸ für Wasserstoff, Halogen, Amino, Cyano oder Alkyl mit 1 bis 4 Kohlenstoffatomen steht und
R¹⁹ für Halogen, Alkyl mit 1 bis 4 Kohlenstoffatomen oder Halogenalkyl mit 1 bis 4 Kohlenstoffatomen und 1 bis 5 Halogenatomen steht,
oder
A für einen Rest der Formel 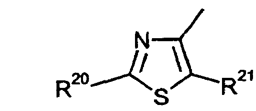 steht, worin
R²⁰ für Wasserstoff, Halogen, Amino, Cyano oder Alkyl mit 1 bis 4 Kohlenstoffatomen steht und
R²¹ für Halogen, Alkyl mit 1 bis 4 Kohlenstoffatomen oder Halogenalkyl mit 1 bis 4 Kohlenstoffatomen und 1 bis 5 Halogenatomen steht,
oder
A für einen Rest der Formel 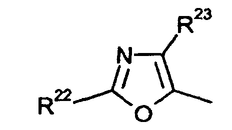 steht, worin
R²² für Wasserstoff oder Alkyl mit 1 bis 4 Kohlenstoffatomen steht und
R²³ für Halogen oder Alkyl mit 1 bis 4 Kohlenstoffatomen steht,
oder
A für einen Rest der Formel 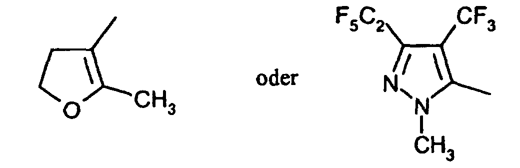 steht,
Q für Alkylen mit 1 bis 4 Kohlenstoffatomen, Alkenylen mit 2 bis 4 Kohlenstoffatomen, Alkinylen mit 2 bis 4 Kohlenstoffatomen oder eine Gruppe der Formel 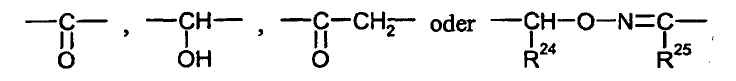 steht, worin
R²⁴ und R²⁵ unabhängig voneinander für Wasserstoff, Alkyl mit 1 bis 4 Kohlenstoffatomen, Cycloalkyl mit 3 bis 6 Kohlenstoffatomen, Alkenyl mit 2 bis 4 Kohlenstoffatomen oder Alkinyl mit 2 bis 4 Kohlenstoffatomen stehen, oder
Q für eine Gruppe der Formel 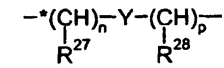 steht, worin
R²⁷ und R²⁸ unabhängig voneinander für Wasserstoff oder Alkyl mit 1 bis 4 Kohlenstoffatomen stehen,
Y für ein Sauerstoffatom oder für S(O)ᵣ steht, wobei
r für die Zahlen 0, 1 oder 2 steht, und
n und p unabhängig voneinander für die Zahlen 0, 1 oder 2 stehen,
wobei der durch (*) gekennzeichnete Molekülteil jeweils mit dem Phenylrest des Anilinteiles verbunden ist,
X für Sauerstoff oder Schwefel steht und
Z für gegebenenfalls substituiertes Phenyl, gegebenenfalls substituiertes Naphthyl, gegebenenfalls substituiertes Anthracenyl oder für gegebenenfalls substituiertes Hetaryl steht.

2. Carbanilide der Formel (I) gemäß Anspruch 1, in welcher
R für Fluor, Chlor, Brom, Nitro, Cyano, Alkyl mit 1 bis 6 Kohlenstoffatomen, Halogenalkyl mit 1 oder 2 Kohlenstoffatomen und 1 bis 5 Fluor-, Chlor- und/oder Bromatomen, Alkoxy mit 1 bis 6 Kohlenstoffatomen, Halogenalkoxy mit 1 oder 2 Kohlenstoffatomen und 1 bis 5 Fluor-, Chlor- und/oder Bromatomen, Alkylthio mit 1 bis 6 Kohlenstoffatomen, Halogenalkylthio mit 1 oder 2 Kohlenstoffatomen und 1 bis 5 Fluor-, Chlor- und/oder Bromatomen, Alkenyloxy mit 2 bis 6 Kohlenstoffatomen, Alkinyloxy mit 2 bis 6 Kohlenstoffatomen, Cycloalkyl mit 3 bis 7 Kohlenstoffatomen, Carbalkoxy mit 1 bis 4 Kohlenstoffatomen im Alkoxyteil oder für Alkoximinoalkyl mit 1 bis 4 Kohlenstoffatomen im Alkoxyteil und 1 bis 4 Kohlenstoffatomen im Alkylteil steht,
m für die Zahlen 0, 1, 2 oder 3 steht, wobei R für gleiche oder verschiedene Reste steht, wenn m für 2 oder 3 steht,
A für einen Rest der Formel 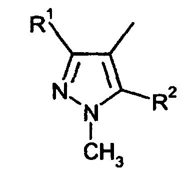 steht, worin
R¹ für Fluor, Chlor, Brom, Iod, Methyl, Ethyl, Isopropyl oder Halogenalkyl mit 1 oder 2 Kohlenstoffatomen und 1 bis 5 Fluor-, Chlor- und/oder Bromatomen steht und
R² für Wasserstoff, Fluor, Chlor, Brom, Iod, Methyl oder Ethyl steht,
oder
A für einen Rest der Formel 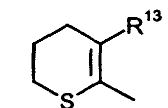 steht, worin
R¹³ für Methyl, Ethyl oder Halogenalkyl mit 1 oder 2 Kohlenstoffatomen und 1 bis 5 Fluor-, Chlor- und/oder Bromatomen steht,
oder
A für einen Rest der Formel 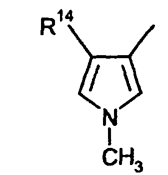 steht, worin
R¹⁴ für Fluor, Chlor, Brom, Cyano, Methyl, Ethyl oder Halogenalkyl mit 1 oder 2 Kohlenstoffatomen und 1 bis 5 Fluor-, Chlor- und/oder Bromatomen steht,
oder
A für einen Rest der Formel 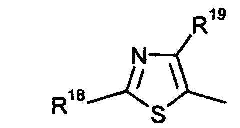 steht, worin
R¹⁸ für Wasserstoff, Fluor, Chlor, Brom, Amino, Cyano, Methyl oder Ethyl steht und
R¹⁹ für Fluor, Chlor, Brom, Methyl, Ethyl oder Halogenalkyl mit 1 oder 2 Kohlenstoffatomen und 1 bis 5 Fluor-, Chlor- und/oder Bromatomen steht,
oder
A für einen Rest der Formel 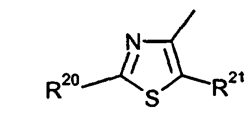 steht, worin
R²⁰ für Wasserstoff, Fluor, Chlor, Brom, Amino, Cyano, Methyl oder Ethyl steht und
R²¹ für Fluor, Chlor, Brom, Methyl, Ethyl oder Halogenalkyl mit 1 oder 2 Kohlenstoffatomen und 1 bis 5 Fluor-, Chlor- und/oder Bromatomen steht,
oder
A für einen Rest der Formel 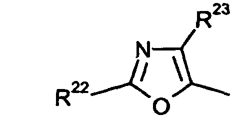 steht, worin
R²² für Wasserstoff, Methyl oder Ethyl steht und
R²³ für Fluor, Chlor, Brom, Methyl oder Ethyl steht,
oder
A für einen Rest der Formel 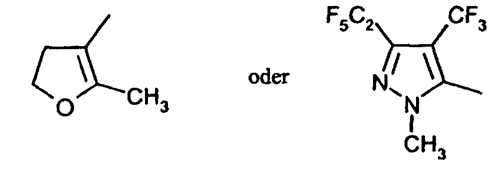 steht,
Q für Alkylen mit 1 bis 3 Kohlenstoffatomen, Alkenylen mit 2 oder 3 Kohlenstoffatomen, Alkinylen mit 2 oder 3 Kohlenstoffatomen oder für eine Gruppe der Formel 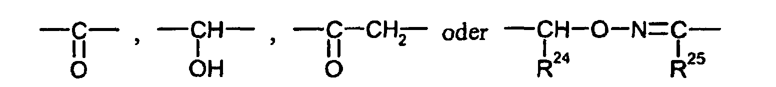 steht, worin
_{R}²⁴ und R²⁵ unabhängig voneinander für Wasserstoff, Methyl, Ethyl, Cyclopropyl, Cyclopentyl, Cyclohexyl, Alkenyl mit 2 oder 3 Kohlenstoffatomen oder Alkinyl mit 2 oder 3 Kohlenstoffatomen stehen, oder
Q für eine Gruppe der Formel 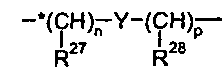 steht, worin
R²⁷ und R²⁸ unabhängig voneinander für Wasserstoff, Methyl oder Ethyl stehen,
Y für ein Sauerstoffatom oder für S(O)ᵣ steht, wobei
r für die Zahlen 0, 1 oder 2 steht, und
n und p unabhängig voneinander für die Zahlen 0, 1 oder 2 stehen,
wobei der durch (*) gekennzeichnete Molekülteil jeweils mit dem Phenylrest des Anilinteiles verbunden ist,
X für Sauerstoff oder Schwefel steht,
Z für Phenyl, Naphthyl oder Anthracenyl steht, wobei jeder dieser Reste einfach bis dreifach, gleichartig oder verschieden substituiert sein kann durch Halogen, Alkyl mit 1 bis 4 Kohlenstoffatomen, Alkylthio mit 1 bis 4 Kohlenstoffatomen, Halogenalkyl mit 1 bis 5 Fluor-, Chlor- und/oder Bromatomen, Halogenalkoxy mit 1 oder 2 Kohlenstoffatomen und 1 bis 5 Fluor-, Chlor- und/oder Bromatomen, Phenyl und/oder Phenoxy,
Z außerdem für Hetaryl mit 5 oder 6 Ringgliedern und 1 bis 3 Heteroatomen, wie Sauerstoff, Schwefel und/oder Stickstoff steht, wobei jeder der Heterocyclen einfach oder zweifach, gleichartig oder verschieden substituiert sein kann durch Halogen, Cyano, Nitro, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 Fluor-, Chlor- und/oder Bromatomen, Halogenalkoxy mit 1 oder 2 Kohlenstoffatomen und 1 bis 5 Fluor-, Chlor- und/oder Bromatomen, Phenyl und/oder Phenoxy.

3. Carbanilide der Formel (I) gemäß Anspruch 1, in welcher
R für Fluor, Chlor, Brom, Nitro, Cyano, Methyl, Ethyl, n-Propyl, isopropyl, n-Butyl, sek.-Butyl, i-Butyl, tert.-Butyl, Trichlormethyl, Trifluormethyl, Difluormethyl, Difluorchlormethyl, Methoxy, Ethoxy, Difluormethoxy, Trifluormethoxy, Difluorchlormethylthio, Allyloxy, Propargyloxy, Cyclopropyl, Cyclopentyl, Cyclohexyl, Cycloheptyl, Methoxycarbonyl, Ethoxycarbonyl, Methoximinomethyl, Ethoximinomethyl, Methoximinoethyl oder Ethoximinoethyl steht.
m für die Zahlen 0, 1, 2 oder 3 steht, wobei R für gleiche oder verschiedene Reste steht, wenn m für 2 oder 3 steht,
A für einen Rest der Formel 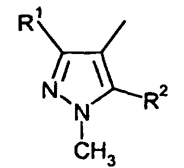 steht, worin
R¹ für Fluor, Chlor, Brom, Iod, Methyl, Ethyl, Isopropyl, Difluormethyl, Trifluormethyl, Difluorchlormethyl oder Trichlormethyl steht und
R² für Wasserstoff, Fluor, Chlor, Brom, Iod, Methyl oder Ethyl steht,
oder
A für einen Rest der Formel 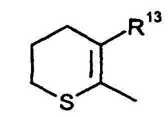 steht, worin
R¹³ für Methyl, Ethyl, Trifluormethyl, Difluormethyl, Difluorchlormethyl oder Trichlormethyl steht,
oder
A für einen Rest der Formel 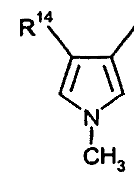 steht, worin
R¹⁴ für Fluor, Chlor, Brom, Cyano, Methyl, Ethyl, Trifluormethyl, Difluormethyl, Difluorchlormethyl oder Trichlormethyl steht,
oder
A für einen Rest der Formel 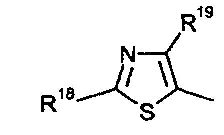 steht, worin
R¹⁸ für Wasserstoff, Fluor, Chlor, Brom, Amino, Cyano, Methyl oder Ethyl steht und
R¹⁹ für Fluor, Chlor, Brom, Methyl, Ethyl, Trifluormethyl, Difluormethyl, Difluorchlormethyl oder Trichlormethyl steht,
oder
A für einen Rest der Formel 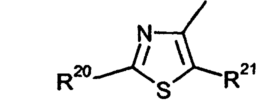 steht, worin
R²⁰ für Wasserstoff, Fluor, Chlor, Brom, Amino, Cyano, Methyl oder Ethyl steht und
R²¹ für Fluor, Chlor, Brom, Methyl, Ethyl, Trifluormethyl, Difluormethyl, Difluorchlormethyl oder Trichlormethyl steht,
oder
A für einen Rest der Formel 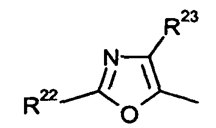 steht, worin
R²² für Wasserstoff, Methyl oder Ethyl steht und
R²³ für Fluor, Chlor, Brom, Methyl oder Ethyl steht,
oder
A für einen Rest der Formel 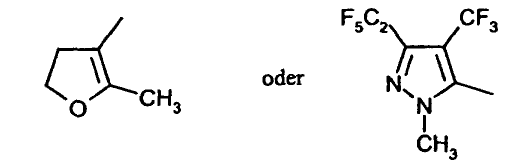 steht,
Q für Alkylen mit 1 bis 3 Kohlenstoffatomen, Alkenylen mit 2 oder 3 Kohlenstoffatomen, Alkinylen mit 2 oder 3 Kohlenstoffatomen oder für eine Gruppe der Formel 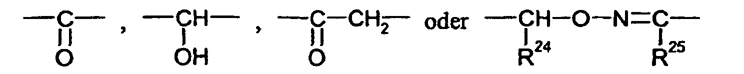 steht, worin
R²⁴ und R²⁵ unabhängig voneinander für Wasserstoff, Methyl, Ethyl, Cyclopropyl, Cyclopentyl, Cyclohexyl, Allyl oder Propargyl stehen, oder
Q für eine Gruppe der Formel 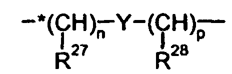 steht, worin
R²⁷ und R²⁸ unabhängig voneinander für Wasserstoff, Methyl oder Ethyl stehen,
Y für ein Sauerstoffatom oder für S(O)ᵣ steht, wobei
r für die Zahlen 0, 1 oder 2 steht, und
n und p unabhängig voneinander für die Zahlen 0, 1 oder 2 stehen,
wobei der durch (*) gekennzeichnete Molekülteil jeweils mit dem Phenylrest des Anilinteiles verbunden ist.
X für Sauerstoff oder Schwefel steht,
Z für Phenyl, Naphthyl oder Anthracenyl steht, wobei jeder dieser Reste einfach bis dreifach, gleichartig oder verschieden substituiert sein kann durch Fluor, Chlor, Brom, Methyl, Ethyl, n-Propyl, Isopropyl, n-Butyl, sek.-Butyl, i-Butyl, tert.-Butyl, Methoxy, Ethoxy, Methylthio, Ethylthio, Trifluormethyl, Difluormethyl, Difluorchlormethyl, Trichlormethyl, Trifluormethoxy, Difluormethoxy, Difluorchlormethoxy, Trichlormethoxy, Phenyl und/oder Phenoxy,
Z außerdem für Pyrrolyl, Furyl, Pyrazolyl, Imidazolyl, Thiazolyl, isothiazolyl, Oxazolyl, Isoxazolyl, Oxadiazolyl, Thiadiazolyl, 1,2,3-Triazinyl, 1,2,4-Triazinyl, 1,3,5-Triazinyl, Pyridyl, Pyrimidyl, Pyrazinyl oder Pyridazinyl steht, wobei jeder dieser Reste einfach oder zweifach, gleichartig oder verschieden substituiert sein kann durch Fluor, Chlor, Brom, Cyano, Nitro, Methyl, Ethyl, n-Propyl, Isopropyl, n-Butyl, sek.-Butyl, i-Butyl, tert.-Butyl, Methoxy, Ethoxy, Methylthio, Ethylthio, Trifluormethyl, Difluormethyl, Difluorchlormethyl, Trichlormethyl, Trifluormethoxy, Difluormethoxy, Difluorchlormethoxy, Trichlormethoxy, Phenyl und/oder Phenoxy.

4. Verfahren zur Herstellung von Carbaniliden der Formel (I) gemäß Anspruch 1, **dadurch gekennzeichnet, dass** man
a) Säurehalogenide der Formel 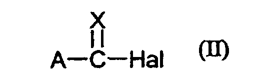 in welcher
A und X die in Anspruch 1 angegebenen Bedeutungen haben und
Hal für Halogen steht,
mit Anilin-Derivaten der Formel 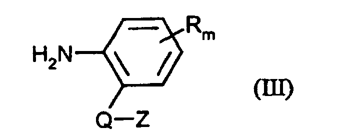 in welcher
Q, R, Z und m die in Anspruch 1 angegebenen Bedeutungen haben,
gegebenenfalls in Gegenwart eines Säurebindemittels und gegebenenfalls in Gegenwart eines Verdünnungsmittels umsetzt, oder
b) Carbanilid-Derivate der Formel 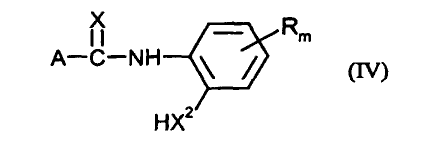 in welcher
A, R, X und m die in Anspruch 1 angegebenen Bedeutungen haben und
X² für Sauerstoff oder Schwefel steht,
mit Verbindungen der Formel (V) 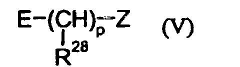 in welcher
R²⁸, Z und p die in Anspruch 1 angegebenen Bedeutungen haben und
E für eine Austrittsgruppe steht,
gegebenenfalls in Gegenwart eines Säurebindemittels und gegebenenfalls in Gegenwart eines Verdünnungsmittels umsetzt, oder
c) Carbanilid-Derivate der Formel 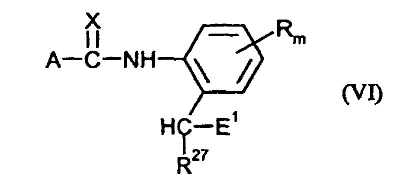 in welcher
A, R, R²⁷, X und m die in Anspruch 1 angegebenen Bedeutungen haben, und
E¹ für eine Austrittsgruppe steht,
mit Verbindungen der Formel (VII) 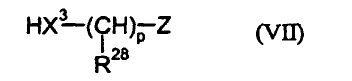 in welcher
R²⁸, Z und p die in Anspruch 1 angegebenen Bedeutungen haben und
X³ für Sauerstoff oder Schwefel steht,
gegebenenfalls in Gegenwart eines Säurebindemittels und gegebenenfalls in Gegenwart eines Verdünnungsmittels umsetzt, oder
d) Carbanilid-Derivate der Formel 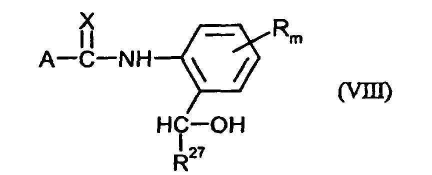
in welcher
A, R, R²⁷, X und m die in Anspruch 1 angegebenen Bedeutungen haben,
mit Verbindungen der Formel (V) 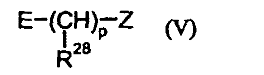 in welcher
R²⁸, Z und p die in Anspruch 1 angegebenen Bedeutungen haben und
E für eine Austrittsgruppe steht,
gegebenenfalls in Gegenwart eines Säurebindemittels und gegebenenfalls in Gegenwart eines Verdünnungsmittels umsetzt.

5. Schädlingsbekämpfungsmittel, **gekennzeichnet durch** einen Gehalt an mindestens einem Carbanilid der Formel (I) gemäß Anspruch 1.

6. Verwendung von Carbaniliden der Formel (I) gemäß Anspruch 1 zur Bekämpfung von Schädlingen.

7. Verfahren zur Bekämpfung von Schädlingen, **dadurch gekennzeichnet, dass** man Carbanilide der Formel (I) gemäß Anspruch 1 auf die Schädlinge und/oder deren Lebensraum ausbringt.

8. Verfahren zur Herstellung von Schädlingsbekämpfungsmitteln, **dadurch gekennzeichnet, dass** man Carbanilide der Formel (I) gemäß Anspruch 1 mit Streckmitteln und/oder oberflächenaktiven Stoffen vermischt.

## Claims

1. Carbanilides of the formula 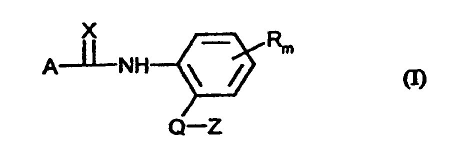 in which
R represents halogen, nitro, cyano, alkyl having 1 to 8 carbon atoms, haloalkyl having 1 to 6 carbon atoms and 1 to 5 halogen atoms, alkoxy having 1 to 8 carbon atoms, halgenoalkoxy having 1 to 6 carbon atoms and 1 to 5 halogen atoms, alkylthio having 1 to 8 carbon atoms, haloalkylthio having 1 to 6 carabon atoms and 1 to 5 halogen atoms, alkenyloxy having 2 to 8 carbon atoms, alkynyloxy having 2 to 8 carbon atoms, cycloalkyl having 3 to 8 carbon atoms, carbalkoxy having 1 to 8 carbon atoms in the alkoxy moiety or alkoximinoalkyl having 1 to 6 carbon atoms in the alkoxy moiety and 1 to 6 carbon atoms in the alkyl moiety,
m represents numbers 0, 1, 2, 3 or 4,
A represents a radical of the formula 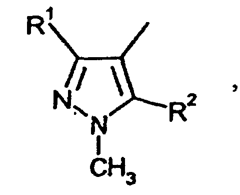 in which
R1 represents halogen, alkyl having 1 to 4 carbon atoms or haloalkyl having 1 to 4 carbon atoms and 1 to 5 halogen atoms and
R2 represents hydrogen, halogen, cyano or alkyl having 1 to 4 carbon atoms, or
A represents a radical of the formula 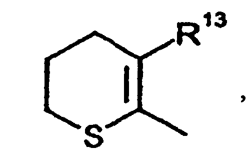 in which
R¹³ represents alkyl having 1 to 4 carbon atoms or represents haloalkyl having 1 to 4 carbon atoms and 1 to 5 halogen atoms,
or
A represents a radical of the formula 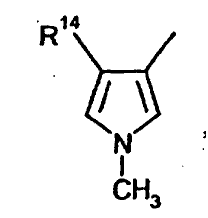 in which
R¹⁴ represents halogen, cyano, alkyl having 1 to 4 carbon atoms or haloalkyl having 1 to 4 carbon atoms and 1 to 5 halogen atoms,
or
A represents a radical of the formula 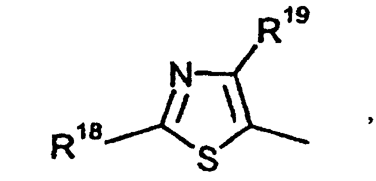 in which
R¹⁸ represents hydrogen, halogen, amino, cyano or alkyl having 1 to 4 carbon atoms and
R¹⁹ represents halogen, alkyl having 1 to 4 carbon atoms or haloalkyl having 1 to 4 carbon atoms and 1 to 5 halogen atoms,
or
A represents a radical of the formula 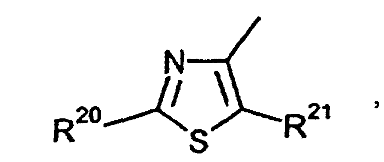 in which
R²⁰ represents hydrogen, halogen, amino, cyano or alkyl having 1 to 4 carbon atoms and
R²¹ represents halogen alkyl having 1 to 4 carbon atoms or haloalkyl having 1 to 4 carbon atoms and 1 to 5 halogen atoms,
or
A represents a radical of the formula 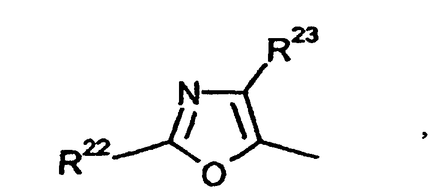 in which
R²² represents hydrogen or alkyl having 1 to 4 carbon atoms and
R²³ represents halogen or alkyl having 1 to 4 carbon atoms,
or
A represents a radical of the formula 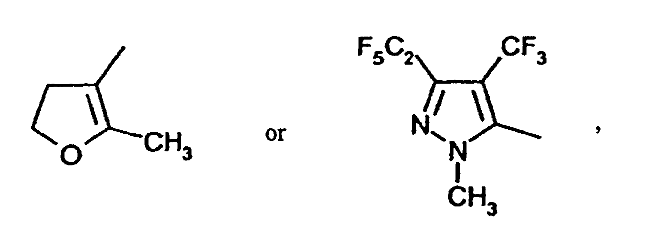
Q represents alkylene having 1 to 4 carbon atoms, alkenylene having 2 to 4 carbon atoms, alkynylene having 2 to 4 carbon atoms or a group of the formula 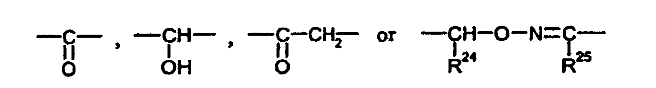 in which
R²⁴ and R²⁵ independently of one another each represent hydrogen, alkyl having 1 to 4 carbon atoms, cycloalkyl having 3 to 6 carbon atoms, alkenyl having 2 to 4 carbon atoms or alkynyl having 2 to 4 carbon atoms, or
Q represents a group of the formula 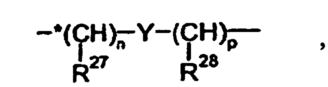 in which
R²⁷ and R²⁸ independently of one another each represent hydrogen or alkyl having 1 to 4 carbon atoms,
Y represents an oxygen atom or represents S(O)ᵣ, where
r represents the numbers 0, 1 or 2, and
n and p independently of one another each represent the numbers 0, 1 or 2,
where the molecular moiety labeled (*) is in each case attached to the pheynyl radical of the aniline moiety,
X represents oxygen or sulphur and
Z represents optionally substituted phenyl, optionally substituted naphthyl, optionally substituted anthracenyl or represents optionally substituted hetaryl.

2. Carbanilides of the formula (I) according to Claim 1 in which
R represents fluorine, chlorine, bromine, nitro, cyano, alkyl having 1 to 6 carbon atoms, haloalkyl having 1 or 2 carbon atoms and 1 to 5 fluorine, chlorine and/or bromine atoms, alkoxy having 1 to 6 carbon atoms, haloalkoxy having 1 or 2 carbon atoms and 1 to 5 fluorine, chlorine and/or bromine atoms, alkylthio having 1 to 6 carbon atoms, haloalkylthio having 1 or 2 carbon atoms and 1 to 5 fluorine, chlorine and/or bromine atoms, alkenyloxy having 2 to 6 carbon atoms, alkynyloxy having 2 to 6 carbon atoms, cycloalkyl having 3 to 7 carbon atoms, carbalkoxy having 1 to 4 carbon atoms in the alkoxy moiety or represents alkoximinoalkyl having 1 to 4 carbon atoms in the alkoxy moiety and 1 to 4 carbon atoms in the alkyl moiety,
m represents the numbers 0, 1, 2 or 3, where R represents identical or different radicals if m represents 2 or 3,
A represents a radical of the formula 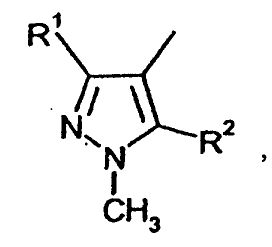 in which
R¹ represents fluorine, chlorine, bromine, iodine, methyl, ethyl, isopropyl or haloalkyl having 1 or 2 carbon atoms and 1 to 5 fluorine, chlorine and/or bromine atoms and
R² represents hydrogen, fluorine, chlorine, bromine, iodine, methyl or ethyl,
or
A represents a radical of the formula 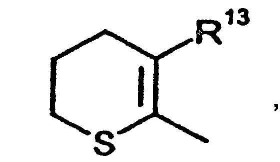 in which
R¹³ represents methyl, ethyl or haloalkyl having 1 or 2 carbon atoms and 1 to 5 fluorine, chlorine and/or bromine atoms,
or
A represents a radical of the formula 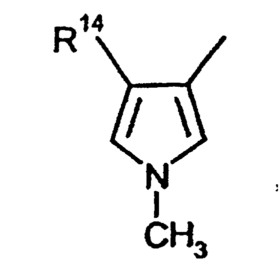 in which
R¹⁴ represents fluorine, chlorine, bromine, cyano, methyl, ethyl or haloalkyl having 1 or 2 carbon atoms and 1 to 5 fluorine, chlorine and/or bromine atoms,
or
A represents a radical of the formula 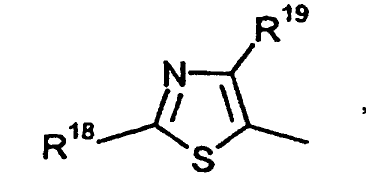 in which
R¹⁸ represents hydrogen, fluorine, chlorine, bromine, amino, cyano, methyl or ethyl and
R¹⁹ represents fluorine, chlorine, bromine, methyl, ethyl or haloalkyl having 1 or 2 carbon atoms and 1 to 5 fluorine, chlorine and/or bromine atoms,
or
A represents a radical of the formula 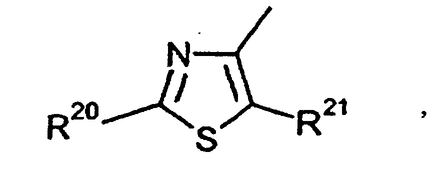 in which
R²⁰ represents hydrogen, fluorine, chlorine, bromine, amino, cyano, methyl or ethyl and
R²¹ represents fluorine, chlorine, bromine, methyl, ethyl or haloalkyl having 1 or 2 carbon atoms and 1 to 5 fluorine, chlorine and/or bromine atoms,
or
A represents a radical of the formula 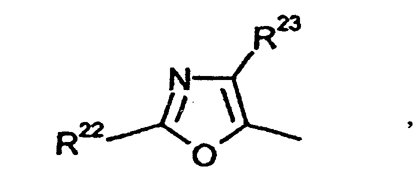 in which
R²² represents hydrogen, methyl or ethyl and
R²³ represents fluorine, chlorine, bromine, methyl or ethyl,
or
A represents a radical of the formula 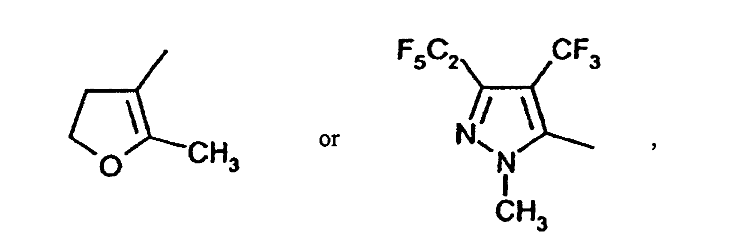
Q represents alkylene having 1 to 3 carbon atoms, alkenylene having 2 or 3 carbon atoms, alkynylene having 2 or 3 carbon atoms or represents a group of the formula 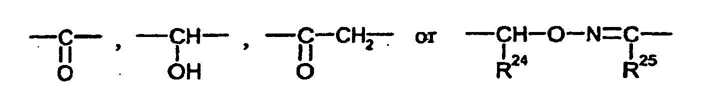 in which
R²⁴ and R²⁵ independently of one another represent hydrogen, methyl, ethyl, cyclopropyl, cyclopentyl, cyclohexyl, alkenyl having 2 or 3 carbon atoms or alkynyl having 2 or 3 carbon atoms, or
Q represents a group of the formula 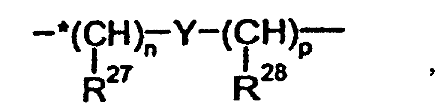 in which
R²⁷ and R²⁸ independently of one another represent hydrogen, methyl or ethyl,
Y represents an oxygen atom or represents S(O)ᵣ, where
r represents the numbers 0, 1 or 2, and
n and p independently of one another represent the numbers 0, 1 or 2,
where the molecular moiety labelled (*) is in each case attached to the phenyl radical of the aniline moiety,
X represents oxygen or sulphur,
Z represents phenyl, naphthyl or anthracenyl, where each of these radicals may be mono- to trisubstituted by identical or different substituents from the group consisting of halogen, alkyl having 1 to 4 carbon atoms, alkylthio having 1 to 4 carbon atoms, haloalkyl having 1 to 5 fluorine, chlorine and/or bromine atoms, haloalkoxy having 1 or 2 carbon atoms and 1 to 5 fluorine, chlorine and/or bromine atoms, phenyl and/or phenoxy,
Z furthermore represents hetaryl having 5 or 6 ring members and 1 to 3 heteroatoms, such as oxygen, sulphur and/or nitrogen, where each of these heterocycles may be mono- or disubstituted by identical or different substituents from the group consisting of halogen, cyano, nitro, alkyl having 1 to 4 carbon atoms, alkoxy having 1 to 4 carbon atoms, alkylthio having 1 to 4 carbon atoms, haloalkyl having 1 or 2 carbon atoms and 1 to 5 fluorine, chlorine and/or bromine atoms, haloalkoxy having 1 or 2 carbon atoms and 1 to 5 fluorine, chlorine and/or bromine atoms, phenyl and/or phenoxy.

3. Carbanilides of the formula (I) according to Claim 1 in which
R represents fluorine, chlorine, bromine, nitro, cyano, methyl, ethyl, n-propyl, isopropyl, n-butyl, sec-butyl, isobutyl, tert-butyl, trichloromethyl, trifluoromethyl, difluoromethyl, difluorochloromethyl, methoxy, ethoxy, difluoromethoxy, trifluoromethoxy, difluorochloromethylthio, allyloxy, propargyloxy, cyclopropyl, cyclopentyl, cyclohexyl, cycloheptyl, methoxycarbonyl, ethoxycarbonyl, methoximinomethyl, ethoximinomethyl, methoximinoethyl or ethoximinoethyl,
m represents the numbers 0, 1, 2 or 3, where R represents identical or different radicals if m represents 2 or 3,
A represents a radical of the formula 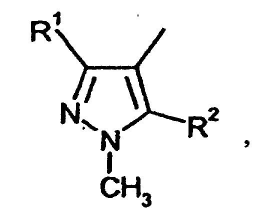 in which
R¹ represents fluorine, chlorine, bromine, iodine, methyl, ethyl, isopropyl, difluoromethyl, trifluoromethyl, difluorochloromethyl or trichloromethyl and
R² represents hydrogen, fluorine, chlorine, bromine, iodine, methyl or ethyl,
or
A represents a radical of the formula 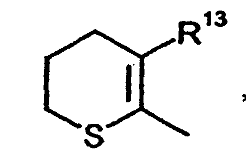 in which
R¹³ represents methyl, ethyl, trifluoromethyl, difluoromethyl, difluorochloromethyl or trichloromethyl,
or
A represents a radical of the formula 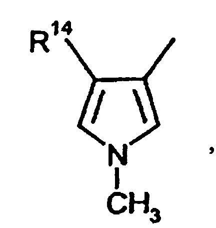 in which
R14 represents fluorine, chlorine, bromine, cyano, methyl, ethyl, trifluoromethyl, difluoromethyl, difluorochloromethyl or trichloromethyl,
or
A represents a radical of the formula 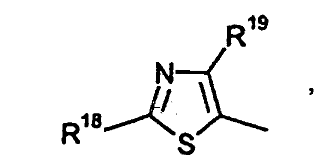 in which
R¹⁸ represents hydrogen, fluorine, chlorine, bromine, amino, cyano, methyl or ethyl and
R¹⁹ represents fluorine, chlorine, bromine, methyl, ethyl, trifluoromethyl, difluoromethyl, difluorochloromethyl or trichloromethyl,
or
A represents a radical of the formula 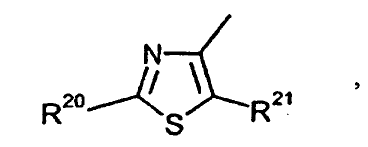 in which
R²⁰ represents hydrogen, fluorine, chlorine, bromine, amino, cyano, methyl or ethyl and
R²¹ represents fluorine, chlorine, bromine, methyl, ethyl, trifluoromethyl, difluoromethyl, difluorochloromethyl or trichloromethyl,
or
A represents a radical of the formula 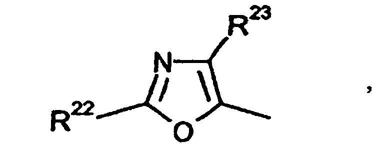 in which
R²² represents hydrogen, methyl or ethyl and
R²³ represents fluorine, chlorine, bromine, methyl or ethyl,
or
A represents a radical of the formula 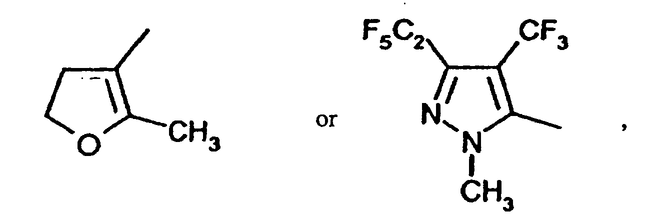
Q represents alkylene having 1 to 3 carbon atoms, alkenylene having 2 or 3 carbon atoms, alkynylene having 2 or 3 carbon atoms or represents a group of the formula 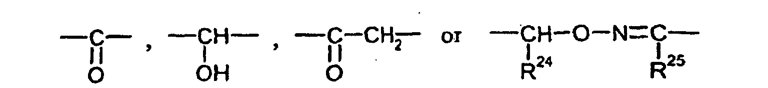 in which
R²⁴ and R²⁵ independently of one another represent hydrogen, methyl, ethyl cyclopropyl, cyclopentyl, cyclohexyl, allyl or propargyl, or
Q represents a group of the formula 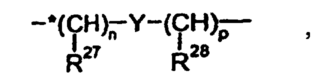 in which
R²⁷ and R²⁸ independently of one another represent hydrogen, methyl or ethyl,
Y represents an oxygen atom or represents S(O)ᵣ, where
r represents the numbers 0, 1 or 2, and
n and p independently of one another represent the numbers 0, 1 or 2,
where the molecular moiety labelled (*) is in each case attached to the phenyl radical of the aniline moiety,
X represents oxygen or sulphur,
Z represents phenyl, naphthyl or anthracenyl, where each of these radicals may be mono- to trisubstituted by identical or different substituents from the group consisting of fluorine, chlorine, bromine, methyl, ethyl, n-propyl, isopropyl, n-butyl, sec-butyl, i-butyl, tert-butyl, methoxy, ethoxy, methylthio, ethylthio, trifluoromethyl, difluoromethyl, difluorochloromethyl, trichloromethyl, trifluoromethoxy, difluoromethoxy, difluorochloromethoxy, trichloromethoxy, phenyl and/or phenoxy,
Z furthermore represents pyrrolyl, furyl, pyrazolyl, imidazolyl, thiazolyl, isothiazolyl, oxazolyl, isoxazolyl, oxadiazolyl, thiadiazolyl, 1,2,3-triazinyl, 1,2,4-triazinyl, 1,3,5-triazinyl, pyridyl, pyrimidyl, pyrazinyl or pyridazinyl, where each of these radicals may be mono- or disubstituted by identical or different substituents from the group consisting of fluorine, chlorine, bromine, cyano, nitro, methyl, ethyl, n-propyl, isopropyl, n-butyl, sec-butyl, isobutyl, tert-butyl, methoxy, ethoxy, methylthio, ethylthio, trifluoromethyl, difluoromethyl, difluorochloromethyl, trichloromethyl, trifluoromethoxy, difluoromethoxy, difluorochloromethoxy, trichloromethoxy, phenyl and/or phenoxy.

4. Process for preparing carbanilides of the formula (I) according to Claim 1, **characterized in that**
a) acyl halides of the formula 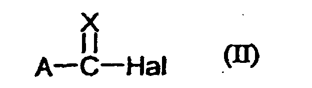 in which
A and X are each as defined in Claim 1 and
Hal represents halogen are reacted with aniline derivatives of the formula 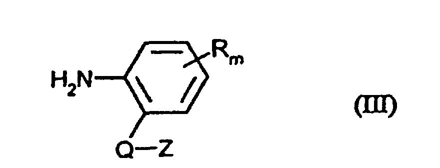
in which
Q, R, Z and m are each as defined in Claim 1,
if appropriate in the presence of an acid binder and if appropriate in the presence of a diluent,
or
b) carbanilide derivatives of the formula 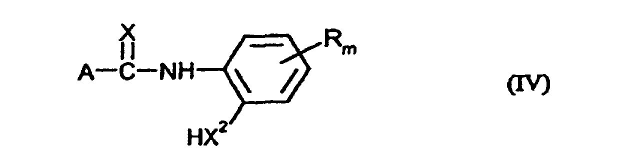 in which
A, R, X and m are each as defined in Claim 1 and
X² represents oxygen or sulphur,
are reacted with compounds of the formula 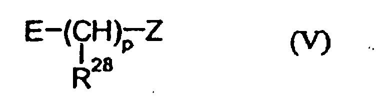 in which
R²⁸, Z and p are each as defined in Claim 1 and
E represents a leaving group,
if appropriate in the presence of an acid binder and if appropriate in the presence of a diluent, or
c) carbanilide derivatives of the formula 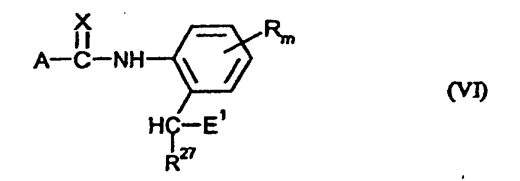 in which
A, R, R²⁷, X and m are each as defined in Claim 1, and
E¹ represents a leaving group
are reacted with compounds of the formula (VII) 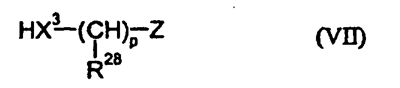 in which
R²⁸, Z and p are each as defined in Claim 1 and
X³ represents oxygen or sulphur,
if appropriate in the presence of an acid binder and if appropriate in the presence of a diluent, or
d) carbanilide derivatives of the formula 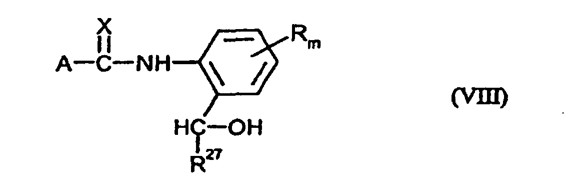 in which
A, R, R²⁷, X and m are each as defined in Claim 1
are reacted with compounds of the formula (V) 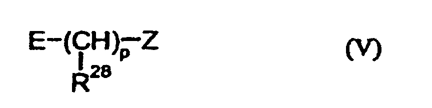 in which
R²⁸ , Z and p are each as defined in Claim 1 and
E represents a leaving group,
if appropriate in the presence of an acid binder and if appropriate in the presence of a diluent.

5. Pesticides, **characterized in that** they contain at least one carbanilide of the formula (1) according to Claim 1.

6. Use of carbanilides of the formula (I) according to Claim 1 for controlling pest.

7. Method for controlling pests, **characterized in that** carbanilides of the formula (I) according to Claim 1 are applied to the pests and/or their habitat.

8. Process for preparing pesticides, **characterized in that** carbanilides of the formula (I) according to Claim 1 are mixed with extenders and/or surfactants.

## Revendications

1. Carbanilides de formule 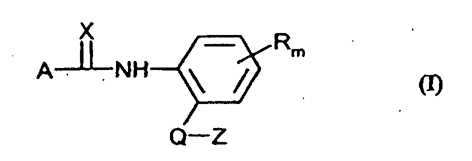 dans laquelle
R représente un halogène, un groupe nitro, cyano, un reste alkyle ayant 1 à 8 atomes de carbone, halogénalkyle ayant 1 à 6 atomes de carbone et 1 à 5 atomes d'halogène, alkoxy ayant 1 à 8 atomes de carbone, halogénalkoxy ayant 1 à 6 atomes de carbone et 1 à 5 atomes d'halogène, alkylthio ayant 1 à 8 atomes de carbone, halogénalkylthio ayant 1 à 6 atomes de carbone et 1 à 5 atomes d'halogène, alcényloxy ayant 2 à 8 atomes de carbone, alcynyloxy ayant 2 à 8 atomes de carbone, cycloalkyle ayant 3 à 8 atomes de carbone, carbalkoxy ayant 1 à 8 atomes de carbone dans la partie alkoxy ou alkoximinoalkyle ayant 1 à 6 atomes de carbone dans la partie alkoxy et 1 à 6 atomes de carbone dans la partie alkyle,
m représente les nombres 0, 1, 2, 3 ou 4,
A est un reste de formule 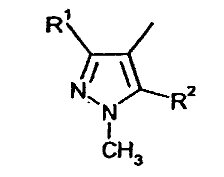 dans laquelle
R¹ est un halogène, un reste alkyle ayant 1 à 4 atomes de carbone ou un reste halogénalkyle ayant 1 à 4 atomes de carbone et 1 à 5 atomes d'halogène, et
R² représente l'hydrogène, un halogène, un groupe cyano ou un reste alkyle ayant 1 à 4 atomes de carbone,
ou bien
A est un reste de formule 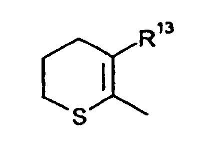 dans laquelle
R¹³ est un reste alkyle ayant 1 à 4 atomes de carbone ou un reste halogénalkyle ayant 1 à 4 atomes de carbone et 1 à 5 atomes d'halogène,
ou bien
A est un reste de formule 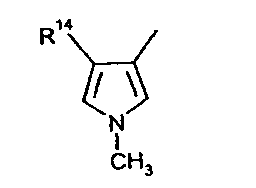 dans laquelle
R¹⁴ est un halogène, un groupe cyano, un reste alkyle ayant 1 à 4 atomes de carbone ou un reste halogénalkyle ayant 1 à 4 atomes de carbone et 1 à 5 atomes d'halogène,
ou bien
A est un reste de formule 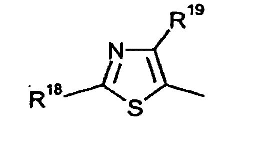 dans laquelle
R¹⁸ représente l'hydrogène, un halogène, un groupe amino, cyano ou un reste alkyle ayant 1 à 4 atomes de carbone, et
R¹⁹ est un halogène, un reste alkyle ayant 1 à 4 atomes de carbone ou un reste halogénalkyle ayant 1 à 4 atomes de carbone et 1 à 5 atomes d'halogène,
ou bien
A est un reste de formule 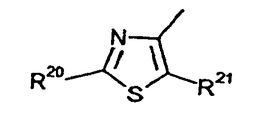 dans laquelle
R²⁰ représente l'hydrogène, un halogène, un groupe amino, cyano ou un reste alkyle ayant 1 à 4 atomes de carbone et
R²¹ est un halogène, un reste alkyle ayant 1 à 4 atomes de carbone ou un reste halogénalkyle ayant 1 à 4 atomes de carbone et 1 à 5 atomes d'halogène,
ou bien
A est un reste de formule 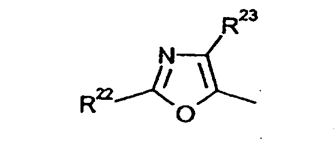 dans laquelle
R²² représente l'hydrogène ou un reste alkyle ayant 1 à 4 atomes de carbone et
R²³ est un halogène ou un reste alkyle ayant 1 à 4 atomes de carbone,
ou bien
A est un reste de formule 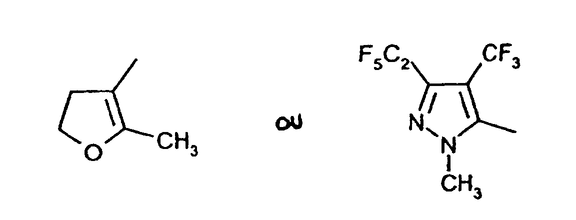
Q est un reste alkylène ayant 1 à 4 atomes de carbone, alcénylène ayant 2 à 4 atomes de carbone, alcynylène ayant 2 à 4 atomes de carbone ou un groupe de formule 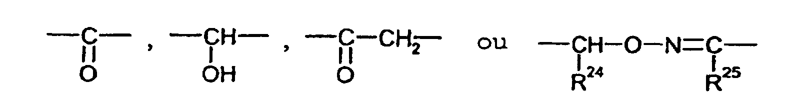 dans laquelle
R²⁴ et R²⁵ représentent, indépendamment l'un de l'autre, l'hydrogène, un reste alkyle ayant 1 à 4 atomes de carbone, cycloalkyle ayant 3 à 6 atomes de carbone, alcényle ayant 2 à 4 atomes de carbone ou alcynyle ayant 2 à 4 atomes de carbone, ou bien
Q est un groupe de formule 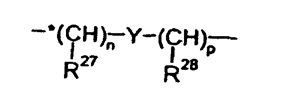 dans laquelle
R²⁷ et R²⁸ représentent, indépendamment l'un de l'autre, l'hydrogène ou un reste alkyle ayant 1 à 4 atomes de carbone,
Y représente un atome d'oxygène ou un groupe S(O)ᵣ, où
r représente le nombre 0, 1 ou 2, et
n et p représentent, indépendamment l'un de l'autre, les nombres 0, 1 ou 2,
la partie de la molécule marquée du signe (*) étant liée dans chaque cas au reste phényle de la partie aniline,
X représente l'oxygène ou le soufre et
Z est un reste phényle éventuellement substitué, naphtyl éventuellement substitué, anthracényle éventuellement substitué ou hétaryle éventuellement substitué.

2. Carbanilides de formule (I) suivant la revendication 1, dans laquelle
R représente le fluor, le chlore, le brome, un groupe nitro, cyano, un reste alkyle ayant 1 à 6 atomes de carbone, halogénalkyle ayant 1 ou 2 atomes de carbone et 1 à 5 atomes de fluor, de chlore et/ou de brome, un reste alkoxy ayant 1 à 6 atomes de carbone, halogénalkoxy ayant 1 ou 2 atomes de carbone et 1 à 5 atomes de fluor, de chlore et/ou de brome, un reste alkylthio ayant 1 à 6 atomes de carbone, un reste halogénalkylthio ayant 1 ou 2 atomes de carbone et 1 à 5 atomes de fluor, de chlore et/ou de brome, un reste alcényloxy ayant 2 à 6 atomes de carbone, un reste alcynyloxy ayant 2 à 6 atomes de carbone, un reste cycloalkyle ayant 3 à 7 atomes de carbone, un reste carbalkoxy ayant 1 à 4 atomes de carbone dans la partie alkoxy ou un reste alkoximinoalkyle ayant 1 à 4 atomes de carbone dans la partie alkoxy et 1 à 4 atomes de carbone dans la partie alkyle,
m représente les nombres 0, 1, 2 ou 3, R représentant des restes identiques ou différents lorsque m a la valeur 2 ou 3,
A est un reste de formule 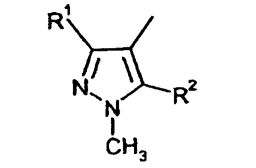 dans laquelle
R¹ représente le fluor, le chlore, le brome, l'iode, un reste méthyle, éthyle, isopropyle ou halogénalkyle ayant 1 ou 2 atomes de carbone et 1 à 5 atomes de fluor, de chlore et/ou de brome, et
R² représente l'hydrogène, le fluor, le chlore, le brome, l'iode, un reste méthyle ou éthyle,
ou bien
A est un reste de formule 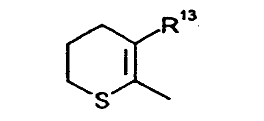 dans laquelle
R¹³ est un reste méthyle, éthyle ou halogénalkyle ayant 1 ou 2 atomes de carbone et 1 à 5 atomes de fluor, de chlore et/ou de brome,
ou bien
A est un reste de formule 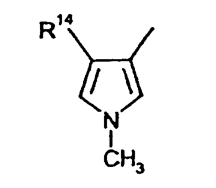 dans laquelle
R¹⁴ représente le fluor, le chlore, le brome, un groupe cyano, un reste méthyle, éthyle ou halogénalkyle ayant 1 ou 2 atomes de carbone et 1 à 5 atomes de fluor, de chlore et/ou de brome,
ou bien
A est un reste de formule 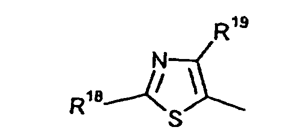 dans laquelle
R¹⁸ représente l'hydrogène, le fluor, le chlore, le brome, un groupe amino, cyano, un reste méthyle ou éthyle et
R¹⁹ représente le fluor, le chlore, le brome, un reste méthyle, éthyle ou halogénalkyle ayant 1 ou 2 atomes de carbone et 1 à 5 atomes de fluor, de chlore et/ou de brome,
ou bien
A est un reste de formule 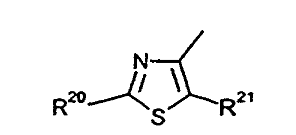 dans laquelle
R²⁰ représente l'hydrogène, le fluor, le chlore, le brome, un groupe amino, cyano, un reste méthyle ou éthyle et
R²¹ représente le fluor, le chlore, le brome, un reste méthyle, éthyle ou halogénalkyle ayant 1 ou 2 atomes de carbone et 1 à 5 atomes de fluor, de chlore et/ou de brome,
ou bien
A est un reste de formule 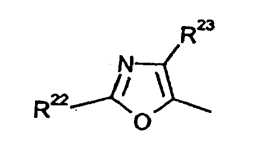 dans laquelle
R²² représente l'hydrogène, un reste méthyle ou éthyle et
R²³ représente le fluor, le chlore, le brome, un reste méthyle ou éthyle,
ou bien
A est un reste de formule 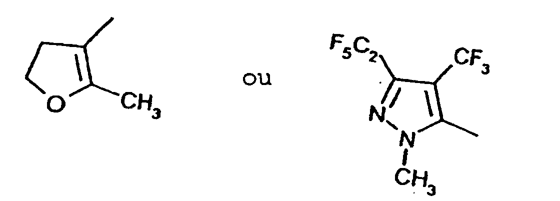
Q représente un reste alkylène ayant 1 à 3 atomes de carbone, un reste alcénylène ayant 2 ou 3 atomes de carbone, un reste alcynylène ayant 2 ou 3 atomes de carbone ou un groupe de formule 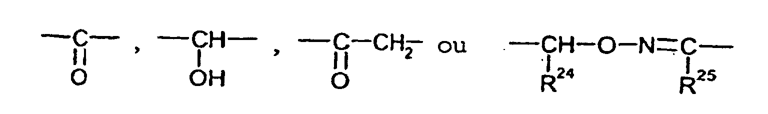 dans laquelle
R²⁴ et R²⁵ représentent, indépendamment l'un de l'autre, l'hydrogène, un reste méthyle, éthyle, cyclopropyle, cyclopentyle, cyclohexyle, alcényle ayant 2 ou 3 atomes de carbone ou alcynyle ayant 2 ou 3 atomes de carbone, ou bien
Q est un groupe de formule 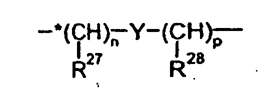 dans laquelle
R²⁷ et R²⁸ représentent, indépendamment l'un de l'autre, l'hydrogène, un reste méthyle ou éthyle,
Y représente un atome d'oxygène ou un groupe S(O)ᵣ, où
r représente le nombre 0, 1 ou 2, et
n et p représentent, indépendamment l'un de l'autre, les nombres 0, 1 ou 2,
la partie de la molécule marquée d'un signe (*) étant liée dans chaque cas au reste phényle de la partie aniline,
X représente l'oxygène ou le soufre,
Z est un reste phényle, naphtyle ou anthracényle, chacun de ces restes pouvant être substitué une à trois fois identiques ou différentes par un halogène, un radical alkyle ayant 1 à 4 atomes de carbone, alkylthio ayant 1 à 4 atomes de carbone, halogénalkyle ayant 1 à 5 atomes de fluor, de chlore et/ou de brome, halogénalkoxy ayant 1 ou 2 atomes de carbone et 1 à 5 atomes de fluor, de chlore et/ou de brome, phényle et/ou phénoxy,
Z représente en outre un reste hétaryle à noyau de 5 ou 6 chaînons et ayant 1 à 3 hétéroatomes tels qu'oxygène, soufre et/ou azote, chacun des hétérocycles pouvant être substitué une ou deux fois identiques ou différentes par un radical halogène, cyano, nitro, alkyle ayant 1 à 4 atomes de carbone, alkoxy ayant 1 à 4 atomes de carbone, alkylthio ayant 1 à 4 atomes de carbone, halogénalkyle ayant 1 ou 2 atomes de carbone et 1 à 5 atomes de fluor, de chlore et/ou de brome, halogénalkoxy ayant 1 ou 2 atomes de carbone et 1 à 5 atomes de fluor, de chlore et/ou de brome, phényl et/ou phénoxy.

3. Carbanilides de formule (I) suivant la revendication 1, dans laquelle
R représente le fluor, le chlore, le brome, un groupe nitro, cyano, un reste méthyle, éthyle, n-propyle, isopropyle, n-butyle, sec.-butyle, isobutyle, tertiobutyle, trichlorométhyle, trifluorométhyle, difluorométhyle, difluorochlorométhyle, méthoxy, éthoxy, difluorométhoxy, trifluorométhoxy, difluorochlorométhylthio, allyloxy, propargyloxy, cyclopropyle, cyclopentyle, cyclohexyle, cycloheptyle, méthoxycarbonyle, éthoxycarbonyle, méthoximinométhyle, éthoximinométhyle, méthoximinoéthyle ou éthoximinoéthyle,
m représente les nombres 0, 1, 2 ou 3, R représentant des restes identiques ou différents lorsque m a la valeur 2 ou 3,
A est un reste de formule 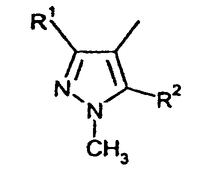 dans laquelle
R¹ représente le fluor, le chlore, le brome, l'iode, un reste méthyle, éthyle, isopropyle, difluorométhyle, trifluorométhylme, difluorochlorométhyle ou trichlorométhyle et
R² représente l'hydrogène, le fluor, le chlore, le brome, l'iode, un reste méthyle ou éthyle,
ou bien
A est un reste de formule 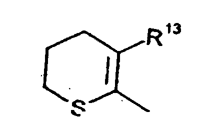 dans laquelle
R¹³ est un reste méthyle, éthyle, trifluorométhyle, difluorométhyle, difluorochlorométhyle ou trichlorométhyle,
ou bien
A est un reste de formule 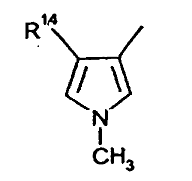 dans laquelle
R¹⁴ représente le fluor, le chlore, le brome, un groupe cyano, un reste méthyle, éthyle, trifluorométhyle, difluorméthyle, difluorochlorométhyle ou trichlorométhyle,
ou bien
A est un reste de formule 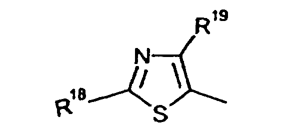 dans laquelle
R¹⁸ représente l'hydrogène, le fluor, le chlore, le brome, un groupe amino, cyano, un reste méthyle ou éthyle et
R¹⁹ représente le fluor, le chlore, le brome, un reste méthyle, éthyle, trifluorométhyle, difluorométhyle, difluorochlorométhyle ou trichlorométhyle,
ou bien
A est un reste de formule 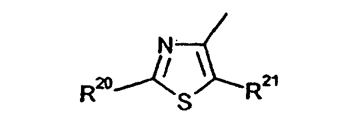 dans laquelle
R²⁰ représente l'hydrogène, le fluor, le chlore, le brome, un groupe amino, cyano, un reste méthyle ou éthyle et
R²¹ représente le fluor, le chlore, le brome, un reste méthyle, éthyle, trifluorométhyle, difluorométhyle, difluorochlorométhyle ou trichlorométhyle,
ou bien
A est un reste de formule 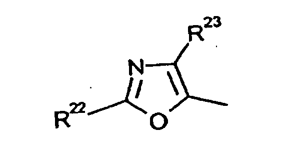 dans laquelle
R²² représente l'hydrogène, un reste méthyle ou éthyle et
R²³ représente le fluor, le chlore, le brome, un reste méthyle ou éthyle,
A est un reste de formule 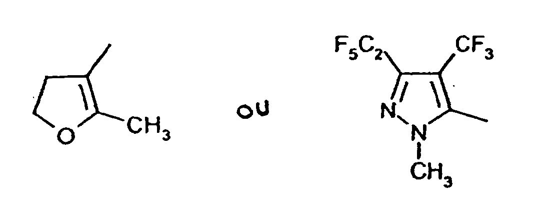
Q est un reste alkylène ayant 1 à 3 atomes de carbone, un reste alcénylène ayant 2 ou 3 atomes de carbone, un reste alcynylène ayant 2 ou 3 atomes de carbone ou un groupe de formule 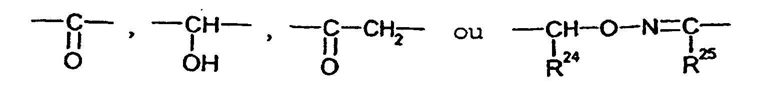 dans laquelle
R²⁴ et R²⁵ représentent, indépendamment l'un de l'autre, l'hydrogène, un reste méthyle, éthyle, cyclopropyle, cyclopentyle, cyclohexyle, allyle ou propargyle, ou bien
Q est un groupe de formule 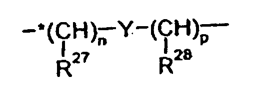 dans laquelle
R²⁷ et R²⁸ représentent, indépendamment l'un de l'autre, l'hydrogène, un reste méthyle ou éthyle,
Y est un atome d'oxygène ou un groupe S(O)ᵣ, dans lequel
r représente les nombres 0, 1 ou 2, et
n et p représentent, indépendamment l'un de l'autre, les nombres 0, 1 ou 2,
la partie de la molécule marquée du signe (*) étant liée dans chaque cas au reste phényle de la partie aniline,
X représente l'oxygène ou le soufre,
Z est un reste phényle, naphtyle ou anthracényle, chacun de ces restes pouvant être substitué une à trois fois identiques ou différentes par du fluor, du chlore, du brome, un radical méthyle, éthyle, n-propyle, isopropyle, n-butyle, sec.-butyle, isobutyle, tertiobutyle, méthoxy, éthoxy, méthylthio, éthylthio, trifluorométhyle, difluorométhyle, difluorochlorométhyle, trichlorométhyle, trifluorométhoxy, difluorométhoxy, difluorochlorométhoxy, trichlorométhoxy, phényle et/ou phénoxy,
Z représente en outre un reste pyrrolyle, furyle, pyrozolyle, imidazolyle, thiazolyle, isothiazolyle, oxazolyle, isoxazolyle, oxadiazolyle, thiadiazolyle, 1,2,3-triazinyle, 1,2,4-triazinyle, 1,3,5-triazinyle; pyridyle, pyrimidyle, pyrazinyle ou pyridazinyle, chacun de ces restes pouvant être substitué une ou deux fois identiques ou différentes par du fluor, du chlore, du brome, un radical cyano, nitro, méthyle, éthyle, n-propyle, isopropyle, n-butyle, sec.-butyle, isobutyle, tertiobutyle, méthoxy, éthoxy, méthylthio, éthylthio, trifluorométhyle, difluorométhyle, difluorochlorométhyle, trichlorométhyle, trifluorométhoxy, difluorométhoxy, difluorochlorométhoxy, trichlorométhoxy, phényle et/ou phénoxy.

4. Procédé de production de carbanilides de formule (I) suivant la revendication 1, **caractérisé en ce que** :
a) on fait réagir des halogénures d'acides de formule 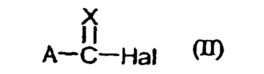 dans laquelle
A et X ont les définitions indiquées dans la revendication 1 et
Hal est un halogène,
avec des dérivés d'aniline de formule 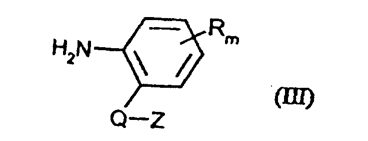 dans laquelle
Q, R, Z et m ont les définitions indiquées dans la revendication 1,
éventuellement en présence d'un accepteur d'acide et, le cas échéant, en présence d'un diluant, ou bien
b) on fait réagir des dérivés de carbanilides de formule 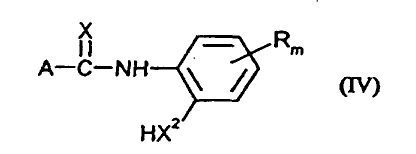 dans laquelle
A, R, X et m ont les définitions indiquées dans la revendication 1 et
X² représente l'oxygène ou le soufre,
avec des composés de formule (V) 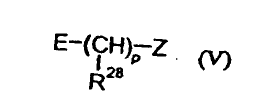 dans laquelle
R²⁸, Z et p ont les définitions indiquées dans la revendication 1 et
E est un groupe partant, ou bien
c) on fait réagir des dérivés de carbanilides de formule 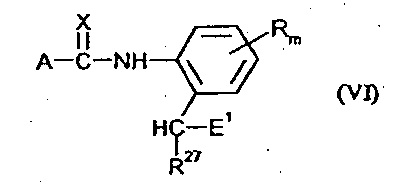 dans laquelle
A, R, R²⁷, X et m ont les définitions indiquées dans la revendication 1, et
E¹ est un groupe partant,
allié avec des composés de formule (VII) 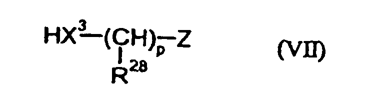 dans laquelle
R²⁸, Z et p ont les définitions indiquées dans la revendication 1 et
X³ représente l'oxygène ou le soufre,
éventuellement en présence d'un accepteur d'acide et, le cas échéant, en présence d'un diluant, ou bien
d) on fait réagir des dérivés de carbanilides de formule 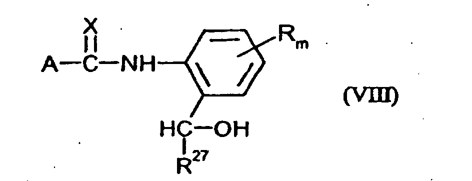 dans laquelle
A, R, R²⁷, X et m ont les définitions indiquées dans la revendication 1,
avec des composés de formule (V) 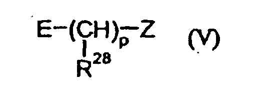 dans laquelle
R²⁸, Z et p ont les définitions indiquées dans la revendication 1 et
E représente un groupe partant,
éventuellement en présence d'un accepteur d'acide et, le cas échéant, en présence d'un diluant.

5. Compositions pesticides **caractérisées par** une teneur en au moins un carbanilide de formule (I) suivant la revendication 1.

6. Utilisation de carbanilides de formule (I) suivant la revendication 1 pour combattre des parasites.

7. Procédé pour combattre des parasites, **caractérisé en ce qu'**on épand des carbanilides de formule (I) suivant la revendication 1 sur les parasites et/ou sur leur milieu.

8. Procédé de préparation de compositions pesticides, **caractérisé en ce qu'**on mélange des carbanilides de formule (I) suivant la revendication 1 avec des diluants et/ou des agents tensioactifs.
